# EUROPEAN PATENT SPECIFICATION

(11) **EP 3 563 813 B1**
(45) Date of publication and mention of the grant of the patent: **20.04.2022**
(21) Application number: 17885581.3
(22) Date of filing: 14.12.2017
(51) Int. Cl.: A61F 5/56

(54) **MOUTHPIECE**
MUNDSTÜCK
EMBOUT BUCCAL

(30) Priority: 27.12.2016 JP 2016253255; 27.12.2016 JP 2016253263; 27.12.2016 JP 2016253265; 08.02.2017 JP 2017021254; 28.02.2017 JP 2017037311; 01.06.2017 JP 2017108996
(43) Date of publication of application: 06.11.2019
(73) Proprietor: Mitsui Chemicals, Inc., Minato-ku Tokyo 105-7122 (JP)
(72) Inventor: YUKITA, Takashi, Chiba 299-0265 (JP); NAGAI, Hideyuki, Yamaguchi 740-0061 (JP); TSUCHIYA, Yasufumi, Tokyo 105-7122 (JP); RAAD, Walid, Brooklyn, NY 11201 (US)
(74) Representative: Epping - Hermann - Fischer
(86) International application number: PCT/JP2017/044804
(87) International publication number: WO 2018/123602

(56) References cited:
- WO-A1-2011/063180
- WO-A1-2015/144374
- DE-U1-202005 007 981
- GB-A- 2 505 975
- JP-A- 2002 519 137
- JP-A- 2015 519 995
- US-A1- 2013 112 210
- US-A1- 2014 326 252
- US-A1- 2015 075 540
- US-B1- 6 604 527

## Description

### Technical Field

The present invention relates to an oral appliance.

### Background Art

In recent years, oral appliances have been used for, for example, prevention and treatment of sleep apnea syndrome.

For example, PTL 1 describes an oral appliance including a pair of upper and lower jaw oral appliances facing each other, the lower jaw oral appliance including upward flange portions that protrude upward, the upper jaw oral appliance including downward flange portions that protrude downward. The oral appliance allows each downward flange portion to abut against the relevant upward flange portion upon biting during wearing and thereby limits rearward displacement of the lower jaw of a user wearing the oral appliance to prevent narrowing of the airway, enabling prevention of pauses in breathing or infrequent breathing during sleep. Also, the oral appliance includes turnbuckle mechanisms each including one end portion fixed to the lower jaw oral appliance and another end portion fixed to the relevant upward flange portion, as position adjustment sections. A position of each upward flange portion can be adjusted using the relevant turnbuckle mechanism to move the lower jaw to the front side relative to the upper jaw by an amount suitable for fitting a shape of the jaw or the throat of the user, preventing the airway of the user from further narrowing.

PTL 2 describes an oral appliance including a pair of upper and lower jaw oral appliances facing each other, the lower jaw oral appliance including upward flange portions that protrude upward, the upper jaw oral appliance including downward flange portions that protrude downward. In each downward flange portion, an abutment member that abuts against the relevant upward flange portion as a result of position adjustment in a front-rear direction along a guide section is provided, and the position of the abutment member can be adjusted forward to move the lower jaw of a user to the front side relative to the upper jaw by an amount suitable for fitting a shape of the jaw of the user, preventing the airway of the user from further narrowing.

WO 2011/063180 A1, which discloses the features of the preamble of claim 1, describes a dental implant for treatment of sleep apnea (intraoral appliance) having an upper and lower bodies. The lower body can have an angled or fin shaped protrusion which engages an angled protrusion or wall of the upper body.

US 2014/326252 A1 describes a sleep apnea appliance having an upper and lower tray structures. The lower tray structure has fins disposed for matingly contacted by boss members.

US 2015/075540 A1 describes an apparatus for preventing sleep apnea in a wearer having a top and bottom teeth guard. The bottom teeth guard has a bottom protrusion extending from the bottom teeth guard and engage with adjusting block.

GB 2 505 975 A describes an oral appliance having upper and lower tray. The lower tray has a flange extending from mounting region of the lower tray.

DE 20 2005 007981 A describes an orthodontic device having first and second molded part.

### Summary of Invention

### Technical Problem

In the oral appliance described in WO 00/01317, upward flange portions and the relevant turnbuckle mechanism are in a positional relationship of a second-class lever with the portion of the turnbuckle mechanism, the portion being fixed to the lower jaw oral appliance, as a fulcrum, the portion of abutment between the upward flange portion and the upper jaw oral appliance as a point of effort, and the turnbuckle mechanism as a point of load. Therefore, during wearing, when the lower jaw oral appliance is displaced in a right-left direction because of, for example, teeth grinding, outward stress applied to each upward flange portion, which is a point of effort, turns into larger outward stress in the body of the relevant turnbuckle mechanism, which is a point of load. Therefore, the oral appliance described in WO 00/01317 has the problem of the turnbuckle mechanisms (position adjustment sections), which are relatively fragile structures, being prone to breaking by such large outward stress.

In view of the above problem, an object of first to fifth aspects is to provide an oral appliance including position adjustment sections for adjusting positions of a lower jaw oral appliance and an upper jaw oral appliance upon biting, the position adjustment sections being less likely to be broken by displacement in a right-left direction upon biting.

Also, in each of the oral appliances described in WO 00/01317 and US 2013/0112210, each upward flange portion and the relevant downward flange portion abut against each other when a user wearing the oral appliance opens the mouth or adjustment of positions of the upward flange portion and the downward flange portion becomes necessary when the user bites after opening of the mouth, which is likely to give a feeling of discomfort to the user.

Furthermore, in each of the oral appliances described in WO 00/01317 and US 2013/0112210, the oral appliance is fitted to the entire rows of teeth of the upper jaw and the lower jaw of a user, and thus, the user wearing the oral appliance is likely to have a feeling of discomfort.

In view of the above problem, an object of a sixth aspect is to provide an oral appliance that reduces a feeling of discomfort a user has when the user opens the mouth or during the user wearing the oral appliance.

### Solution to Problem

The invention relates to an oral appliance with the features of claim 1. In a first aspect, when a lower jaw oral appliance is displaced in a right-left direction during wearing because of, for example, teeth grinding, no stress is applied to position adjustment sections or only small stress is applied to the position adjustment sections, to suppress damage of the position adjustment sections due to the displacement in the right-left direction.

More specifically, one mode relating to the first aspect relates to an oral appliance including: a pair of upper and lower jaw oral appliances facing each other; a pair of positioning members each being adjustable in position in a front-rear direction, the pair of positioning members being provided in one oral appliance of the upper and lower jaw oral appliances; and a pair of stoppers provided in another oral appliance of the upper and lower jaw oral appliances, the pair of stoppers abutting against the respective positioning members and thereby restricting rearward displacement of the lower jaw oral appliance, and abutting against the one oral appliance and thereby restricting displacement in a right-left direction of the upper jaw oral appliance and lower jaw oral appliance.

In a second aspect, stress applied to each position adjustment section, which is a point of load of a second-class lever, is distributed in a front-rear direction of the oral appliance to suppress damage of the position adjustment sections due to the displacement in the right-left direction.

More specifically, one mode relating to the second aspect relates to an oral appliance including: a pair of upper and lower jaw oral appliances facing each other; a pair of positioning members adjustable in position in a front-rear direction along a guide section including opposite ends supported by a pair of fixing portions provided in one oral appliance of the upper and lower jaw oral appliances; and a pair of stoppers provided in another oral appliance of the upper and lower jaw oral appliances, the pair of stoppers respectively abutting against the positioning members and thereby restricting rearward displacement of the lower jaw oral appliance.

In a third aspect, a distance between each position adjustment section, which is a point of load of a second-class lever, and a fixing portion, which is a fulcrum of the second-class lever, is decreased to suppress damage of the position adjustment sections due to an excessive increase of stress applied to each upward flange portion, which is a point of effort of the second-class lever, in the relevant position adjustment section, which is a point of load of the second-class lever.

More specifically, one mode relating to the third aspect relates to an oral appliance including: a pair of upper and lower jaw oral appliances facing each other; a pair of stoppers provided in one oral appliance of the upper and lower jaw oral appliances, the pair of stoppers having a height that is equal to or lower than a height of the one oral appliance; protrusion portions fixed to another oral appliance of the upper and lower jaw oral appliances, the protrusion portions protruding from the other oral appliance toward the one oral appliance; and a pair of positioning members to be adjusted in position in a front-rear direction along a guide section including an end supported by the corresponding protrusion portion, at a position at which the positioning member is located lateral to the one oral appliance upon biting, each of the positioning members abutting against the corresponding stopper and thereby restricting rearward displacement of the lower jaw oral appliance.

In a fourth aspect, a decrease in amount of movement of each position adjustment section, which is a point of load of a second-class lever, is enabled by enabling adjustment of an amount of forward movement of the lower jaw oral appliance in each of a pair of upper and lower jaw oral appliances. In the fourth aspect, stress applied to each position adjustment section due to the principle of second-class lever is decreased by means of the above configuration to suppress damage of the position adjustment sections due to the displacement in the right-left direction.

More specifically, one mode relates to an oral appliance including: a pair of upper and lower jaw oral appliances facing each other; a pair of positioning members provided in one oral appliance of the upper and lower jaw oral appliances and being adjustable in position in a front-rear direction; and a pair of stoppers provided in another oral appliance of the upper and lower jaw oral appliances, the pair of stoppers respectively abutting against the positioning members and thereby restricting rearward displacement of the lower jaw oral appliance, and the pair of stoppers being adjustable in position in a front-rear direction.

In a fifth aspect, when the lower jaw oral appliance is moved forward, the amount of movement of each position adjustment section is further decreased to decrease stress applied to each position adjustment section due to the principle of second-class lever and thereby suppress damage of the position adjustment sections due to the displacement in the right-left direction.

More specifically, one mode relates to an oral appliance including: a pair of upper and lower jaw oral appliances facing each other; a pair of positioning members provided in one oral appliance of the upper and lower jaw oral appliances and being adjustable in position in a front-rear direction; position adjustment sections provided forward or rearward relative to the positioning members of the one oral appliance and provided for adjusting positions of the positioning members in the front-rear direction; and a pair of stoppers provided in another oral appliance of the the upper and lower jaw oral appliances, abutting against the positioning members from the same direction as the position adjustment sections and thereby restricting rearward displacement of the lower jaw oral appliance.

In a sixth aspect, either of the upper and lower jaw oral appliances is fitted to only a part of a row of teeth to reduce a feeling of discomfort a user has because of wearing the oral appliance and thereby improve a feeling of fitting a user has.

More specifically, one mode relating to the sixth aspect relates to an oral appliance including: one oral appliance that can be fitted to a row of teeth of one of an upper jaw and a lower jaw; a pair of other oral appliances that can be fitted to respective parts of a row of teeth of another one of the upper jaw and the lower jaw, the pair of other oral appliances being separated into a right and a left; joining portions that each join the one oral appliance and the relevant other oral appliance to each other and fixes respective positions in a mouth opening direction of the one oral appliance and the relevant other oral appliance relative to each other; and guide sections that each adjust a relative position in a front-rear direction of the relevant other oral appliance relative to the one oral appliance.

### Advantageous Effects of Invention

The first to the fifth aspects provide an oral appliance including position adjustment sections for adjusting positions of a lower jaw oral appliance and an upper jaw oral appliance upon biting, the position adjustment sections being less likely to be broken by displacement in a right-left direction upon biting.

The sixth aspect provides an oral appliance that reduces a feeling of discomfort a user has when the user opens the mouth or during the user wearing the oral appliance.

### Brief Description of Drawings

FIG. 1A is a schematic perspective view illustrating an upper jaw oral appliance according to Embodiment 1 of the present invention and FIG. 1B is a schematic perspective view illustrating a lower jaw oral appliance according to Embodiment 1 of the present invention;
FIG. 2 is a schematic side view illustrating an oral appliance according to Embodiment 1 of the present invention;
FIG. 3 is a schematic side view illustrating a manner in which the lower jaw oral appliance of the oral appliance according to Embodiment 1 of the present invention is moved in a mouth opening direction during wearing;
FIG. 4A is a schematic perspective view illustrating a mode of a stopper provided in the upper jaw oral appliance according to Embodiment 1, in which a front end portion includes a cutout portion, FIG. 4B is a schematic perspective view illustrating a mode of the stopper provided in the upper jaw oral appliance according to Embodiment 1, in which the front end portion includes a flat inclined portion, and FIG. 4C is a schematic perspective view of the stopper provided in the upper jaw oral appliance according to Embodiment 1, in which the front end portion includes a curved portion;
FIG. 5 is a schematic plan view illustrating a manner of forward movement of the lower jaw oral appliance of the oral appliance according to Embodiment 1 of the present invention;
FIG. 6 is a schematic side view illustrating an oral appliance according to Embodiment 2 of the present invention;
FIG. 7 is a schematic side view illustrating an oral appliance according to Embodiment 3 of the present invention;
FIG. 8A is a schematic perspective view illustrating an upper jaw oral appliance according to Embodiment 4 of the present invention and FIG. 8B is a schematic perspective view illustrating a lower jaw oral appliance according to Embodiment 4 of the present invention;
FIG. 9 is a schematic side view illustrating an oral appliance according to Embodiment 4 of the present invention;
FIG. 10 is a schematic side view illustrating an oral appliance according to Embodiment 5 of the present invention;
FIG. 11 is a schematic plan view illustrating a manner of forward movement of a lower jaw oral appliance of the oral appliance according to Embodiment 5 of the present invention;
FIG. 12A is a schematic perspective view illustrating an upper jaw oral appliance according to Embodiment 6 of the present invention and FIG. 12B is a schematic perspective view illustrating a lower jaw oral appliance according to Embodiment 6 of the present invention;
FIG. 13 is a schematic side view of an oral appliance according to Embodiment 6 of the present invention;
FIG. 14 is a schematic side view illustrating an oral appliance according to Embodiment 7 of the present invention;
FIG. 15 is a schematic plan view illustrating a manner of forward movement of a lower jaw oral appliance of an oral appliance according to Embodiment 8 of the present invention;
FIG. 16A is a schematic perspective view illustrating an upper jaw oral appliance according to Embodiment 8 of the present invention and FIG. 16B is a schematic perspective view illustrating the lower jaw oral appliance according to Embodiment 8 of the present invention;
FIG. 17 is a schematic side view illustrating the oral appliance according to Embodiment 8 of the present invention;
FIG. 18 is a schematic side view illustrating an oral appliance according to Embodiment 9 of the present invention;
FIG. 19 is a schematic side view illustrating a manner in which a lower jaw oral appliance of the oral appliance according to Embodiment 9 of the present invention is moved in a mouth opening direction during wearing;

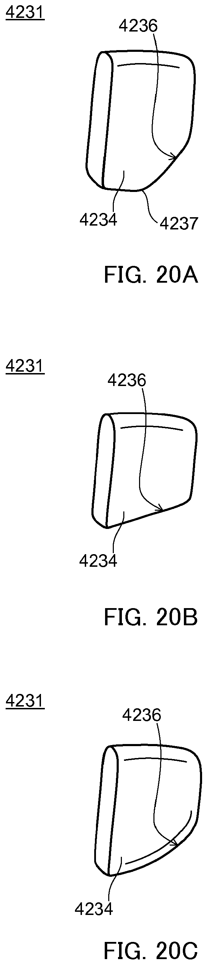

FIG. 20A is a schematic perspective view illustrating a mode of a stopper provided in an upper jaw oral appliance according to Embodiment 9, in which a front end portion includes a cutout portion, FIG. 20B is a schematic perspective view illustrating a mode of the stopper provided in the upper jaw oral appliance according to Embodiment 9, in which the front end portion includes a flat inclined portion, and FIG. 20C is a schematic perspective view of a mode of the stopper provided in the upper jaw oral appliance according to Embodiment 9, in which the front end portion includes a curved portion;
FIG. 21 is a schematic plan view illustrating a manner of forward movement of the lower jaw oral appliance of the oral appliance according to Embodiment 9 of the present invention;
FIG. 22 is a schematic side view illustrating an oral appliance according to Embodiment 10 of the present invention;
FIG. 23 is a schematic side view illustrating an oral appliance according to Embodiment 11 of the present invention;
FIG. 24A is a schematic perspective view illustrating an upper jaw oral appliance according to Embodiment 12 of the present invention and FIG. 24B is a schematic perspective view illustrating a lower jaw oral appliance according to Embodiment 12 of the present invention;
FIG. 25 is a schematic side view illustrating an oral appliance according to Embodiment 12 of the present invention;
FIG. 26 is a schematic plan view illustrating a manner of forward movement of the lower jaw oral appliance of the oral appliance according to Embodiment 12 of the present invention;
FIG. 27 is a schematic side view illustrating an oral appliance according to Embodiment 13 of the present invention;
FIG. 28 is a schematic side view illustrating an oral appliance according to Embodiment 14 of the present invention;
FIG. 29 is a schematic side view illustrating an oral appliance according to Embodiment 15 of the present invention;
FIG. 30 is a schematic perspective view illustrating an oral appliance according to Embodiment 16 of the present invention;
FIG. 31 is a schematic side view illustrating the oral appliance according to Embodiment 16 of the present invention;
FIG. 32 is a schematic side view illustrating a manner in which a lower jaw oral appliance of the oral appliance according to Embodiment 16 of the present invention is moved in a mouth opening direction during wearing;
FIG. 33 is a schematic side view illustrating an oral appliance according to Embodiment 17 of the present invention;
FIG. 34 is a schematic side view illustrating an oral appliance according to Embodiment 18 of the present invention; and
FIG. 35 is a schematic side view illustrating a manner in which a lower jaw oral appliance of the oral appliance according to Embodiment 18 of the present invention is moved in a mouth opening direction during wearing.

### Description of Embodiments

Oral appliances of the present invention will be described using a plurality of embodiments.

In the below description, "front" or "forward" and "rear" or "rearward" mean a direction toward the front side of a user wearing the oral appliance (direction toward the lips side as viewed from the tongue body in the mouth cavity) and a direction toward the rear side of the user (direction toward the throat side in the mouth cavity), respectively, a "right-left direction" means a right-left direction with reference to a middle of the upper jaw of the user wearing the oral appliance (more specifically, a direction toward each of the cheek sides as viewed from the middle of the upper jaw), "inner side" or "inside" and "outer side" or "outside" means the side far from the body surface of the user wearing the oral appliance and the side close to the body surface, respectively, and a "mouth opening direction" means a direction in which the lower jaw moves when the user wearing the oral appliance opens the mouth.

Also, in the below description, an "inner side surface" and an "outer side surface" of the oral appliance mean a surface facing the mouth cavity of the user wearing the oral appliance and a surface facing the body surface of the user when the user closes the mouth, respectively. Also, an upper jaw oral appliance and a lower jaw oral appliance do not need to be physically easily separable from each other and may be fixed to each other.

### 1. Oral Appliance Relating to First Aspect

In an oral appliance relating to a first aspect of the present invention, when a lower jaw oral appliance is displaced in a right-left direction because of, for example, teeth grinding during wearing, no stress is applied to position adjustment sections or only small stress is applied to the position adjustment sections, to suppress damage of the position adjustment sections due to the displacement in the right-left direction.

### [Embodiment 1]

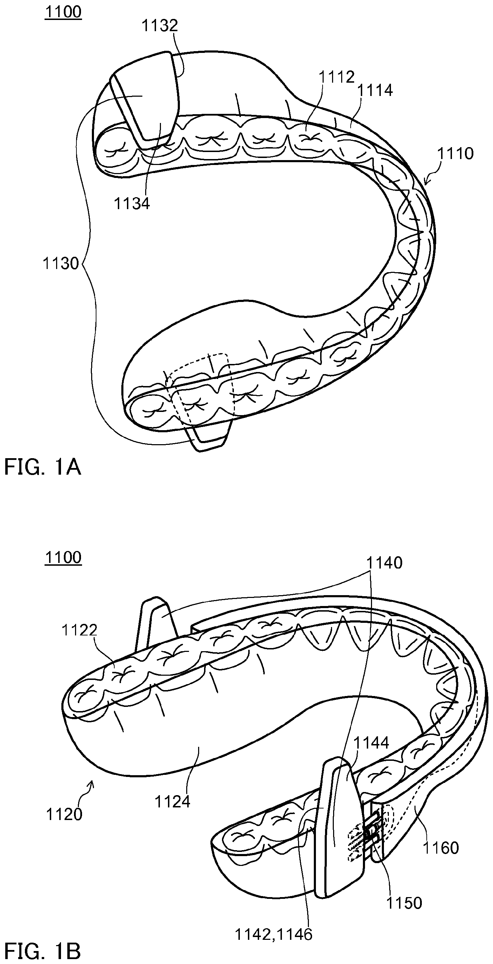

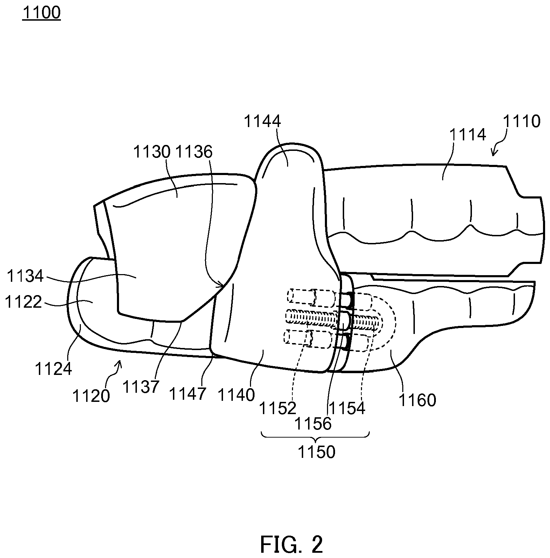

Oral appliance 1100 according to Embodiment 1 of the present invention includes a pair of upper and lower jaw oral appliances facing each other as illustrated in FIGS. 1A and 1B, which are schematic perspective views thereof, and FIG. 2, which is a side view thereof, and upper jaw oral appliance 1110 can be fitted to the upper jaw of a user and lower jaw oral appliance 1120 can be fitted to the lower jaw of the user, respectively.

As illustrated in FIG. 1A, upper jaw oral appliance 1110 includes upper jaw oral appliance body portion 1114 with teeth row shape portion 1112 formed therein, teeth row shape portion 1112 being able to be fitted to a row of teeth of the upper jaw of the user, and on an outer side surface thereof, a pair of right and left stoppers 1130 is provided.

As illustrated in FIG. 1B, lower jaw oral appliance 1120 includes lower jaw oral appliance body portion 1124 with teeth row shape portion 1122 formed therein, teeth row shape portion 1122 being able to be fitted to the row of teeth of the lower jaw of the user, and on an outer side surface thereof, a pair of right and left positioning members 1140, a position of each of the pair of right and left positioning members 1140 being adjustable in a front-rear direction is provided.

As illustrated in FIG. 2, the position of each positioning member 1140 can be adjusted to a position at which rear surface 1142 of positioning member 1140 and front surface 1132 of relevant stopper 1130 abut against each other when the user wearing upper jaw oral appliance 1110 and lower jaw oral appliance 1120 bites (hereinafter also simply referred to "upon biting"). The abutment between positioning members 1140 and stoppers 1130 limits rearward displacement of lower jaw oral appliance 1120 upon biting and thus limits rearward displacement of the lower jaw of the user wearing oral appliance 1100. Consequently, oral appliance 1100 can prevent narrowing of the airway of the user wearing oral appliance 1100 due to rearward displacement of the lower jaw of the user and thus suppress pauses in breathing or infrequent breathing during sleep.

Also, the positions of positioning members 1140 can be adjusted in the front-rear direction by respective position adjustment sections 1150 provided on the outer side surface of lower jaw oral appliance body portion 1124. The positions of positioning members 1140 are adjusted rearward by position adjustment sections 1150, enabling positioning members 1140 to, upon biting, limit rearward displacement of lower jaw oral appliance 1120 while moving lower jaw oral appliance 1120 forward. Consequently, oral appliance 1100 allows the lower jaw of the user wearing oral appliance 1100 to be moved forward and thus allows the airway of the user to open more largely, enabling effective suppression of pauses in breathing or infrequent breathing during sleep.

Each position adjustment section 1150 includes male-threaded screw rod 1152 including an end fixed to fixing portion 1160 provided on the outer side surface of lower jaw oral appliance body portion 1124, male-threaded screw rod 1154 including an end fixed to positioning member 1140, and turnbuckle portion 1156 including a screw hole to which respective other end portions of screw rods 1152 and 1154 are threadably connected. Position adjustment section 1150 can adjust the position of positioning member 1140 fixed to screw rod 1152, by inserting a rotation pin into a pin hole provided in turnbuckle portion 1156 and rotating turnbuckle portion 1156 to adjust amounts of the thread connection between turnbuckle portion 1156 and screw rods 1152 and 1154.

Each positioning member 1140 includes wing 1144 that protrudes upward from lower jaw oral appliance body portion 1124. A rear surface of wing 1144 (which is also rear surface 1142 of positioning member 1140, rear surface 1142 abutting against relevant stopper 1130) is substantially parallel to front surface 1132 of stopper 1130, and upon biting, abuts against front surface 1132 of stopper 1130 on the outer side of upper jaw oral appliance body portion 1114 and wing 1144 thereby limits rearward displacement of the lower jaw of the user.

Each stopper 1130 is provided at a position on the outer side surface of the upper jaw oral appliance, at which upon biting, stopper 1130 is located on the rear side relative to the relevant positioning member, and upon biting, front surface 1132 of each stopper 1130 abuts against rear surface 1142 of relevant positioning member 1140 and stopper 1130 thereby limits rearward displacement of lower jaw oral appliance 1120.

Each stopper 1130 includes extension portion 1134 that extends downward from upper jaw oral appliance body portion 1114. When lower jaw oral appliance 1120 is displaced in the right-left direction upon biting, each extension portion 1134 abuts against lower jaw oral appliance 1120 and thereby restricts the displacement in the right-left direction.

Here, each wing 1144 is disposed at a position at which when the lower jaw oral appliance is displaced in the right-left direction upon biting and extension portion 1134 thus abuts against lower jaw oral appliance 1120, wing 1144 does not abut against upper jaw oral appliance 1110. Consequently, stress caused by displacement in the right-left direction is applied only to extension portions 1134 of stoppers 1130 and is not applied to positioning members 1140. Therefore, damage of position adjustment sections 1150 due to displacement in the right-left direction of the lower jaw oral appliance can be suppressed.

For example, a thickness in an inside-outside direction of wings 1144 is smaller than those of stoppers 1130 and extension portions 1134 and an outer side surface of each wing 1144 is provided at a position at which the outer side surface is substantially in plane with outer side surfaces of relevant stopper 1130 and relevant extension portion 1134. Consequently, a space between each wing 1144 and upper jaw oral appliance body portion 1114 upon biting is larger than a space between each extension portion 1134 and lower jaw oral appliance body portion 1124 upon biting.

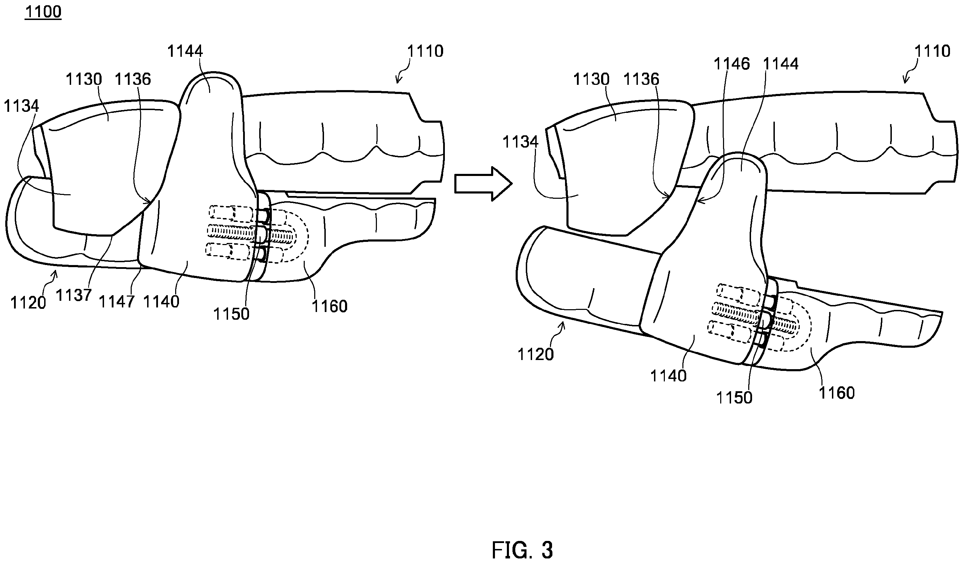

Stoppers 1130 and wings 1144 each have a shape that when lower jaw oral appliance 1120 is moved in a mouth opening direction during wearing, prevent stopper 1130 from limiting movement of wing 1144. More specifically, as illustrated in FIG. 3, each stopper 1130 may include inclined portion 1136 that facilitates movement in the mouth opening direction of lower jaw oral appliance 1120. Also, each wing 1144 may include inclined portion 1146 that facilitates movement in the mouth opening direction of lower jaw oral appliance 1120. In other words, each stopper 1130 may include a rearward inclined surface in a front end portion thereof, the inclined surface including an upper end positioned further on the front side and a lower end positioned further on the rear side, and each wing 1144 may include a rearward inclined surface in a rear end portion thereof, the inclined surface including an upper end positioned further on the front side and a lower end positioned further on the rear side. Here, inclined portion 1146 included in each wing 1144 abuts against relevant stopper 1130 upon biting.

As illustrated in FIG. 3, when lower jaw oral appliance 1120 is moved in the mouth opening direction during wearing, lower jaw oral appliance 1120 is moved on a circular arc-like path. In such movement, movement of wings 1144 is not limited by stoppers 1130 as a result of the front end portions of stoppers 1130 and the rear end portions of wings 1144 including the respective inclined surfaces described above, making it easy for the user wearing oral appliance 1100 to open the mouth.

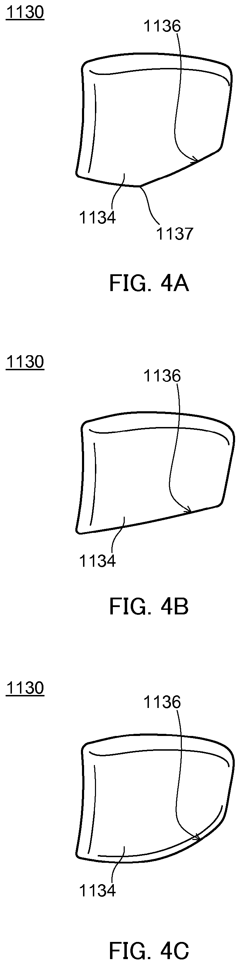

In each stopper 1130 including the inclined surface in the front end portion, the upper end of the front end portion thereof may only be positioned further on the front side and the rear end of the front end portion thereof may only be positioned further on the rear side. In this case, the front end portion of each stopper 1130 can have a shape of, for example, a cutout portion, which is illustrated in FIG. 4A, a flat inclined portion, which illustrated in FIG. 4B, or a curved portion, which is illustrated in FIG. 4C.

Likewise, in each wing 1144 including the inclined surface, the upper end of the rear end portion thereof may only be positioned further on the front side and the rear end of the rear end portion thereof may only be positioned further on the rear side. In this case, as with the front end portion of each stopper 1130, the rear end portion of each wing 1144 can have a shape of, for example, a cutout portion, a flat inclined portion, or a curved portion.

Here, the front end portion of each stopper 1130 is inclined rearward in extension portion 1134. Also, extension portions 1134 have a height that is equal to or less than half a height of stoppers 1130, and upon biting, a lower end of each extension portion 1134 is positioned above a lower end surface of lower jaw oral appliance body portion 1124. Also, upon biting, lower end 1137 of the front end portion of each stopper 1130 and lower end 1147 of the rear end portion of relevant positioning member 1140 are preferably spaced from each other. Also, upon biting, front surface 1132 of each stopper 1130 (front surface of each inclined portion 1136) and rear surface 1142 of relevant positioning member 1140 are preferably spaced from each other below an occlusal surface, and also more preferably consistently spaced from each other when the lower jaw oral appliance and/or the upper jaw oral appliance are moved in the mouth opening direction. Consequently, extension portions 1134 are less likely to limit movement in the mouth opening direction of lower jaw oral appliance 1120 during wearing, more facilitating movement in the mouth opening direction of lower jaw oral appliance 1120. Here, the height of each extension portion 1134 means a height of a part of a rear end portion of relevant stopper 1130, the part extending from a lower end of upper jaw oral appliance body portion 1114, and the height of each stopper 1130 means a height of the rear end portion of stopper 1130.

In this case, from the perspective of suppression of damage of stoppers 1130 due to displacement in the right-left direction of lower jaw oral appliance 1120, where the front end portion of each stopper 1130 includes a cutout portion such as illustrated in FIG. 4A, lower end 1137 of the cutout portion is preferably obtuse. Also, likewise, from the perspective of suppression of damage of stoppers 1130, the projection area of projection of each stopper 1130 to lower jaw oral appliance body portion 1124 from a lateral side direction upon biting is preferably 3 mm² or more. An upper limit of the projection area is not specifically provided. The projection area is, for example, preferably 3 mm² to 1000 mm², more preferably 3 mm² to 500 mm², further preferably 3 mm² to 450 mm², particularly preferably 50 mm² to 450 mm².

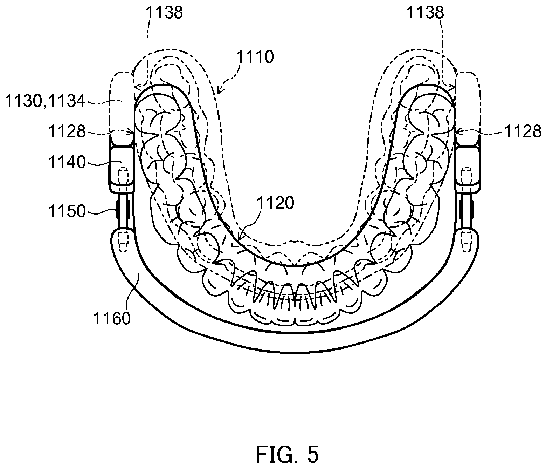

In this case, also, a shape and disposition of each of portions of stoppers 1130, the portions abutting against lower jaw oral appliance body portion 1124, and portions of lower jaw oral appliance 1120, the portions abutting against respective stoppers 1130, are those that do not limit forward movement of lower jaw oral appliance 1120. More specifically, as illustrated in FIG. 5, which is a schematic plan view of oral appliance 1100 (the upper jaw oral appliance and the stoppers are each indicated by a dashed line and the lower jaw oral appliance, the positioning members, and the fixing portions are each indicated by a solid line), each of abutment portions 1128 of lower jaw oral appliance body portion 1124, abutment portions 1128 abutting against respective stoppers 1130, has a substantially flat shape. Also, a pair of abutment surfaces 1138 of extension portions 1134 of the pair of stoppers 1130, abutment surfaces 1138 abutting against lower jaw oral appliance 1120, are substantially parallel to each other and abutment portions 1128 positioned in right and left parts of the outer side surface of lower jaw oral appliance body portion 1124, abutment portions 1128 abutting against respective extension portions 1134 of the pair of stoppers 1130, are substantially parallel to each other.

Consequently, lower jaw oral appliance 1120 can be moved forward without movement of lower jaw oral appliance 1120 being restricted by extension portions 1134. In this case, for example, a thickness in the right-left direction of each part, in the vicinity of the back teeth, of lower jaw oral appliance body portion 1124 is larger on the front side (for example, a part, in the vicinity of the first molar tooth (tooth number: 6), of the outer side surface) and is smaller on the rear side (for example, a part, in the vicinity of the second molar tooth (tooth number: 7) or the third molar tooth (tooth number: 8), of the outer side surface).

Stoppers 1130 may be detachably attachable to upper jaw oral appliance body portion 1114 but may be bonded to upper jaw oral appliance body portion 1114. If stoppers 1130 are bonded to upper jaw oral appliance body portion 1114, stoppers 1130 are firmly fixed to the upper jaw oral appliance body portion and thus damage of stoppers 1130 by stress caused when stoppers 1130 abut against lower jaw oral appliance body portion 1124 by displacement in the right-left direction can be suppressed.

Alternatively, stoppers 1130 may be integrated with the outer side surface of upper jaw oral appliance body portion 1114. If stoppers 1130 are integrated with upper jaw oral appliance body portion 1114, stoppers 1130 are further firmly fixed to upper jaw oral appliance body portion 1114 and thus damage of stoppers 1130 by stress caused when stoppers 1130 abuts against lower jaw oral appliance body portion 1124 by displacement in the right-left direction can more effectively be suppressed.

Fixing portion 1160 may be detachably attachable to lower jaw oral appliance body portion 1124 but may be bonded to lower jaw oral appliance body portion 1124. If fixing portion 1160 is bonded to lower jaw oral appliance body portion 1124, misalignment of fixing portion 1160 and positioning members 1140 is less likely to occur during wearing.

According to the present embodiment, a majority of stress that is conventionally applied only to wings 1144 of positioning members 1140 upon displacement in the right-left direction is applied to stoppers 1130. Therefore, oral appliance 1100 according to the present embodiment enables suppression of damage of position adjustment sections 1150 by stress caused when wings 1144 of positioning members 1140 abut against upper jaw oral appliance body portion 1114 as a result of displacement in the right-left direction.

Furthermore, according to the present embodiment, each stopper 1130 includes extension portion 1134 that extends downward and each positioning member 1140 includes wing 1144 that protrudes upward, and thus, for example, even when the user opens the mouth during wearing, front surface 1132 of stopper 1130 and the rear surface of wing 1144 can abut against each other. Therefore, oral appliance 1100 according to the present embodiment is less likely to cause a misalignment between stoppers 1130 and positioning members 1140 due to, for example, opening of the mouth during wearing and enables more effective suppression of rearward displacement of the lower jaw of a user wearing oral appliance 1100.

### [Embodiment 2]

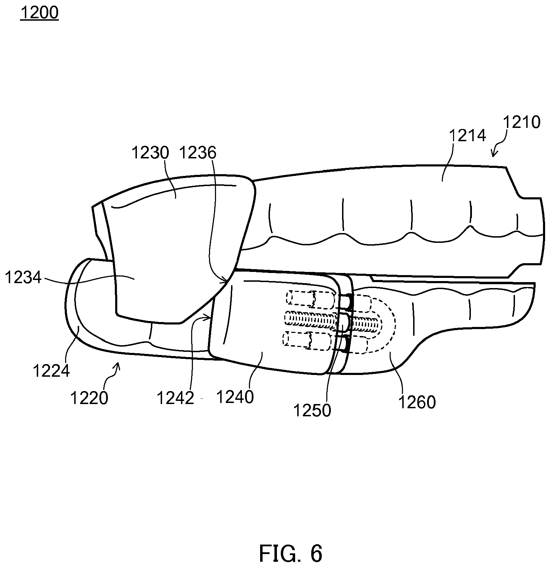

Oral appliance 1200 according to Embodiment 2 of the present invention includes a pair of upper and lower jaw oral appliances facing each other as illustrated in FIG. 6, which is a schematic side view thereof, upper jaw oral appliance 1210 can be fitted to the upper jaw of a user and lower jaw oral appliance 1220 can be fitted to the lower jaw of the user. Also, a pair of right and left positioning members 1240 which are adjustable in position in a front-rear direction is provided on an outer side surface of lower jaw oral appliance body portion 1224 and a pair of right and left stoppers 1230 is provided on an outer side surface of upper jaw oral appliance body portion 1214. In the below, description of components that are in common with Embodiment 1 may be omitted.

In the present embodiment, also, the positions of positioning members 1240 can be adjusted in the front-rear direction by respective position adjustment sections 1250 provided on the outer side surface of lower jaw oral appliance body portion 1224. The positions of positioning members 1240 are adjusted rearward by respective position adjustment sections 1250, enabling positioning members 1240 to, upon biting, limit rearward displacement of lower jaw oral appliance 1220 while moving lower jaw oral appliance 1220 forward.

On the other hand, in the present embodiment, positioning members 1240 have a height that is equal to or lower than a height of lower jaw oral appliance body portion 1224.

The above-described configuration substantially prevents positioning members 1240 from abutting against upper jaw oral appliance 1210 upon displacement in a right-left direction. Therefore, stress caused by displacement in the right-left direction is less likely to be applied to positioning members 1240 and damage of position adjustment sections 1250 due to the stress is thus suppressed.

On the other hand, each stopper 1230 includes extension portion 1234 that extends downward from upper jaw oral appliance body portion 1214. Each extension portion 1234 abuts against rear surface 1242 of relevant positioning member 1240 and upon biting, limits rearward displacement of lower jaw oral appliance 1220.

Therefore, each stopper 1230 limits rearward displacement of lower jaw oral appliance 1220 upon biting by means of relevant extension portion 1234 abutting against relevant positioning member 1240 and thus can limit rearward displacement of the lower jaw of the user wearing oral appliance 1200. Consequently, oral appliance 1200 prevents narrowing of the airway of the user wearing oral appliance 1200 due to rearward displacement of the lower jaw of the user, enabling suppression of pauses in breathing or infrequent breathing during sleep.

Here, each stopper 1230 has a shape that when lower jaw oral appliance 1220 moves in a mouth opening direction during wearing, prevents stopper 1230 from limiting movement of relevant positioning member 1240. More specifically, each stopper 1230 may include inclined portion 1236 that facilitates movement in the mouth opening direction of lower jaw oral appliance 1220. In other words, a front end portion of each stopper 1230 may include a rearward inclined surface including an upper end positioned further on the front side and a lower end positioned further on the rear side.

In each stopper 1230 including the inclined surface in the front end portion, the upper end of the front end portion thereof may only be positioned further on the front side and the rear end of the front end portion thereof may only be positioned further on the rear side. In this case, as in Embodiment 1, the front end portion of each stopper 1230 can have a shape of, for example, a cutout portion, a flat inclined portion, or a curved portion.

Here, also, the front end portion of each stopper 1230 is inclined rearward in extension portion 1234. Also, extension portion 1234 has a height that is equal to or less than half a height of stoppers 1230, and upon biting, a lower end of each extension portion 1234 is positioned above a lower end surface of lower jaw oral appliance body portion 1224.

Also, upon biting, the upper end of the front end portion of each stopper 1230 and an upper end of a rear end portion of relevant positioning member 1240 are preferably spaced from each other. Also, upon biting, a front surface of each stopper 1230 (front surface of each inclined portion 1236) and rear surface 1242 of relevant positioning member 1240 are preferably spaced from each other below an occlusal surface, and also more preferably consistently spaced from each other when the lower jaw oral appliance and/or the upper jaw oral appliance are moved in the mouth opening direction. Consequently, extension portions 1234 are less likely to limit movement in the mouth opening direction of lower jaw oral appliance 1220 during wearing, facilitating movement in the mouth opening direction of lower jaw oral appliance 1220.

In this case, from the perspective of suppression of damage of stopper 1230 due to displacement in the right-left direction of lower jaw oral appliance 1220, where the front end portion of each stopper 1230 includes a cutout portion, a lower end of the cutout portion is preferably obtuse. Also, the projection area of projection of each stopper 1230 to lower jaw oral appliance body portion 1224 from a lateral side direction upon biting is preferably 3 mm² or more. An upper limit of the projection area is not specifically provided. The projection area is preferably, for example, 3 mm² to 1000 mm², more preferably 3 mm² to 500 mm², further preferably 3 mm² to 450 mm², particularly preferably 50 mm² to 450 mm².

In this case, also, a shape and disposition of each of portions of stoppers 1230, the portions abutting against lower jaw oral appliance body portion 1224, and portions of lower jaw oral appliance 1220, the portions abutting against respective stoppers 1230, are those that do not limit forward movement of lower jaw oral appliance 1220. More specifically, each of the abutment portions of lower jaw oral appliance body portion 1224, the abutment portions abutting against respective stoppers 1230, has a substantially flat shape. Also, a pair of abutment surfaces of extension portion 1234 of the pair of stoppers 1230, the abutment surfaces abutting against lower jaw oral appliance 1220, are substantially parallel to each other, and abutment portions positioned in right and left parts of the outer side surface of lower jaw oral appliance body portion 1224, the abutment portions abutting against respective extension portions 1234 of the pair of stoppers 1230, are substantially parallel to each other.

Here, also, stoppers 1230 may be detachably attachable to upper jaw oral appliance body portion 1214 but may be bonded to upper jaw oral appliance body portion 1214. Alternatively, stoppers 1230 may be integrated with the outer side surface of upper jaw oral appliance body portion 1214.

Here, also, fixing portion 1260 may be detachably attachable to lower jaw oral appliance body portion 1224 but may be bonded to lower jaw oral appliance body portion 1224.

The height of the lower jaw oral appliance may vary depending on the position. In this case, in the present embodiment, positioning members 1240 may only have a height that is equal to or lower than a height of a part, in which positioning members 1240 are provided, of lower jaw oral appliance body portion 1224.

The height of each positioning member 1240 may be partially larger than that of lower jaw oral appliance body portion 1224, but is preferably consistently equal to or lower than the height of lower jaw oral appliance body portion 1224.

Also, an inclined surface may be provided in a rear surface of each positioning member 1240 to make respective surfaces of positioning member 1240 and extension portion 1234, the surfaces abutting against each other, substantially parallel to each other.

According to the present embodiment, upon displacement in the right-left direction, stress is less likely to be applied to positioning members 1240. Therefore, oral appliance 1200 according to the present embodiment enables suppression of damage of position adjustment sections 1250 due to displacement in the right-left direction.

Also, according to the present embodiment, positioning members 1240 each include no wing that protrudes upward from lower jaw oral appliance 1220, which is less likely to give a user a feeling of discomfort due to the wings abutting against the inner cheeks of the user during wearing.

Also, according to the present embodiment, positioning members 1240 each include no wing that protrudes upward from lower jaw oral appliance 1220, and thus, upon movement in the mouth opening direction of lower jaw oral appliance 1220, positioning members 1240 and stoppers 1230 are less likely to abut against each other. Therefore, oral appliance 1200 according to the present embodiment further facilitates movement in the mouth opening direction of lower jaw oral appliance 1220.

### [Embodiment 3]

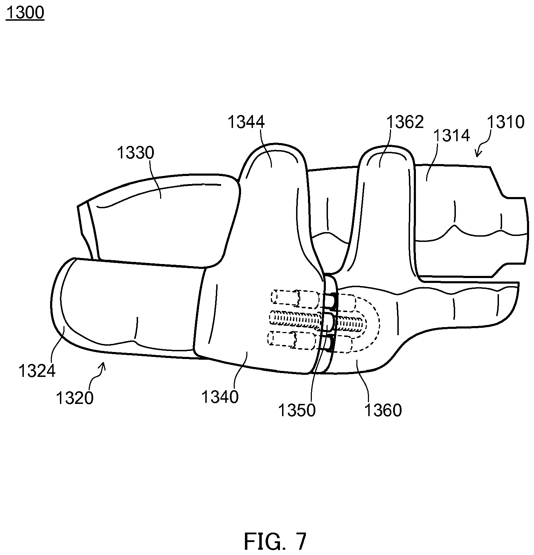

Oral appliance 1300 according to Embodiment 3 of the present invention includes a pair of upper and lower jaw oral appliances facing each other as illustrated in FIG. 7, which is a schematic side view thereof, upper jaw oral appliance 1310 can be fitted to the upper jaw of a user and lower jaw oral appliance 1320 can be fitted to the lower jaw of the user. Also, a pair of right and left positioning members 1340 which are adjustable in position in a front-rear direction is provided on an outer side surface of lower jaw oral appliance body portion 1324 and a pair of right and left stoppers 1330 is provided on an outer side surface of upper jaw oral appliance body portion 1314. In the below, description of components that are in common with Embodiment 1 may be omitted.

In the present embodiment, also, the positions of positioning members 1340 can be adjusted in the front-rear direction by respective position adjustment sections 1350 provided on the outer side surface of lower jaw oral appliance body portion 1324. The positions of positioning members 1340 are adjusted rearward by respective position adjustment sections 1350, enabling positioning members 1340 to, upon biting, limit rearward displacement of lower jaw oral appliance 1320 while moving lower jaw oral appliance 1320 forward.

In the present embodiment, also, each positioning member 1340 includes wing 1344 that protrudes upward from lower jaw oral appliance body portion 1324. A rear surface of each wing 1344, the rear surface abutting against relevant stopper 1330, is substantially parallel to a front surface of stopper 1330, and upon biting, abuts against the front surface of stopper 1330 on the outer side of upper jaw oral appliance body portion 1314 and wing 1344 thereby limits rearward displacement of the lower jaw of the user.

On the other hand, in the present embodiment, fixing portion 1360 includes protrusion portions 1362 that extend above lower jaw oral appliance 1320. When lower jaw oral appliance 1320 is displaced in a right-left direction upon biting, each protrusion portion 1362 abuts against upper jaw oral appliance 1310 and thereby limits the displacement in the right-left direction.

In this case, when the lower jaw oral appliance is displaced in the right-left direction upon biting and protrusion portions 1362 thereby abut against upper jaw oral appliance 1310, wings 1344 are disposed at respective positions at which wings 1344 are less likely to abut against upper jaw oral appliance 1310. Consequently, a majority of stress caused by displacement in the right-left direction is applied to protrusion portions 1362 of fixing portion 1360 but is less likely to be applied to positioning members 1340. Therefore, damage of position adjustment sections 1350 due to displacement in the right-left direction of the lower jaw oral appliance can be suppressed.

For example, a thickness in an inside-outside direction of wings 1344 is smaller than that of protrusion portions 1362 of fixing portion 1360 and an outer side surface of each wing 1344 is provided at a position at which the outer side surface is substantially in plane with an outer side surface of fixing portion 1360 and relevant protrusion portion 1362 thereof. Consequently, a distance between each wing 1344 and upper jaw oral appliance body portion 1314 upon biting is larger than a distance between relevant protrusion portion 1362 and upper jaw oral appliance body portion 1314 upon biting.

Protrusion portions 1362 are preferably provided further on the rear side of the outer side surface of fixing portion 1360 and are preferably provided at respective rear ends of the outer side surface (for example, parts of the outer side surface, the parts corresponding to the respective first premolar teeth (tooth number: 4)). The skin of the inner cheeks of a human more easily stretches and shrinks further on the rear side. Therefore, as protrusion portions 1362 are provided further on the rear side, which means that protrusion portions 1362 are provided at respective positions corresponding to the parts, in which the skin more easily stretches and shrinks, of the inner cheeks of the user wearing oral appliance 1300, a feeling of fitting the user wearing oral appliance 1300 has is improved.

Also, a thickness in the inside-outside direction of protrusion portions 1362 is preferably smaller than that of fixing portion 1360 and an outer side surface of each protrusion portion 1362 is preferably provided at a position at which the outer side surface is located on the inner side relative to the outer side surface of fixing portion 1360. Consequently, upon opening of the mouth during wearing, the user is less likely to have a feeling of discomfort due to protrusion portions 1362 abutting against the inner cheeks.

In Embodiment 3, also, each stopper 1330 may be detachably attachable to upper jaw oral appliance body portion 1314 but may be bonded to upper jaw oral appliance body portion 1314. Alternatively, each stopper 1330 may be integrated with the outer side surface of upper jaw oral appliance body portion 1314.

Also, in Embodiment 3, fixing portion 1360 may be detachably attachable to lower jaw oral appliance body portion 1324 but may be bonded to lower jaw oral appliance body portion 1324.

According to the present embodiment, a majority of stress that is conventionally applied only to wings 1344 of positioning members 1340 upon displacement in the right-left direction is applied to fixing portion 1360. Therefore, oral appliance 1300 according to the present embodiment enables suppression of damage of position adjustment sections 1350 by stress caused when wings 1344 of positioning members 1340 abut against upper jaw oral appliance body portion 1314 as a result of displacement in the right-left direction.

### [Variation of Embodiment 3]

In Embodiment 3, as in Embodiment 1, each stopper 1330 may include an extension portion (not illustrated in FIG. 7). Consequently, stress caused by displacement in the right-left direction is distributed not only to protrusion portions 1362 of fixing portion 1360 but also to the extension portions of stoppers 1330, and thus, only small stress is applied to position adjustment sections 1350, enabling more effective suppression of damage of position adjustment sections 1350 due to displacement in the right-left direction.

In this case, as in Embodiment 1, stoppers 1330 each have a shape that prevents stopper 1330 from limiting movement of relevant positioning member 1340 when lower jaw oral appliance 1320 is moved in a mouth opening direction during wearing. More specifically, each stopper 1330 may include an inclined portion that facilitates movement in the mouth opening direction of lower jaw oral appliance 1320. In other words, a front end portion of each stopper 1330 may include an inclined surface including an upper end positioned further on the front side and a lower end positioned further on the rear side.

In each stopper 1330 including the inclined surface in the front end portion, the upper end of the front end portion thereof may only be positioned further on the front side and the rear end of the front end portion thereof may only be positioned further on the rear side. In this case, as in Embodiment 1, the front end portion of each stopper 1330 can have a shape of, for example, a cutout portion, a flat inclined portion or a curved portion.

Here, also, the front end portion of each stopper 1330 may be inclined rearward in the relevant extension portion. Also, the extension portions may have a height that is equal to or less than half a height of stoppers 1330, and upon biting, a lower end of each extension portion may be positioned above a lower end surface of lower jaw oral appliance body portion 1324. Also, the lower end of each stopper 1330 and a lower end of relevant positioning member 1340 are preferably spaced from each other upon biting. Also, upon biting, a front surface of each stopper 1330 and a rear surface of relevant positioning member 1340 are preferably spaced from each other below an occlusal surface, and also more preferably consistently spaced from each other when the lower jaw oral appliance and/or the upper jaw oral appliance are moved in the mouth opening direction.

In this case, also, from the perspective of suppression of damage of stoppers 1330 due to displacement in a right-left direction of lower jaw oral appliance 1320, where the front end portion of each stopper 1330 includes a cutout portion, the lower end of the cutout portion is preferably obtuse. Also, the projection area of projection of each stopper 1330 to lower jaw oral appliance body portion 1324 from a lateral side direction upon biting is preferably 3 mm² or more. An upper limit of the projection area is not specifically provided. The projection area is, for example, preferably 3 mm² to 1000 mm², more preferably 3 mm² to 500 mm², further preferably 3 mm² to 450 mm², particularly preferably 50 mm² to 450 mm².

In this case, also, a shape and disposition of each of portions of stoppers 1330, the portions abutting against lower jaw oral appliance body portion 1324, and portions of lower jaw oral appliance 1320, the portions abutting against respective stoppers 1330, may be those that do not limit forward movement of lower jaw oral appliance 1320. More specifically, each of abutment portions of lower jaw oral appliance body portion 1324, the abutment portions abutting against respective stoppers 1330, may have a substantially flat shape. Also, a pair of abutment surfaces of the extension portions of the pair of stoppers 1330, the abutment surfaces abutting against lower jaw oral appliance 1320, are substantially parallel to each other and abutment portions positioned in right and left parts of the outer side surface of lower jaw oral appliance body portion 1324, the abutment portions abutting against the respective extension portions of the pair of stoppers 1330, are substantially parallel to each other.

In this case, also, as in Embodiment 2, positioning members 1340 may have a height that is equal to or lower than a height of lower jaw oral appliance body portion 1324.

### [Variations of Embodiments 1 to 3]

Each of Embodiments 1 to 3 indicates an example of the first aspect of the present invention, and it is a matter of course that: the first aspect of the present invention is not limited to the above embodiments; and various other embodiments are possible within the scope of the idea of the first aspect of the present invention.

For example, in each of Embodiments 1 to 3, the positioning members are provided in the lower jaw oral appliance and the stoppers are provided in the upper jaw oral appliance, but positioning members may be provided in an upper jaw oral appliance and stoppers may be provided in a lower jaw oral appliance. In this case, as a matter of course, a fixing portion and position adjustment sections are also provided in the upper jaw oral appliance. Also, in this case, the positioning members, the fixing portion, and the position adjustment sections are disposed further on the rear side and the stoppers are disposed further on the front side, and the rear surface and the rear end portion of each positioning member are deemed to be replaced with a front surface and a front end portion of each positioning member and the front surface and the front end portion of each stopper are deemed to be replaced with a rear surface and a rear end portion of each stopper.

Also, in each of Embodiments 1 to 3, the positioning members and the stoppers are provided on the outer side surface of the lower or upper jaw oral appliance, but positioning members and stoppers may be provided on an inner side surface of a lower or upper jaw oral appliance. In this case, as a matter of course, a fixing portion and position adjustment sections are also provided on the inner side surface of the lower or upper jaw oral appliance.

Also, in each of Embodiments 1 to 3, the upper jaw oral appliance body portion and the lower jaw oral appliance body portion each include a teeth row shape portion that can be fitted to a row of teeth. However, the upper jaw oral appliance body portion and the lower jaw oral appliance body portion may have a shape that is not a teeth row shape as long as the shape is a shape that receives a row of teeth, and each of the upper jaw oral appliance body portion and the lower jaw oral appliance body portion may only partly include a teeth row shape portion.

Also, the upper and lower jaw oral appliances may be each formed of a plurality of materials and may be formed by a combination of a part formed of an organic substance and a part formed of an inorganic substance.

Also, in each of Embodiments 1 to 3, each of the position adjustment sections adjusts a position of the relevant positioning member by means of adjustment of amounts of thread connection between the turnbuckle portion and the two screw rods using the turnbuckle mechanism, but for each of position adjustment sections, a jackscrew mechanism that can adjust a position of a positioning member by adjusting an amount of thread connection between a male screw and a female screw may be used. Also, each position adjustment section may move the relevant positioning member via a gear using a rack jack mechanism that can adjust a position of the positioning member fixed to a rack by adjusting an amount of rotation of the rack, and fix the positioning member via a screw. Each position adjustment section may adjust a position of a positioning member using another mechanism as long as such other mechanism can move the position of the positioning member and fix the positioning member at the adjusted position.

Also, in each of Embodiments 1 to 3, the front end portion of each stopper is inclined further rearward in the extension portion. However, each stopper may start being inclined further rearward above an extension portion or may be inclined further rearward from a certain point partway of the extension portion.

Also, in each of Embodiments 1 to 3, a gap is provided between each extension portion and the lower jaw oral appliance and a gap is provided between each protrusion portion and the upper jaw oral appliance to allow movement in the right-left direction of the upper jaw and the lower jaw of a user wearing the oral appliance. However, a configuration in which no gaps are provided between the aforementioned members and no movement in a right-left direction of the upper jaw and the lower jaw of a user wearing the oral appliance is allowed may be employed.

Also, in Embodiment 1, each of the wings is disposed at a position at which when the lower jaw oral appliance is displaced in the right-left direction upon biting and the extension portions thereby abut against the lower jaw oral appliance, the wing does not abut against the upper jaw oral appliance. Also, in Embodiment 3, each of the wings is disposed at a position at which when the lower jaw oral appliance is displaced in the right-left direction upon biting and the protrusion portions thereby abut against the upper jaw oral appliance, the wing does not abut against the upper jaw oral appliance. However, the wings may abut against the upper jaw oral appliance simultaneously with the extension portions abutting against the lower jaw oral appliance or the protrusion portions abutting against the upper jaw oral appliance.

Consequently, stress caused by displacement in the right-left direction is applied not only to the wings of the positioning members but also to the extension portions of the stoppers or the protrusion portions of the fixing portion and is thus distributed to both the wings and the extension portions or the protrusion portions. Therefore, only small stress is applied to the wings and damage of the position adjustment sections due to an increase of the stress applied to the wings, each of which is a point of effort of a second-class lever, in the position adjustment sections, each of which is a point of load of the second-class lever, is suppressed.

Consequently, displacement in the right-left direction of the lower jaw oral appliance is restricted by the wings and the extension portions or the protrusion portions simultaneously. Therefore, misalignment between the upper jaw oral appliance and the lower jaw oral appliance due to, for example, teeth grinding of a user wearing the oral appliance is effectively suppressed and the feeling of fitting is further improved.

Also, in Embodiment 1, where stress caused by displacement in the right-left direction is applied only to the extension portions of the stoppers, the thickness in the inside-outside direction of the wings of the positioning members is made to be smaller than that of the extension portions of the stoppers. Also, in Embodiment 3, where stress caused by displacement in the right-left direction is applied only to the protrusion portions of the fixing portion, the thickness in the inside-outside direction of the wings of the positioning members is made to be smaller than that of the protrusion portions of the fixing portion. However, positioning members may be disposed on the outer side relative to respective stoppers or a fixing portion to prevent the wings from abutting against an upper jaw oral appliance when a lower jaw oral appliance is displaced in a right-left direction. In this case, outer side surfaces of the positioning members and the wings thereof may fail to be substantially in plane with outer side surfaces of the respective stoppers and respective extension portion thereof or outer side surfaces of the fixing portion and protrusion portions thereof.

### [Example Configurations of Oral Appliance Relating to First Aspect]

The first aspect of the present invention provides
an oral appliance including:
a pair of upper and lower jaw oral appliances facing each other;
a pair of positioning members each being adjustable in position in a front-rear direction, the pair of positioning members being provided in one oral appliance of the upper jaw oral appliance and the lower jaw oral appliance; and
a pair of stoppers provided in another oral appliance of the upper jaw oral appliance and the lower jaw oral appliance, the pair of stoppers abutting against the respective positioning members and thereby restricting rearward displacement of the lower jaw oral appliances, and abutting against the one oral appliance and thereby restricting displacement in a right-left direction of the upper jaw oral appliance and the lower jaw oral appliance.

In the oral appliance, position adjustment sections for adjusting respective positions of the lower jaw oral appliance and the upper jaw oral appliance upon biting are less likely to be broken even by displacement in the right-left direction upon biting.

The first aspect of the present invention provides
the oral appliance in which each of the positioning members includes a wing that abuts against the relevant stopper on an outer side of the other oral appliance.

In the oral appliance, the stoppers and the wings abut against each other on an outer side of an upper jaw oral appliance body portion, enabling limiting rearward displacement of the lower jaw of a user.

The first aspect of the present invention provides
the oral appliance in which each of the wings is disposed at a position at which when the relevant stopper abuts against the one oral appliance as a result of movement in the right-left direction, the wing does not abut against the other oral appliance.

In the oral appliance, stress caused by displacement in the right-left direction is applied only to extension portions of the stoppers and is not applied to the positioning members. Therefore, the oral appliance enables suppression of damage of the position adjustment sections due to displacement in the right-left direction of the lower jaw oral appliance.

The first aspect of the present invention provides
the oral appliance in which each of the wings includes an inclined portion that abuts against the relevant stopper upon biting and facilitates movement in a mouth opening direction of the lower jaw oral appliance.

The oral appliance makes it easy for a user wearing the oral appliance to open the mouth.

The first aspect of the present invention provides
the oral appliance in which the positioning members have a height that is equal to or lower than a height of the one oral appliance.

In the oral appliance, the positioning members do not substantially abut against the upper jaw oral appliance upon displacement in the right-left direction. Therefore, stress caused by displacement in the right-left direction is less likely to be applied to the positioning members and damage of the position adjustment sections by the stress is thus suppressed.

The first aspect of the present invention provides
the oral appliance in which each of the stoppers includes an inclined portion that facilitates movement in the mouth opening direction of the lower jaw oral appliance.

The oral appliance makes it easy for the user wearing the oral appliance to open the mouth.

The first aspect according to the present invention provides
the oral appliance in which each of the stoppers includes an extension portion that abuts against the one oral appliance and has a height that is equal to or less than half a height of the stopper.

In the oral appliance, the extension portions abut against the one oral appliance and stress caused by displacement in the right-left direction is applied to the extension portions. Consequently, stress caused by displacement in the right-left direction is less applied to the positioning members, and thus, damage of the position adjustment sections due to displacement of the lower jaw oral appliance in the right-left direction can be suppressed.

The first aspect of the present invention provides the oral appliance in which the stoppers and the positioning members have respective shapes that, upon biting, allow respective end portions on the one oral appliance side of the stoppers and the positioning members to be spaced from each other.

The oral appliance facilitates movement in the mouth opening direction of the lower jaw oral appliance.

The first aspect of the present invention provides the oral appliance in which the stoppers and the positioning members have respective shapes that, upon biting or upon movement in the mouth opening direction of the lower jaw oral appliance, allow respective surfaces of the stoppers and the positioning members, the surfaces facing each other, to be spaced from each other on the one oral appliance side relative to an occlusal surface between the upper and lower jaw oral appliances.

The oral appliance further facilitates movement in the mouth opening direction of the lower jaw oral appliance.

The first aspect of the present invention provides the oral appliance in which each of the stoppers has a shape that is obtuse in a lower end of an end portion on the one oral appliance side of a front end portion of the stopper.

The oral appliance suppresses damage of the stoppers due to displacement in the right-left direction of the lower jaw oral appliance.

The first aspect of the present invention provides the oral appliance in which each of the stoppers has a shape that allows a projection area of projection of the stopper to the one oral appliance from a lateral side direction upon biting is 3 mm² or more.

The oral appliance suppresses damage of the stoppers due to displacement in the right-left direction of the lower jaw oral appliance.

The first aspect of the present invention provides
the oral appliance in which abutment portions of the one oral appliance, each of the abutment portions abutting against the respective stoppers has a substantially flat shape.

In the oral appliance, the lower jaw oral appliance can be moved forward without movement of the lower jaw oral appliance being restricted by the extension portions.

The first aspect of the present invention provides
the oral appliance in which
respective abutment surfaces of the pair of stoppers, the abutment surfaces abutting against the one oral appliance, are substantially parallel to each other, and
respective abutment surfaces of the one oral appliance, the abutment surfaces abutting against the pair of stoppers, are substantially parallel to each other.

In the oral appliance, the lower jaw oral appliance can be moved forward without movement of the lower jaw oral appliance being restricted by the extension portions.

The first aspect of the present invention provides
the oral appliance in which the positioning members are provided in the lower jaw oral appliance.

In the oral appliance, protrusion portions are provided at respective positions corresponding to the rear parts, in which the skin more easily stretches and shrinks, of the inner cheeks of the user wearing the oral appliance, and thus, a feeling of fitting the user wearing the oral appliance has is improved.

The first aspect of the present invention provides
the oral appliance in which the stoppers are bonded to the upper or lower jaw oral appliance.

The oral appliance facilitates manufacture of stoppers having various shapes.

The first aspect of the present invention provides
the oral appliance in which the stoppers are integrated with the upper or lower jaw oral appliance.

The oral appliance is easy to manufacture.

### 2. Oral Appliance Relating to Second Aspect

In an oral appliance relating to a second aspect of the present invention, stress applied to each position adjustment section, which is a point of load of a second-class lever, is distributed in a front-rear direction of the oral appliance to suppress damage of the position adjustment sections due to displacement in a right-left direction.

### [Embodiment 4]

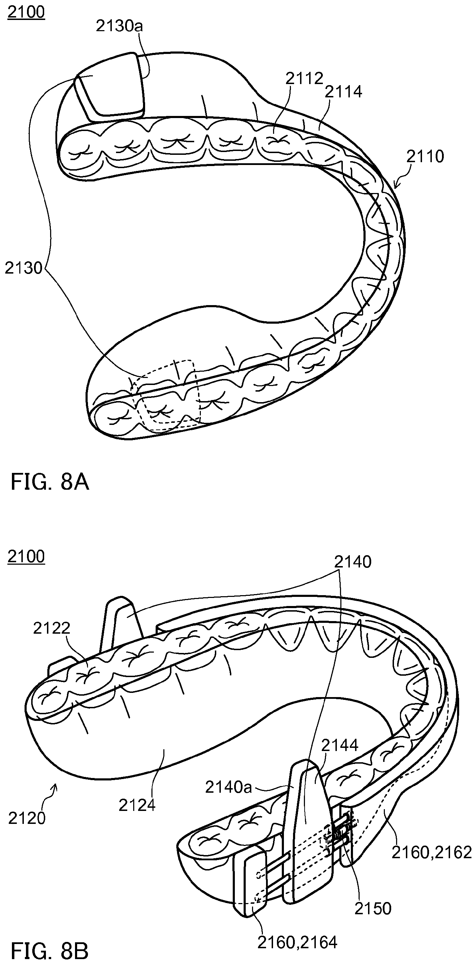

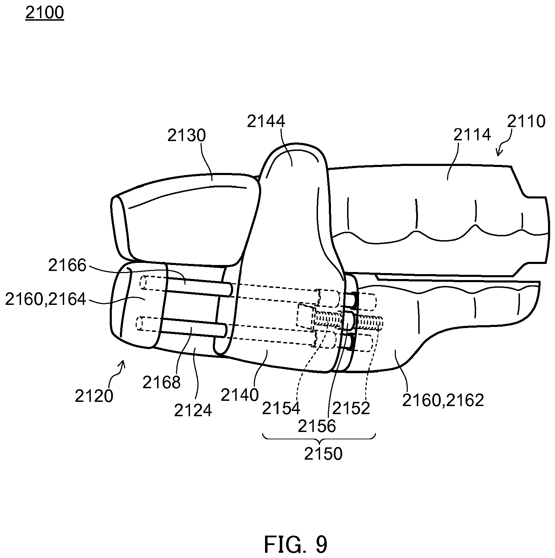

Oral appliance 2100 according to Embodiment 4 of the present invention includes a pair of upper and lower jaw oral appliances facing each other as illustrated in FIGS. 8A and 8B, which are schematic perspective views thereof, and FIG. 9, which is a side view thereof, and upper jaw oral appliance 2110 can be fitted to the upper jaw of a user and lower jaw oral appliance 2120 can be fitted to the lower jaw of the user.

As illustrated in FIG. 8A, upper jaw oral appliance 2110 includes upper jaw oral appliance body portion 2114 with teeth row shape portion 2112 formed therein, teeth row shape portion 2112 being able to be fitted to a row of teeth of the upper jaw of the user, and on an outer side surface thereof, a pair of right and left stoppers 2130 is provided.

As illustrated in FIG. 8B, lower jaw oral appliance 2120 includes lower jaw oral appliance body portion 2124 with teeth row shape portion 2122 formed therein, teeth row shape portion 2122 being able to be fitted to a row of teeth of the lower jaw of the user, and on an outer side surface thereof, a pair of right and left positioning members 2140, a position of each of the pair of right and left positioning members 2140 being adjustable in a front-rear direction, is provided.

Each stopper 2130 is provided at a position on the outer side surface of the upper jaw oral appliance, at which stopper 2130 is located on the rear side relative to the relevant positioning member when the user wearing upper jaw oral appliance 2110 and lower jaw oral appliance 2120 bites.

Each stopper 2130 may have a height that is equal to or lower than a height of upper jaw oral appliance 2110. Note that the height of upper jaw oral appliance 2110 may vary depending on the position. In this case, the height of stopper 2130 may only be equal to or lower than a height of a part, in which stopper 2130 is provided, of upper jaw oral appliance body portion 2114.

The position of each positioning member 2140 can be adjusted in the front-rear direction along a pair of front and rear guide sections 2160 provided on the outer side surface of lower jaw oral appliance body portion 2124, by relevant position adjustment section 2150 provided on the outer side surface of lower jaw oral appliance body portion 2124. Upon biting, rear surface 2140a of each positioning member 2140 abuts against front surface 2130a of relevant stopper 2130 and thereby limits rearward displacement of lower jaw oral appliance 2120.

In this case, as illustrated in FIG. 9, the position of each positioning member 2140 can be adjusted to a position at which rear surface 2140a of positioning member 2140 and front surface 2130a of relevant stopper 2130 abut against each other upon biting. The abutment between positioning members 2140 and stoppers 2130 limits rearward displacement of lower jaw oral appliance 2120 upon biting and thereby limits rearward displacement of the lower jaw of the user wearing oral appliance 2100. Consequently, positioning members 2140 prevents narrowing of the airway of the user wearing oral appliance 2100 due to rearward displacement of the lower jaw of the user, enabling suppression of pauses in breathing or infrequent breathing during sleep.

Also, as a result of the positions of positioning members 2140 being adjusted rearward, upon biting, positioning members 2140 can limit rearward displacement of lower jaw oral appliance 2120 while moving lower jaw oral appliance 2120 forward. Consequently, positioning members 2140 move the lower jaw of the user wearing oral appliance 2100 forward to open the airway of the user more largely, enabling more effective suppression of pauses in breathing or infrequent breathing during sleep.

Each positioning member 2140 includes wing 2144 that protrudes upward from lower jaw oral appliance body portion 2124. A rear surface of wing 2144 (which is also rear surface 2140a of positioning member 2140, rear surface 2140a abutting against stopper 2130) is substantially parallel to front surface 2130a of stopper 2130, and upon biting, abuts against front surface 2130a of stopper 2130 on the outer side of upper jaw oral appliance body portion 2114 and wing 2144 thereby limits rearward displacement of the lower jaw of the user.

Each position adjustment section 2150 includes male-threaded screw rod 2152 including an end fixed to one (front fixing portion 2162) of the relevant pair of guide sections 2160 provided on the outer side surface of lower jaw oral appliance body portion 2124, male-threaded screw rod 2154 including an end fixed to relevant positioning member 2140, and turnbuckle portion 2156 including a screw hole to which respective other ends of screw rods 2152 and 2154 are threadably connected. Position adjustment section 2150 can adjust the position of positioning member 2140 fixed to screw rod 2152, by inserting a rotation pin into a pin hole provided in turnbuckle portion 2156 and rotating turnbuckle portion 2156 to adjust amounts of the thread connection between turnbuckle portion 2156 and screw rods 2152 and 2154.

Guide sections 2160 include a front-rear pair of front fixing portion 2162 and rear fixing portion 2164 provided on the outer side surface of lower jaw oral appliance body portion 2124 and two support bars 2166 and 2168, respective opposite ends of which are supported by front fixing portion 2162 and rear fixing portion 2164, respectively.

Front fixing portion 2162 and rear fixing portion 2164 are bonded to the outer side surface of lower jaw oral appliance body portion 2124 and support the respective opposite ends of support bars 2166 and 2168. Front fixing portion 2162 and rear fixing portion 2164 may only be provided so as to form a front-rear pair as oral appliance 2100 is viewed from a lateral side direction, and for example, as illustrated in FIG. 8B, right and left front fixing portions 2162 may be joined and thereby integrated in front of the row of teeth.

Support bars 2166 and 2168 extend through respective through holes provided in the front-rear direction inside positioning member 2140 and movably support positioning member 2140. Consequently, stress applied to each wing 2144, which is a point of effort of a second-class lever, when the lower jaw oral appliance is displaced in a right-left direction, is distributed also to between relevant positioning member 2140 and rear fixing portion 2164 by relevant support bars 2166 and 2168 in relevant guide section 2160, which is a point of load of the second-class lever, and is prevented from being applied only to between relevant positioning member 2140 and front fixing portion 2162.

From the perspective of the stress being more broadly distributed for further suppression of damage of position adjustment sections 2150, a space between front fixing portion 2162 and each rear fixing portion 2164 is preferably wide, and for example, rear fixing portions 2164 are preferably disposed on respective rear ends of lower jaw oral appliance body portion 2124.

Stoppers 2130 and wings 2144 have respective shapes that when lower jaw oral appliance 2120 is moved in a mouth opening direction during wearing, prevent stoppers 2130 from limiting movement of wings 2144. More specifically, each stopper 2130 may include an inclined portion that facilitates movement in the mouth opening direction of lower jaw oral appliance 2120. Also, each wing 2144 may include an inclined portion in a rear end surface thereof, the inclined portion facilitating movement in the mouth opening direction of lower jaw oral appliance 2120. In other words, each stopper 2130 may include a rearward inclined surface in a front end portion thereof, the inclined surface including an upper end positioned further on the front side and a lower end positioned further on the rear side, and each wing 2144 may include a rearward inclined surface in a rear end portion thereof, the inclined surface including an upper end positioned further on the front side and a lower end positioned further on the rear side. The inclined portions of stoppers 2130 and the inclined portions of wings 2144 abut against each other upon biting.

When lower jaw oral appliance 2120 is moved in the mouth opening direction during wearing, lower jaw oral appliance 2120 is moved on a circular arc-like path. In such movement, movement of wings 2144 is not limited by stoppers 2130 as a result of the front end portions of stoppers 2130 and the rear end portions of wings 2144 including the respective inclined surfaces described above, making it easy for the user wearing oral appliance 2100 to open the mouth.

According to the present embodiment, stress applied to each wing 2144, which is a point of effort of a second-class lever, when the lower jaw oral appliance is displaced in the right-left direction is distributed to between front fixing portion 2162 and rear fixing portion 2164 by relevant support bars 2166 and 2168 in relevant guide section 2160, which is a point of load of the second-class lever. Consequently, stress that is conventionally applied only to between positioning members 2140 and front fixing portion 2162 is distributed also to between positioning members 2140 and rear fixing portions 2164, and thus, even if the lower jaw oral appliance is displaced in the right-left direction, position adjustment sections 2150 are less likely to be damaged.

Also, according to the present embodiment, since the guide sections abut against the parts on the lower jaw side of the cheeks on which the skin more easily stretches and shrinks, a feeling of fitting a user wearing oral appliance 2100 has is more favorable.

### [Embodiment 5]

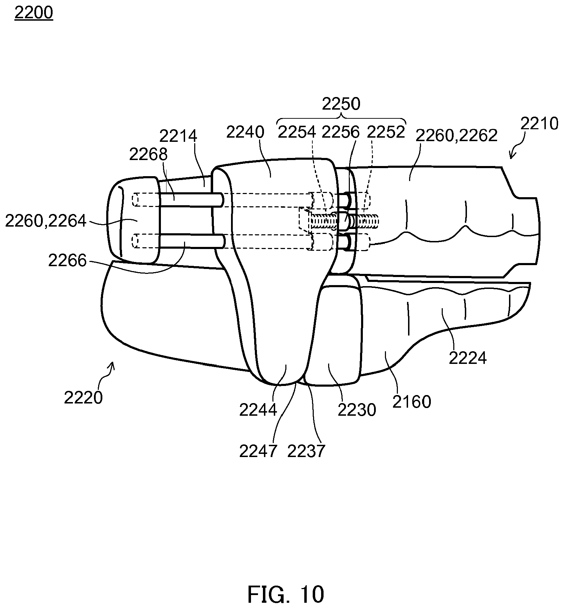

Oral appliance 2200 according to Embodiment 5 of the present invention includes a pair of upper and lower jaw oral appliances facing each other as illustrated in FIG. 10, which is a schematic side view thereof, upper jaw oral appliance 2210 can be fitted to the upper jaw of a user, and lower jaw oral appliance 2220 can be fitted to the lower jaw of the user. In the below, description of components that are in common with Embodiment 4 may be omitted.

In the present embodiment, a pair of right and left positioning members 2240 which are adjustable in position in a front-rear direction is provided on an outer side surface of upper jaw oral appliance body portion 2214, and a pair of right and left stoppers 2230 is provided on an outer side surface of lower jaw oral appliance body portion 2224.

Each stopper 2230 is provided at a position on the outer side surface of the lower jaw oral appliance, at which stopper 2230 is located on the front side relative to the relevant positioning member upon biting, and upon biting, a rear surface of stopper 2230 abuts against a front surface of relevant positioning member 2240 and stopper 2230 thereby limits rearward displacement of lower jaw oral appliance 2220.

Each stopper 2230 has a height that is equal to or lower than a height of lower jaw oral appliance 2220. Note that the height of lower jaw oral appliance 2220 may vary depending on the position. In this case, each stopper 2230 may only have a height that is equal to or lower than a height, in a part in which stopper 2230 is provided, of upper jaw oral appliance body portion 2214.

A position of each positioning member 2240 can be adjusted in the front-rear direction along a front-rear pair of guide sections 2260 provided on the outer side surface of lower jaw oral appliance body portion 2224, by relevant position adjustment section 2250 provided on the outer side surface of lower jaw oral appliance body portion 2224.

In this case, as illustrated in FIG. 10, the position of each positioning member 2240 can be adjusted to a position at which a front surface of positioning member 2240 and a rear surface of relevant stopper 2230 abut against each other upon biting. The abutment between positioning members 2240 and stoppers 2230 limits rearward displacement of lower jaw oral appliance 2220 upon biting and thus limits rearward displacement of the lower jaw of a user wearing oral appliance 2200.

Also, as a result of the positions of positioning members 2240 being adjusted forward, upon biting, positioning members 2240 can limit rearward displacement of lower jaw oral appliance 2220 while moving lower jaw oral appliance 2220 forward.

Also, each positioning member 2240 includes wing 2244 that protrudes downward from upper jaw oral appliance body portion 2214. A front surface of wing 2244 (which is also the front surface of positioning member 2240, the front surface abutting against stopper 2230) is substantially parallel to the rear surface of stopper 2230, upon biting, abuts against the rear surface of stopper 2230 on the outer side of lower jaw oral appliance body portion 2224, and wing 2244 thereby limits rearward displacement of the lower jaw of the user.

Each position adjustment section 2250 includes male-threaded screw rod 2252 including an end fixed to one (front fixing portion 2262) of the relevant pair of guide sections 2260 provided on the outer side surface of upper jaw oral appliance body portion 2214, male-threaded screw rod 2254 including an end fixed to relevant positioning member 2240, and turnbuckle portion 2256 including a screw hole to which respective other ends of screw rods 2252 and 2254 are threadably connected.

Guide sections 2260 include a front-rear pair of front fixing portion 2262 and rear fixing portion 2264 provided on the outer side surface of upper jaw oral appliance body portion 2214 and two support bars 2266 and 2268, respective opposite ends of which are supported by front fixing portion 2262 and rear fixing portion 2264, respectively.

Front fixing portion 2262 and rear fixing portion 2264 are bonded to the outer side surface of upper jaw oral appliance body portion 2214 and support the respective opposite ends of support bars 2266 and 2268.

Support bars 2266 and 2268 extend through respective through holes provided in the front-rear direction inside positioning member 2240 and movably support positioning member 2240. Consequently, stress applied to each wing 2244, which is a point of effort of a second-class lever, when the lower jaw oral appliance is displaced in a right-left direction, is distributed also to between relevant positioning member 2240 and relevant rear fixing portion 2264 by relevant support bars 2266 and 2268 in relevant guide section 2260, which is a point of load of the second-class lever, and is prevented from being applied only to between relevant positioning member 2240 and front fixing portion 2262.

From the perspective of the stress being more broadly distributed for further suppression of damage of position adjustment sections 2250, a space between front fixing portion 2262 and each rear fixing portion 2264 is preferably wide, and for example, rear fixing portions 2264 are preferably disposed on respective rear ends of upper jaw oral appliance body portion 2214.

Stoppers 2230 and wings 2244 have respective shapes that when lower jaw oral appliance 2220 is moved in a mouth opening direction during wearing, prevent stoppers 2230 from limiting movement of wings 2244. More specifically, each stopper 2230 may include an inclined portion that facilitates movement in the mouth opening direction of lower jaw oral appliance 2220. Also, each wing 2244 may include an inclined portion in a front end surface thereof, the inclined portion facilitating movement in the mouth opening direction of lower jaw oral appliance 2220. In other words, each stopper 2230 may include a rearward inclined surface in a rear end portion thereof, the inclined surface including an upper end positioned further on the front side and a lower end positioned further on the rear side, and each wing 2244 may include a rearward inclined surface in a front end portion thereof, the inclined surface including an upper end positioned further on the front side and a lower end positioned further on the rear side. The inclined portions of stoppers 2230 and the inclined portions of wings 2244 abut against each other upon biting.

When lower jaw oral appliance 2220 is moved in the mouth opening direction during wearing, lower jaw oral appliance 2220 is moved on a circular arc-like path. In such movement, movement of wings 2244 is not limited by stoppers 2230 as a result of the rear end portions of stoppers 2230 and the front end portions of wings 2244 including the respective inclined surfaces, making it easy for the user wearing oral appliance 2200 to open the mouth.

Lower end 2247 of the front end portion of each wing 2244 and lower end 2237 of the rear end portion of relevant stopper 2230 are preferably spaced from each other upon biting. Consequently, wings 2244 are less likely to limit movement in the mouth opening direction of lower jaw oral appliance 2220 during wearing, more facilitating movement in the mouth opening direction of lower jaw oral appliance 2220.

In this case, from the perspective of suppression of damage of wings 2244 due to displacement in the right-left direction of lower jaw oral appliance 2220, lower end 2247 of the front end portion of each wing 2244 is preferably obtuse. Also, likewise, from the perspective of suppression of damage of wings 2244, the projection area of projection of each wing 2244 to lower jaw oral appliance body portion 124 from a lateral side direction upon biting is preferably 3 mm² or more. An upper limit of the projection area is not specifically provided. The projection area is, for example, preferably 3 mm² to 1000 mm², more preferably 3 mm² to 500 mm², further preferably 3 mm² to 450 mm², particularly preferably 50 mm² to 450 mm².

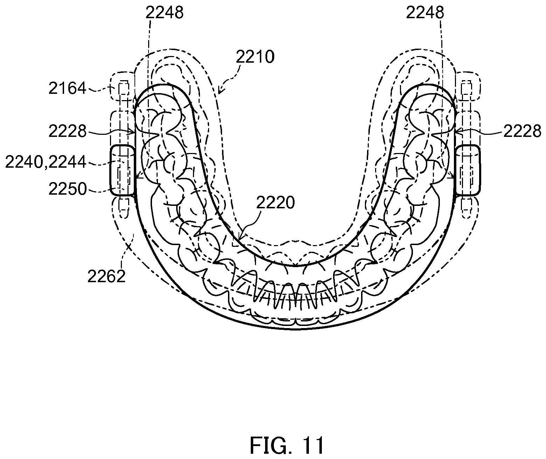

Also, a shape and disposition of each of portions of wings 2244, the portions abutting against lower jaw oral appliance body portion 2224, and portions of lower jaw oral appliance 2220, the portions abutting against respective wings 2244, are those that do not limit forward movement of lower jaw oral appliance 2220. More specifically, as illustrated in FIG. 11, which is a schematic plan view of oral appliance 2200 (the upper jaw oral appliance, the positioning members, the position adjustment sections, and the guide sections are each indicated by a dashed line and the lower jaw oral appliance and the stoppers are each indicated by a solid line), each of abutment portions 2228 of lower jaw oral appliance body portion 2224, abutment portions 2228 abutting against respective wings 2244, have a substantially flat shape. Also, a pair of abutment surfaces 2248 of the pair of wings 2244, abutment surfaces 2248 abutting against lower jaw oral appliance 2220, are substantially parallel to each other and abutment portions 2228 positioned in right and left parts of the outer side surface of lower jaw oral appliance body portion 2224, abutment portions 2228 abutting against the pair of wings 2244, are substantially parallel to each other.

Consequently, lower jaw oral appliance 2220 can be moved forward without movement of lower jaw oral appliance 2220 being limited by wings 2244. In this case, for example, a thickness in the right-left direction of each part, in the vicinity of the back teeth, of the lower jaw oral appliance body portion 2224 is larger on the front side (for example, a part, in the vicinity of the first molar tooth (tooth number: 6), of the outer side surface) and is smaller on the rear side (for example, a part, in the vicinity of the second molar tooth (tooth number: 7) or the third molar tooth (tooth number: 8), of the outer side surface).

According to the present embodiment, the positioning members, the position adjustment sections, and the guide sections are provided on the upper jaw side, which does not move largely upon opening of the mouth, and thus, rubbing between these members and the cheeks is less likely to occur, and the user wearing oral appliance 2100 less feels discomfort when the user opens the mouth.

### [Variations of Embodiments 4 and 5]

Each of Embodiments 4 and 5 indicates an example of the second aspect of the present invention, and it is a matter of course that: the second aspect of the present invention is not limited to the above embodiments; and various other embodiments are possible within the scope of the idea of the second aspect of the present invention.

For example, in each of Embodiments 4 and 5, the positioning members, the stoppers, the position adjustment sections, and the guide sections are provided on the outer side surface of the lower or upper jaw oral appliance, but positioning members, stoppers, position adjustment sections, and guide sections may be provided on an inner side surface of a lower or upper jaw oral appliance.

Also, in each of Embodiments 4 and 5, the front fixing portion and the rear fixing portions are bonded to the lower or upper jaw oral appliance body portion, but may be detachably attachable to the lower or upper jaw oral appliance body portion.

Also, in each of Embodiments 4 and 5, the upper jaw oral appliance body portion and the lower jaw oral appliance body portion each include a teeth row shape portion that can be fitted to a row of teeth. However, the upper jaw oral appliance body portion and the lower jaw oral appliance body portion may have a shape that is not a teeth row shape as long as the shape is a shape that receives a row of teeth, and each of the upper jaw oral appliance body portion and the lower jaw oral appliance body portion may only partly include a teeth row shape portion.

Also, the upper and lower jaw oral appliances may be each formed of a plurality of materials and may be formed by a combination of a part formed of an organic substance and a part formed of an inorganic substance.

Also, in each of Embodiments 4 and 5, each of the position adjustment sections adjusts a position of the relevant positioning member by means of adjustment of amounts of thread connection between the turnbuckle portion and the two screw rods using the turnbuckle mechanism, but for each of position adjustment sections, a jackscrew mechanism that can adjust a position of a positioning member by adjusting an amount of thread connection between a male screw and a female screw may be used. Also, each position adjustment section may move the relevant positioning member via a gear using a rack jack mechanism that can adjust a position of a positioning member fixed to a rack by adjusting an amount of rotation of the rack, and fix the positioning member via a screw. Each position adjustment section may adjust a position of a positioning member using another mechanism as long as such other mechanism can move the position of the positioning member and fix the positioning member at the adjusted position.

Also, in Embodiment 5, the front end portion of each positioning member is inclined rearward in the wing. However, each positioning member may start being inclined rearward above a wing or may be inclined rearward from a certain point partway of the wing.

Also, in each of Embodiments 4 and 5, a gap is provided between each wing and the lower or upper jaw oral appliance to allow movement in the right-left direction of the upper jaw and the lower jaw of a user wearing the oral appliance. However, a configuration in which no gaps are provided between the aforementioned members and no movement in a right-left direction of the upper jaw and the lower jaw of a user wearing the oral appliance is allowed may be employed.

### [Example Configurations of Oral Appliance Relating to Second Aspect]

The second aspect of the present invention provides
an oral appliance including:
a pair of upper and lower jaw oral appliances facing each other;
a pair of positioning members, a position of each of the pair of positioning members being adjustable in a front-rear direction along a guide section including opposite ends supported by a pair of fixing portions provided in one oral appliance of the upper and lower jaw oral appliances; and
a pair of stoppers provided in another oral appliance of the upper and lower jaw oral appliances, the pair of stoppers abutting against the respective positioning members and thereby restricting rearward displacement of the lower jaw oral appliance.

In the oral appliance, position adjustment sections for adjusting respective positions of the lower jaw oral appliance and the upper jaw oral appliance upon biting are less likely to be broken even by displacement in a right-left direction upon biting.

The second aspect of the present invention provides
the oral appliance in which each of the positioning members includes a wing that abuts against the relevant stopper on an outer side of the other oral appliance.

In the oral appliance, the stoppers and the wings abut against each other on an outer side of an upper jaw oral appliance body portion, enabling limiting rearward displacement of the lower jaw of a user.

The second aspect of the present invention provides
the oral appliance in which the stoppers have a height that is equal to or lower than a height of the other oral appliance.

In the oral appliance, each stopper is less likely to limit position adjustment of the relevant positioning member.

The second aspect of the present invention provides
the oral appliance in which one of the pair of fixing portions is disposed at a rear end of the one oral appliance.

The oral appliance enables more effective suppression of damage of the position adjustment sections by more broadly distributing stress applied to the wings when the lower jaw oral appliance is displaced in the right-left direction.

The second aspect of the present invention provides
the oral appliance in which the positioning members are provided in the lower jaw oral appliance.

The oral appliance enables more effective suppression of damage of the position adjustment sections by more broadly distributing stress applied to the wings when the lower jaw oral appliance is displaced in the right-left direction.

Since protrusion portions are provided at respective positions corresponding to the parts, in which the skin more easily stretches and shrinks, of the inner cheeks of the user wearing the oral appliance, a feeling of fitting the user wearing the oral appliance has is improved.

### 3. Oral Appliance Relating to Third Aspect

In an oral appliance relating to the third aspect of the present invention, a distance between each position adjustment section, which is a point of load of a second-class lever, and a fixing portion, which is a fulcrum of the second-class lever, is decreased to suppress damage of the position adjustment sections due to an excessive increase of stress applied to each upward flange portion, which is a point of effort of the second-class lever, in the relevant position adjustment section, which is a point of load of the second-class lever.

### [Embodiment 6]

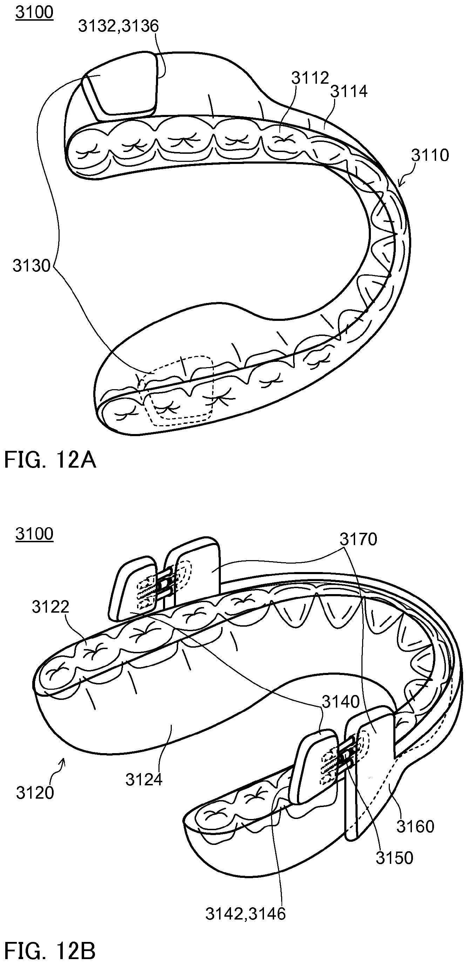

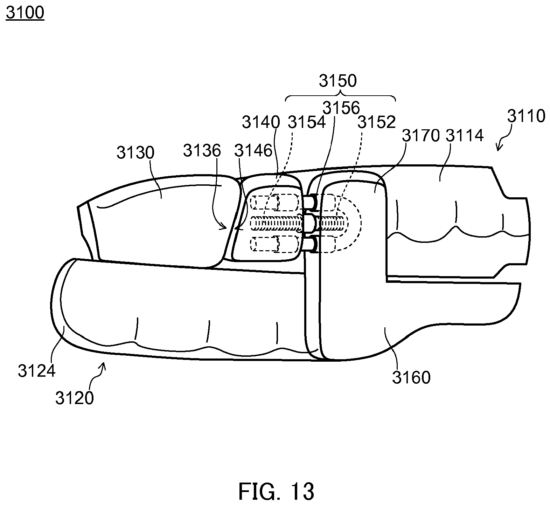

Oral appliance 3100 according to Embodiment 6 of the present invention includes a pair of upper and lower jaw oral appliances facing each other as illustrated in FIGS. 12A and 12B, which are schematic perspective views thereof, and FIG. 13, which is a side view thereof, and upper jaw oral appliance 3110 can be fitted to the upper jaw of a user and lower jaw oral appliance 3120 can be fitted to the lower jaw of the user.

As illustrated in FIG. 12A, upper jaw oral appliance 3110 includes upper jaw oral appliance body portion 3114 with teeth row shape portion 3112 formed therein, teeth row shape portion 3112 being able to be fitted to a row of teeth of the upper jaw of the user, and on an outer side surface thereof, a pair of right and left stoppers 3130 is provided.

As illustrated in FIG. 12B, lower jaw oral appliance 3120 includes lower jaw oral appliance body portion 3124 with teeth row shape portion 3122 formed therein, teeth row shape portion 3122 being able to be fitted to a row of teeth of the lower jaw of the user, and on an outer side surface, a pair of right and left protrusion portions 3170 fixed to lower jaw oral appliance body portion 3124 by fixing portion 3160 is provided. Each protrusion portion 3170 supports an end of relevant guide section 3150, and each guide section 3150 supports relevant positioning member 3140 in such a manner that positioning member 3140 is movable in a front-rear direction.

Each stopper 3130 is provided at a position on the outer side surface of upper jaw oral appliance 3110, at which stopper 3130 is located on the rear side relative to relevant protrusion portion 3170 and relevant positioning member 3140 when the user wearing upper jaw oral appliance 3110 and lower jaw oral appliance 3120 bites.

Stoppers 3130 have a height that is equal to or lower than a height of upper jaw oral appliance 3110. Note that the height of upper jaw oral appliance 3110 may vary depending on the position. In this case, the height of each stopper 3130 is preferably equal to or lower than a height of a part, in which stopper 3130 is provided, of upper jaw oral appliance body portion 3114. Consequently, even if lower jaw oral appliance 3120 is displaced in a right-left direction upon opening of the mouth, each stopper 3130 and relevant positioning member 3140 are less likely to abut against each other and damage of guide sections 3150 due to application of stress caused by the abutment to positioning members 3140 is thus less likely to occur.

Each protrusion portion 3170 protrudes from fixing portion 3160 provided on the outer side surface of lower jaw oral appliance 3120 toward upper jaw oral appliance 3110.

Each guide section 3150 is supported by relevant protrusion portion 3170 at a position that is lateral to upper jaw oral appliance 3110 upon biting. In other words, each guide section 3150 is disposed at a position at which entire guide section 3150 is located above an upper end of lower jaw oral appliance 3120 upon biting. In still other words, each guide section 3150 is disposed at a position at which entire guide section 3150 is located above a lower end of upper jaw oral appliance 3110 upon biting. In this case, also, a height of each guide section 3150 may only be above an upper end of a part, in which guide section 3150 is provided, of lower jaw oral appliance body portion 3124 and be above a lower end of a part, in which guide section 3150 is provided, of upper jaw oral appliance body portion 3114.

Each guide section 3150 includes male-threaded screw rod 3152 including an end fixed to relevant protrusion portion 3170, male-threaded screw rod 3154 including an end fixed to positioning member 3140, and turnbuckle portion 3156 including a screw hole to which respective other ends of screw rods 3152 and 3154 are threadably connected. Guide section 3150 can adjust a position of positioning member 3140 fixed to and supported by screw rod 3154, by inserting a rotation pin into a pin hole provided in turnbuckle portion 3156 and rotating turnbuckle portion 3156 to adjust amounts of the thread connection between turnbuckle portion 3156 and screw rods 3152 and 3154.

As illustrated in FIG. 13, the position of each positioning member 3140 can be adjusted to a position at which rear surface 3142 of positioning member 3140 and front surface 3132 of relevant stopper 3130 abut against each other upon biting, along relevant guide section 3150. The abutment between positioning members 3140 and stoppers 3130 limits rearward displacement of lower jaw oral appliance 3120 upon biting and thereby limits rearward displacement of the lower jaw of the user wearing oral appliance 3100. Consequently, oral appliance 3100 prevents narrowing of the airway of the user wearing oral appliance 3100 due to rearward displacement of the lower jaw of the user, enabling suppression of pauses in breathing or infrequent breathing during sleep.

Also, the position of each positioning member 3140 is adjusted rearward along relevant guide section 3150, enabling, upon biting, limiting rearward displacement of lower jaw oral appliance 3120 while moving lower jaw oral appliance 3120 forward. Consequently, oral appliance 3100 allows the lower jaw of the user wearing oral appliance 3100 to be moved forward and thus allows the airway of the user to open more largely, enabling more effective suppression of pauses in breathing or infrequent breathing during sleep.

The position of each positioning member 3140 is adjusted in the front-rear direction at a position at which positioning member 3140 is located lateral to upper jaw oral appliance 3110 upon biting. In other words, each positioning member 3140 is disposed at a position at which entire positioning member 3140 is located above a lower end of upper jaw oral appliance 3110 upon biting. In still other words, each positioning member 3140 is disposed at a position at which entire positioning member 3140 is located above an upper end of lower jaw oral appliance 3120 upon biting. Consequently, for example, when lower jaw oral appliance 3120 is displaced in the right-left direction upon opening of the mouth, for example, a corner portion or a side portion in the right-left direction of each positioning member 3140 is less likely to abut against relevant stopper 3130 and thus damage of guide sections 3150 by outward stress that can be applied by the abutment can further be suppressed.

Also, positioning members 3140 preferably have a height that is equal to or lower than a height of upper jaw oral appliance 3110. Consequently, each positioning member 3140 can easily be disposed at a position at which positioning member 3140 is located lateral to upper jaw oral appliance 3110 upon biting.

Each protrusion portion 3170 may be provided at a position at which when lower jaw oral appliance 3120 is displaced in the right-left direction, protrusion portion 3170 abuts against upper jaw oral appliance body portion 3114 and thereby restricts displacement in the right-left direction of lower jaw oral appliance 3120. Consequently, stress caused by the displacement in the right-left direction is applied also to protrusion portions 3170 and thereby distributed, and stress applied to positioning members 3140 thus becomes smaller. Therefore, such configuration enables suppression of damage of guide sections 3150 due to displacement in the right-left direction of lower jaw oral appliance 3120.

In this case, each positioning member 3140 may be provided at a position at which when lower jaw oral appliance 3120 is displaced in the right-left direction, positioning member 3140 does not abut against upper jaw oral appliance body portion 3114. Consequently, a majority of stress caused by displacement in the right-left direction is mainly applied to protrusion portions 3170 and stress applied to positioning members 3140 thus becomes smaller. Therefore, such configuration enables more effective suppression of damage of guide section 3150 due to displacement in the right-left direction of lower jaw oral appliance 3120.

For example, each positioning member 3140 has a thickness in an inside-outside direction thereof, the thickness being smaller than that of protrusion portions 3170, and is provided at a position at which an outer side surface of positioning member 3140 is substantially in plane with an outer side surface of relevant protrusion portion 3170. Consequently, a space between each positioning member 3140 and upper jaw oral appliance body portion 3114 upon biting is larger than a space between each protrusion portion 3170 and upper jaw oral appliance body portion 3114 upon biting.

Stoppers 3130 and positioning members 3140 may have respective shapes that when lower jaw oral appliance 3120 is moved in a mouth opening direction during wearing, prevent positioning members 3140 from limiting movement of stoppers 3130. More specifically, each stopper 3130 may include inclined portion 3136 that abuts against relevant positioning member 3140 upon biting and facilitates movement in the mouth opening direction of lower jaw oral appliance 3120. Also, each positioning member 3140 may include inclined portion 3146 that abuts against relevant stopper 3130 upon biting and facilitates movement in the mouth opening direction of lower jaw oral appliance 3120. In other words, each stopper 3130 may include a rearward inclined surface in a front end portion thereof, the inclined surface including an upper end positioned further on the front side and a lower end positioned further on the rear side, and each positioning member 3140 may include a rearward inclined surface in a rear end portion thereof, the inclined surface including an upper end positioned further on the front side and a lower end positioned further on the rear side.

When lower jaw oral appliance 3120 is moved in the mouth opening direction during wearing, lower jaw oral appliance 3120 is moved on a circular arc-like path. In such movement, movement of positioning members 3140 is not limited by stoppers 3130 as a result of the front end portions of stoppers 3130 and the rear end portions of positioning members 3140 including the respective inclined surfaces described above, making it easy for the user wearing oral appliance 3100 to open the mouth.

Stoppers 3130 may be detachably attachable to upper jaw oral appliance body portion 3114 but may also be bonded to upper jaw oral appliance body portion 3114. Alternatively, stoppers 3130 may be integrated with the outer side surface of upper jaw oral appliance body portion 3114.

Fixing portion 3160 may be detachably attachable to lower jaw oral appliance body portion 3124 but may be bonded to lower jaw oral appliance body portion 3124. If fixing portion 3160 is bonded to lower jaw oral appliance body portion 3124, misalignment of protrusion portions 3170 and positioning members 3140 is less likely to occur during wearing.

Protrusion portions 3170 may be detachably attachable to fixing portion 3160, but may be bonded to fixing portion 3160 or may be integrated with fixing portion 3160. Where protrusion portions 3170 are bonded to or integrated with fixing portion 3160, misalignment between protrusion portions 3170 and fixing portion 3160 is less likely to occur during wearing.

According to the present embodiment, even if lower jaw oral appliance 3120 is displaced in the right-left direction upon opening of the mouth, positioning members 3140 and stoppers 3130 are less likely to abut against each other and thus damage of guide sections 3150 by outward stress that can be applied by the abutment can further be suppressed.

Also, according to the present embodiment, even if positioning members 3140 abut against lower jaw oral appliance 3120 when lower jaw oral appliance 3120 is displaced in the right-left direction, stress that causes positioning members 3140 to move in the right-left direction is less likely to be applied. Therefore, stress applied to positioning members 3140 due to displacement in the right-left direction become smaller or no such stress is applied, and thus damage of guide sections 3150 by such stress is suppressed.

Also, each protrusion portion 3170, relevant guide section 3150, and relevant positioning member 3140 can form a second-class lever with the protrusion portion as a fulcrum, a portion of abutment between positioning member 3140 and upper jaw oral appliance body portion 3114 as a point of effort, and guide section 3150 as a point of load. In this case, according to the present embodiment, a distance between each protrusion portion 3170, which is a fulcrum of a second-class lever, and relevant guide section 3150, which is a point of load of the second-class lever, can be decreased, and thus, damage of guide sections 3150 due to an excessive increase of stress applied to each positioning member 3140, which is a point of effort of a second-class lever, in relevant guide section 3150, which is a point of load of the second-class lever, can be suppressed.

### [Embodiment 7]

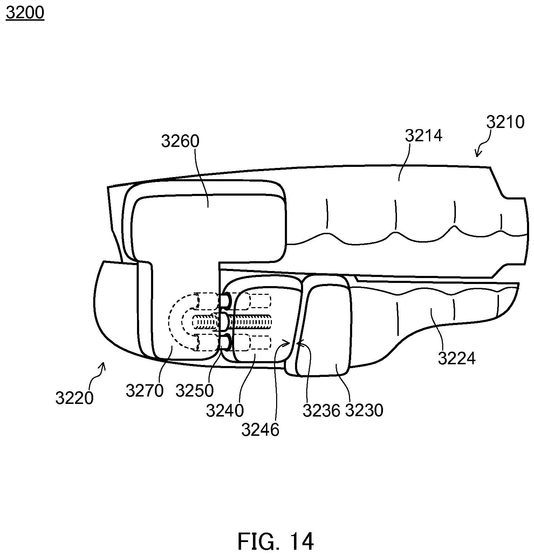

Oral appliance 3200 according to Embodiment 7 of the present invention includes a pair of upper and lower jaw oral appliances facing each other as illustrated in FIG. 14, which is a schematic side view thereof, and upper jaw oral appliance 3210 can be fitted to the upper jaw of a user and lower jaw oral appliance 3220 can be fitted to the lower jaw of the user. Also, a pair of right and left stoppers 3230 is provided on an outer side surface of lower jaw oral appliance body portion 3224 and a pair of right and left protrusion portions 3270 fixed to upper jaw oral appliance body portion 3214 by respective fixing portions 3260 is provided on an outer side surface of upper jaw oral appliance body portion 3214. Each protrusion portion 3270 supports an end of relevant guide section 3250 and guide section 3250 supports positioning member 3240 in such a manner that positioning member 3240 is movable in a front-rear direction. In the below, description of components that are in common with Embodiment 6 may be omitted.

Each stopper 3230 is provided at a position on the outer side surface of lower jaw oral appliance 3220, at which stopper 3230 is located on the front side relative to relevant protrusion portion 3270 and relevant positioning member 3240 upon biting.

Stoppers 3230 has a height that is equal to or lower than a height of lower jaw oral appliance 3220. Note that the height of lower jaw oral appliance 3220 may vary depending on the position. In this case, the height of each stopper 3230 is preferably equal to or lower than a height of a part, in which stopper 3230 is provided, of lower jaw oral appliance body portion 3224. Consequently, even if lower jaw oral appliance 3220 is displaced in a right-left direction upon opening of the mouth, each stopper 3230 and relevant positioning member 3240 are less likely to abut against each other and damage of guide sections 3250 due to application of stress caused by the abutment to positioning members 3240 is thus less likely to occur.

Each protrusion portion 3270 protrudes from relevant fixing portion 3260 provided on the outer side surface of upper jaw oral appliance 3210 toward lower jaw oral appliance 3220.

Each guide section 3250 is supported by relevant protrusion portion 3270 at a position that is lateral to lower jaw oral appliance 210 upon biting. In other words, each guide section 3250 is disposed at a position at which entire guide section 3250 is located below a lower end of upper jaw oral appliance 220 upon biting. In still other words, each guide section 3250 is disposed at a position at which entire guide section 3250 is located below an upper end of lower jaw oral appliance 210 upon biting. In this case, also, a height of each guide section 3250 may only be below a lower end of a part, in which guide section 3250 is provided, of upper jaw oral appliance body portion 3214 and be below an upper end of a part, in which guide section 3250 is provided, of lower jaw oral appliance body portion 3214.

As illustrated in FIG. 14, a position of each positioning member 3240 can be adjusted to a position at which a front surface of positioning member 3240 and a rear surface of relevant stopper 3230 abut against each other upon biting, along relevant guide section 3250. Consequently, oral appliance 3200 prevents narrowing of the airway of the user wearing oral appliance 3200 due to rearward displacement of the lower jaw of the user, enabling suppression of pauses in breathing or infrequent breathing during sleep.

Also, the position of each positioning member 3240 is adjusted forward by relevant guide section 3250, enabling, upon biting, limiting rearward displacement of lower jaw oral appliance 3220 while moving lower jaw oral appliance 3220 forward. Consequently, oral appliance 3200 allows the lower jaw of the user wearing oral appliance 3200 to be moved forward and thus allows the airway of the user to open more largely, enabling more effective suppression of pauses in breathing or infrequent breathing during sleep.

The position of each positioning member 3240 is adjusted in the front-rear direction at a position at which positioning member 3240 is located lateral to lower jaw oral appliance 3220 upon biting. In other words, each positioning member 3240 is disposed at a position at which entire positioning member 3240 is located below an upper end of lower jaw oral appliance 3220. Consequently, for example, when lower jaw oral appliance 3220 is displaced in the right-left direction upon opening of the mouth, for example, a corner portion or a side portion in the right-left direction of each positioning member 3240 is less likely to abut against relevant stopper 3230 and thus damage of guide sections 3250 by outward stress that can be applied by the abutment can further be suppressed.

Also, positioning members 3240 preferably have a height that is equal to or lower than a height of lower jaw oral appliance 3220. Consequently, each positioning member 3240 can easily be disposed at a position at which positioning member 3240 is located lateral to lower jaw oral appliance 3220 upon biting.

Each protrusion portion 3270 may be provided at a position at which when lower jaw oral appliance 3220 is displaced in the right-left direction, protrusion portion 3270 abuts against lower jaw oral appliance body portion 3224 and thereby restricts displacement in the right-left direction of lower jaw oral appliance 3220. Consequently, stress caused by the displacement in the right-left direction is applied also to protrusion portions 3270 and thereby distributed, and stress applied to positioning members 3240 thus becomes smaller. Therefore, such configuration enables suppression of damage of guide section 3250 due to displacement in the right-left direction of lower jaw oral appliance 3220.

In this case, each positioning member 3240 may be provided at a position at which when lower jaw oral appliance 3220 is displaced in the right-left direction, positioning member 3240 does not abut against lower jaw oral appliance body portion 3224. Consequently, a majority of stress caused by displacement in the right-left direction is mainly applied to protrusion portions 3270 and stress applied to positioning members 3240 thus becomes smaller. Therefore, such configuration enables more effective suppression of damage of guide sections 3250 due to displacement in the right-left direction of lower jaw oral appliance 3220.

For example, each positioning member 3240 has a thickness in an inside-outside direction thereof, the thickness being smaller than that of protrusion portions 3270, and is provided at a position at which an outer side surface of positioning member 3240 is substantially in plane with an outer side surface of relevant protrusion portion 3270. Consequently, a space between each positioning member 3240 and lower jaw oral appliance body portion 3224 upon biting is larger than a space between each protrusion portion 3270 and lower jaw oral appliance body portion 3224 upon biting.

Stoppers 3230 and positioning members 3240 may have respective shapes that when lower jaw oral appliance 3220 is moved in a mouth opening direction during wearing, prevent stoppers 3230 from limiting movement of positioning members 3240. More specifically, each stopper 3230 may include inclined portion 3236 that abuts against relevant positioning member 3240 upon biting and facilitates movement in the mouth opening direction of lower jaw oral appliance 3220. Also, each positioning member 3240 may include inclined portion 3246 that abuts against relevant stopper 3230 upon biting and facilitates movement in the mouth opening direction of lower jaw oral appliance 3220. In other words, each stopper 3230 may include a rearward inclined surface in a rear end portion thereof, the inclined surface including an upper end positioned further on the front side and a lower end positioned further on the rear side, and each positioning member 3240 may include a rearward inclined surface in a front end portion thereof, the inclined surface including an upper end positioned further on the front side and a lower end positioned further on the rear side.

In this case, also, a shape and disposition of each of portions of protrusion portions 3270, the portions abutting against lower jaw oral appliance body portion 3224, and portions of lower jaw oral appliance 3220, the portions abutting against respective protrusion portions 3270, may be those that do not limit forward movement of lower jaw oral appliance 3220. Likewise, a shape and disposition of each of portions of positioning members 3240, the portions abutting against lower jaw oral appliance body portion 3224, and portions of lower jaw oral appliance 3220, the portions abutting against respective positioning members 3240, may be those that do not limit forward movement of lower jaw oral appliance 3220.

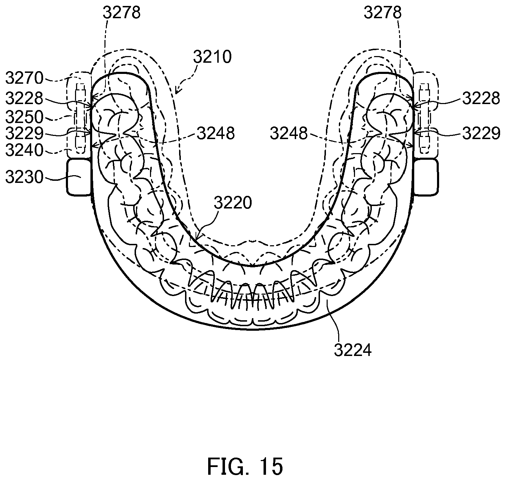

More specifically, as illustrated in FIG. 15, which is a schematic plan view of oral appliance 3200 (the upper jaw oral appliance, the protrusion portions, the guide sections, and the positioning members are each indicated by a dashed line and the lower jaw oral appliance and the stoppers are each indicated by a solid line. For ease of understanding, illustration of the fixing portions is omitted), each of abutment portions 3228 and abutment portion 3229 of lower jaw oral appliance body portion 3224, abutment portions 3228 abutting against respective protrusion portions 3270, abutment portions 3229 abutting against respective positioning members 3240, may have a substantially flat shape. Also, a pair of abutment surfaces 3278 of the pair of protrusion portions 3270, the pair of abutment surfaces 3278 abutting against lower jaw oral appliance 3220, may be substantially parallel to each other, and a pair of abutment surfaces 3248 of the pair of positioning members 3240, the pair of abutment surfaces 3248 abutting against lower jaw oral appliance 3220, are substantially parallel to each other. Also, abutment portions 3228 that abut against the pair of protrusion portions 3270 and abutment portions 3229 that abut against respective positioning members 3240, which are positioned in right and left parts of the outer side surface of lower jaw oral appliance body portion 3224, may be substantially parallel to each other.

Consequently, lower jaw oral appliance 3220 can be moved forward without movement of lower jaw oral appliance 3220 being restricted by protrusion portions 3270 and positioning members 3240. In this case, for example, a thickness in the right-left direction of each part, in the vicinity of the back teeth, of lower jaw oral appliance body portion 3224 is preferably larger on the front side (for example, a part, in the vicinity of the first molar tooth (tooth number: 6), of the outer side surface) and preferably is smaller on the rear side (for example, a part, in the vicinity of the second molar tooth (tooth number: 7) or the third molar tooth (tooth number: 8), of the outer side surface).

In Embodiment 7, also, stoppers 3230 may be detachably attachable to lower jaw oral appliance body portion 3224 but may be bonded to lower jaw oral appliance body portion 3224.

In Embodiment 7, also, fixing portions 3260 may be detachably attachable to upper jaw oral appliance body portion 3214 but may be bonded to upper jaw oral appliance body portion 3214.

In Embodiment 7, also, protrusion portions 3270 may be detachably attachable to fixing portions 3260 but may be boned to fixing portions 3260 or may be integrated with fixing portions 3260.

According to the present embodiment, protrusion portions 3270 are provided at respective positions corresponding to the rear parts, in which the skin more easily stretches and shrinks, of the inner cheeks of the user wearing oral appliance 3200, and thus, a feeling of fitting the user wearing oral appliance 3200 has is improved.

### [Variations of Embodiments 6 and 7]

Each of Embodiments 6 and 7 described above indicates an example of the third aspect of the present invention, and it is a matter of course that: the third aspect of the present invention is not limited to the above embodiments; and various other embodiments are possible within the scope of the idea of the third aspect of the present invention.

For example, in each of Embodiments 6 and 7, the stoppers, the protrusion portions, the guide sections, and the positioning members are provided on the outer side surface of the lower or upper jaw oral appliance, but stoppers, protrusion portions, guide sections, and positioning members may be provided on an inner side surface of a lower or upper jaw oral appliance. In this case, as a matter of course, fixing portion(s) are also provided on the inner side surface of the lower or upper jaw oral appliance.

As described above, stoppers, protrusion portions, guide sections, positioning members, and fixing portion(s) may be provided on either an outer side surface or an inner side surface of a lower or upper jaw oral appliance, but as a matter of course, the stoppers, the protrusion portions, the guide sections, the positioning members, and the fixing portion(s) are preferably all provided on a same side surface that is the outer side surface or the inner side surface of the lower or upper jaw oral appliance.

Also, in each of Embodiments 6 and 7, the upper jaw oral appliance body portion and the lower jaw oral appliance body portion each include a teeth row shape portion that can be fitted to a row of teeth. However, the upper jaw oral appliance body portion and the lower jaw oral appliance body portion may have a shape that is not a teeth row shape as long as the shape is a shape that receives a row of teeth, and each of the upper jaw oral appliance body portion and the lower jaw oral appliance body portion may have a shape that covers a row of teeth or may only partly include a teeth row shape portion.

Also, the upper and lower jaw oral appliances may be each formed of a plurality of materials or may be formed by a combination of a part formed of an organic substance and a part formed of an inorganic substance.

Also, in each of Embodiments 6 and 7, each of the guide sections adjusts a position of the relevant positioning member by means of adjustment of amounts of thread connection between a turnbuckle portion and two screw rods using a turnbuckle mechanism, but for each of the guide sections, a jackscrew mechanism that can adjust a position of a positioning member by adjusting an amount of thread connection between a male screw and a female screw may be used. Also, each guide section may move the relevant positioning member via a gear using a rack jack mechanism that can adjust a position of the positioning member fixed to a rack by adjusting an amount of rotation of the rack, and fix the positioning member via a screw. Each guide section may adjust a position of a positioning member using another mechanism as long as such other mechanism can move the position of the positioning member and fix the positioning member at the adjusted position.

Also, in each of Embodiments 6 and 7, a gap is provided between each protrusion portion and the upper or lower jaw oral appliance and a gap is provided between each positioning member and the upper or lower jaw oral appliance to allow movement in the right-left direction of the upper jaw and the lower jaw of a user wearing the oral appliance. However, a configuration in which no gaps are provided between the aforementioned members and no movement in the right-left direction of the upper jaw and the lower jaw of a user wearing the oral appliance is allowed may be employed.

Also, in each of Embodiments 6 and 7, where a majority of stress caused by displacement in the right-left direction is applied to the protrusion portions, the thickness in the inside-outside direction of the positioning members is made to be smaller than that of the protrusion portions. However, each positioning member may be disposed on the outer side relative to the relevant protrusion portion to prevent, when the lower jaw oral appliance is displaced in the right-left direction, the positioning member from abutting against the upper or lower jaw oral appliance. In this case, the outer side surface of each positioning member may fail to be substantially in plane with an outer side surface of the relevant protrusion portion.

### [Example Configuration of Oral Appliance Relating to Third Aspect]

The third aspect of the present invention provides an oral appliance including:
a pair of upper and lower jaw oral appliances facing each other;
a pair of stoppers provided in one oral appliance of the upper and lower jaw oral appliances, the pair of stoppers having a height that is equal to or lower than a height of the one oral appliance;
protrusion portions fixed to another oral appliance of the upper and lower jaw oral appliances, the protrusion portions protruding from the other oral appliance toward the one oral appliance; and
a pair of positioning members, a position of each of the pair of positioning members being adjusted in a front-rear direction along a guide section including an end supported by the relevant protrusion portion, at a position at which the positioning member is located lateral to the one oral appliance upon biting, each of the positioning members abutting against the relevant stopper, and thereby restricting rearward displacement of the lower jaw oral appliance.

In the oral appliance, position adjustment sections for adjusting respective positions of the lower jaw oral appliance and the upper jaw oral appliance upon biting are less likely to be broken even by displacement in a right-left direction upon biting, in particular, after opening of the mouth.

The third aspect of the present invention further provides
the oral appliance in which each of the guide sections is provided at a position at which the guide section is located lateral to the one oral appliance upon biting.

In the oral appliance, each of the positioning members can easily be disposed at a position at which the relevant stopper and the positioning member are less likely to abut against each other when the lower jaw oral appliance is displaced in the right-left direction, for example, upon biting.

The third aspect of the present invention further provides
the oral appliance in which each of the protrusion portions is provided at a position at which the protrusion portion abuts against the one oral appliance and thereby restricts displacement in the right-left direction of the upper jaw oral appliance and the lower jaw oral appliance.

In the oral appliance, stress caused by displacement in the right-left direction of the lower jaw oral appliance is applied also to the protrusion portions and stress applied to the positioning members thus become smaller or no such stress is applied to the positioning members. Therefore, the oral appliance is less likely to cause damage of the guide sections due to displacement in the right-left direction.

The third aspect of the present invention provides
the oral appliance in which each of the positioning members is disposed at a position at which the positioning member does not abut against the one oral appliance when the relevant protrusion portion abuts against the one oral appliance.

In the oral appliance, a majority of stress caused by displacement in the right-left direction of the lower jaw oral appliance is applied to the protrusion portions and stress applied to the positioning members thus become smaller or no such stress is applied to the positioning members. Therefore, the oral appliance is less likely to cause damage of the guide sections due to displacement in the right-left direction.

The third aspect of the present invention further provides
the oral appliance in which each of the positioning members includes an inclined portion that abuts against the relevant stopper upon biting and facilitates movement in a mouth opening direction of the lower jaw oral appliance.

The oral appliance makes it easy for a user wearing the oral appliance to open the mouth and further reduces a feeling of discomfort the user has.

The third aspect of present invention further provides
the oral appliance in which each of the stoppers includes an inclined portion that abuts against the relevant positioning member upon biting and facilitates movement in the mouth opening direction of the lower jaw oral appliance.

The oral appliance makes it easy for a user wearing the oral appliance to open the mouth and further reduces a feeling of discomfort the user has.

The third aspect of the present invention further provides
the oral appliance in which the stoppers are provided in the lower jaw oral appliance.

In the oral appliance, the protrusion portions are provided at respective positions corresponding to the rear parts, in which the skin more easily stretches and shrinks, of the inner cheeks of a user wearing the oral appliance and a feeling of fitting the user wearing the oral appliance has is improved.

### 4. Oral Appliance Relating to Fourth Aspect

In an oral appliance relating to the fourth aspect of the present invention, a decrease in amount of movement of each position adjustment section, which is a point of load of a second-class lever, is enabled by enabling adjustment of an amount of forward movement of a lower jaw oral appliance in each of a pair of upper and lower jaw oral appliances. With such configuration, the oral appliance relating to the fourth aspect of the present invention reduces stress applied to the position adjustment sections due to the principle of second-class lever and thereby suppresses damage of the position adjustment sections due to displacement in the right-left direction.

### [Embodiment 8]

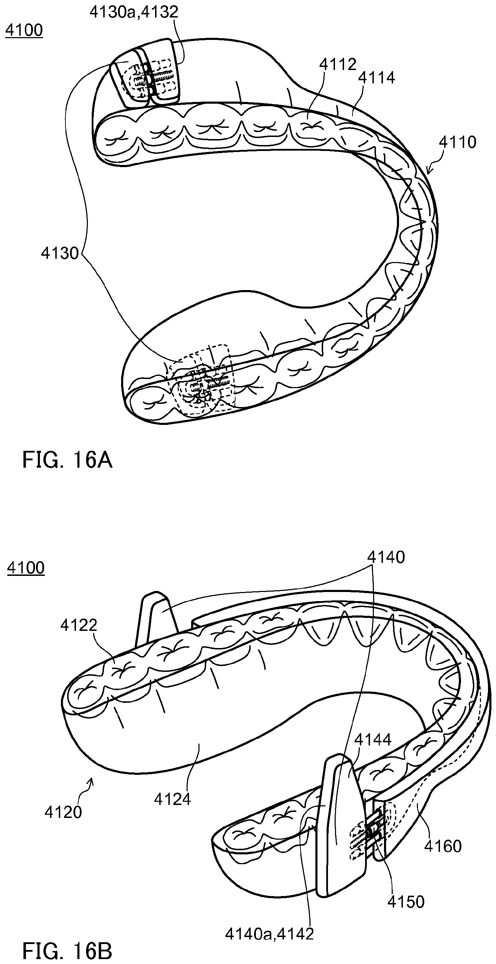

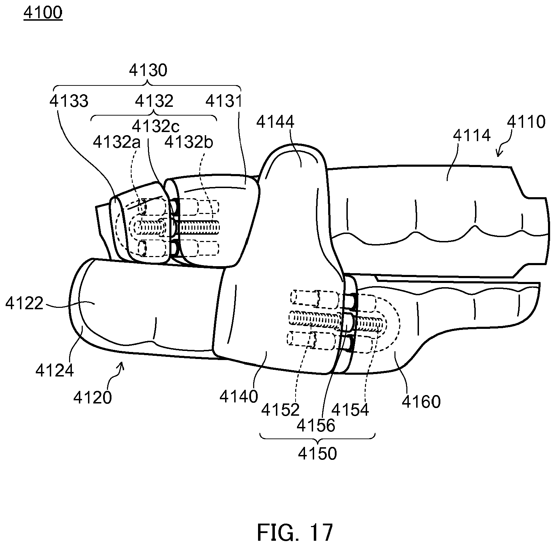

Oral appliance 4100 according to Embodiment 8 of the present invention includes a pair of upper and lower jaw oral appliances facing each other as illustrated in FIGS. 16A and 16B, which are schematic perspective views thereof, and FIG. 17, which is a side view thereof, and upper jaw oral appliance 4110 can be fitted to the upper jaw of a user and lower jaw oral appliance 4120 can be fitted to the lower jaw of the user.

As illustrated in FIG. 16A, upper jaw oral appliance 4110 includes upper jaw oral appliance body portion 4114 with teeth row shape portion 4112 formed therein, teeth row shape portion 4112 being able to be fitted to a row of teeth of the upper jaw of the user, and on an outer side surface thereof, a pair of right and left stoppers 4130 is provided.

As illustrated in FIG. 16B, lower jaw oral appliance 4120 includes lower jaw oral appliance body portion 4124 with teeth row shape portion 4122 formed therein, teeth row shape portion 4122 being able to be fitted to a row of teeth of the lower jaw of the user, and an outer side surface thereof, a pair of right and left positioning members 4140 that enable position adjustment in a front-rear direction is provided.

As illustrated in FIG. 17, a position of each positioning member 4140 can be adjusted to a position at which rear surface 4140a of positioning member 4140 and front surface 4130a of relevant stopper 4130 abut against each other when the user wearing upper jaw oral appliance 4110 and lower jaw oral appliance 4120 bites. The abutment between positioning members 4140 and stoppers 4130 limits rearward displacement of lower jaw oral appliance 4120 upon biting and thus limits rearward displacement of the lower jaw of the user wearing oral appliance 4100. Consequently, oral appliance 4100 can prevent narrowing of the airway of the user wearing oral appliance 4100 due to rearward displacement of the lower jaw of the user and thus suppress pauses in breathing or infrequent breathing during sleep.

Also, the positions of positioning members 4140 can be adjusted in the front-rear direction by respective position adjustment sections 4150 provided on the outer side surface of lower jaw oral appliance body portion 4124. The positions of positioning members 4140 are adjusted rearward by position adjustment sections 4150, enabling positioning members 4140 to, upon biting, limit rearward displacement of lower jaw oral appliance 4120 while moving lower jaw oral appliance 4120 forward. Consequently, oral appliance 4100 allows the lower jaw of the user wearing oral appliance 4100 to be moved forward and thus allows the airway of the user to open more largely, enabling effective suppression of pauses in breathing or infrequent breathing during sleep.

Each position adjustment section 4150 includes male-threaded screw rod 4152 including an end fixed to fixing portion 4160 provided on the outer side surface of lower jaw oral appliance body portion 4124, male-threaded screw rod 4152 including an end fixed to relevant positioning member 4140, and turnbuckle portion 4156 including a screw hole to which respective other ends of screw rods 4152 and 4154 are threadably connected. Position adjustment section 4150 can adjust the position of positioning member 4140 fixed to screw rod 4152, by inserting a rotation pin into a pin hole provided in turnbuckle portion 4156 and rotating turnbuckle portion 4156 to adjust amounts of the thread connection between turnbuckle portion 4156 and screw rods 4152 and 4154.

Each positioning member 4140 includes wing 4144 that protrudes upward from lower jaw oral appliance body portion 4124. A rear surface of wing 4144 (which is also rear surface 4140a of positioning member 4140, rear surface 4140a abutting against relevant stopper 4130) is substantially parallel to front surface 4130a of stopper 4130, and upon biting, abuts against front surface 4130a of stopper 4130 on the outer side of upper jaw oral appliance body portion 4114 and wing 4144 thereby limits rearward displacement of the lower jaw of the user.

Each stopper 4130 is provided at a position on the outer side surface of the upper jaw oral appliance, at which upon biting, stopper 4130 is located on the rear side relative to the relevant positioning member, and upon biting, front surface 4130a of each stopper 4130 abuts against rear surface 4140a of relevant positioning member 4140 and stopper 4130 thereby limits rearward displacement of lower jaw oral appliance 4120.

Each stopper 4130 includes moving portion 4131 that abuts against relevant positioning member 4140, stopper adjustment section 4132 that adjusts a position of moving portion 4131 in the front-rear direction, and stopper fixing portion 4133 fixed to the outer side surface of upper jaw oral appliance body portion 4114. Respective surfaces of moving portion 4131 and stopper fixing portion 4133, the surfaces facing each other, are substantially parallel to each other.

The position of each moving portion 4131 is adjusted forward by relevant stopper adjustment section 4132 and moving portion 4131 thereby abuts against relevant positioning member 4140, enabling, upon biting, limiting rearward displacement of lower jaw oral appliance 4120 while making lower jaw oral appliance 4120 protrude forward.

Each stopper adjustment section 4132 includes male-threaded screw rod 4132a including an end fixed to relevant stopper fixing portion 4133, male-threaded screw rod 4132b including an end fixed to relevant moving portion 4131, and turnbuckle portion 4132c including a screw hole to which respective other ends of screw rods 4132a and 4132b are threadably connected. Stopper adjustment section 4132 can adjust the position of moving portion 4131 fixed to screw rod 4132b, by inserting a rotation pin into a pin hole provided in turnbuckle portion 4132c and rotating turnbuckle portion 4132c to adjust amounts of the thread connection between turnbuckle portion 4132c and screw rods 4132a and 4132b.

Stopper fixing portions 4133 are preferably provided further on the rear side of the outer side surface of upper jaw oral appliance body portion 4114 and are preferably provided at respective rear ends (for example, the outer side surface of the second molar tooth (tooth number: 7) or the third molar tooth (tooth number: 8)), of upper jaw oral appliance body portion 4114. As a result of each stopper fixing portion 4133 being provided further on the rear side, ranges of position adjustment of relevant positioning member 4140 and moving portion 4131 of relevant stopper 4130 can be widened, enabling an increase in amount of forward movement of the lower jaw.

As a result of the position of moving portion 4131 being adjusted forward, stopper 4130 can make moving portion 4131 and positioning member 4140 abut against each other with the position of positioning member 4140 adjusted forward. Consequently, a distance between each wing 4144, which is a point of effort of a second-class lever, and fixing portion 4160, which is a fulcrum of the second-class lever, is decreased, suppressing an excessive increase of stress applied to wing 4144 in position adjustment section 4150, which is a point of load of the second-class lever. Therefore, stoppers 4130 enable suppression of damage of position adjustment sections 4150 due to displacement in the right-left direction of the lower jaw oral appliance.

Also, as a result of the positions of moving portions 4131 being adjusted forward, stoppers 4130 enables, upon biting, limiting rearward displacement of lower jaw oral appliance 4120 while moving lower jaw oral appliance 4120 forward. Consequently, oral appliance 4100 allows the lower jaw of the user wearing oral appliance 4100 to be moved forward and thus allows the airway of the user to open more largely, enabling more effective suppression of pauses in breathing or infrequent breathing during sleep.

In the present embodiment, stoppers 4130 have a height that is equal to or lower than a height of upper jaw oral appliance 4110. Consequently, when lower jaw oral appliance 4120 is displaced in the right-left direction, stoppers 4130 do not substantially abut against lower jaw oral appliance 4120, and thus, damage of stopper adjustment sections 4132 due to displacement in the right-left direction of the lower jaw oral appliance is suppressed.

Note that the height of upper jaw oral appliance 4110 may vary depending on the position. In this case, it is only necessary that the height of stopper 4130 be equal to or lower than a height of a part, in which stopper 4130 is provided, of upper jaw oral appliance body portion 4114.

Stoppers 4130 and wings 4144 have respective shapes that when lower jaw oral appliance 4120 is moved in a mouth opening direction during wearing, prevent stoppers 4130 from limiting movement of wings 4144. More specifically, each stopper 4130 may include an inclined portion that facilitates movement in the mouth opening direction of lower jaw oral appliance 4120. Also, each wing 4144 may include an inclined portion in a rear end surface thereof, the inclined portion facilitating movement in the mouth opening direction of lower jaw oral appliance 4120. In other words, each stopper 4130 may include a rearward inclined surface in a front end portion thereof (front end portion of moving portion 4131), the inclined surface including an upper end positioned further on the front side and a lower end positioned further on the rear side, and each wing 4144 may include a rearward inclined surface in a rear end portion thereof, the inclined surface including an upper end positioned further on the front side and a lower end positioned further on the rear side. The inclined portions of stoppers 4130 and the inclined portions of wings 4144 abut against each other upon biting.

When lower jaw oral appliance 4120 is moved in the mouth opening direction during wearing, lower jaw oral appliance 4120 is moved on a circular arc-like path. In such movement, movement of wings 4144 is not limited by stoppers 4130 as a result of the front end portions of stoppers 4130 and the rear end portions of wings 4144 including the respective inclined surfaces described above, making it easy for the user wearing oral appliance 4100 to open the mouth.

Position adjustment sections 4150 and stopper adjustment sections 4132 may be configured so that a movable width of positioning members 4140 is smaller than a movable width of moving portions 4131 of stoppers 4130. For example, the amounts of thread connection between turnbuckle portion 4156 of each position adjustment section 4150 and relevant screw rods 4152 and 4154 may be smaller than the amounts of thread connection between turnbuckle portion 4132c of each stopper adjustment section 4132 and relevant screw rods 4132a and 4132b. Consequently, the positions of positioning members 4140 are less likely to be adjusted rearward, enabling suppression of damage of position adjustment sections 4150 due to an excessive increase of stress applied to wings 4144, each of which is a point of effort of a second-class lever, in position adjustment sections 4150, each of which is a point of load of the second-class lever.

Fixing portion 4160 may be detachably attachable to lower jaw oral appliance body portion 4124, but may be bonded to lower jaw oral appliance body portion 4124. If fixing portion 4160 is bonded to lower jaw oral appliance body portion 4124, misalignment of fixing portion 4160 and positioning members 4140 is less likely to occur during wearing.

Stopper fixing portions 4133 may be detachably attachable to upper jaw oral appliance body portion 4114 but may be bonded to upper jaw oral appliance body portion 4114. If stopper fixing portions 4133 are bonded to upper jaw oral appliance body portion 4114, misalignment of stopper fixing portions 4133 and stoppers 4130 is less likely to occur during wearing.

According to the present embodiment, a distance between each wing 4144, which is a point of effort of a second-class lever, and relevant fixing portion 4160, which is a fulcrum of the second-class lever, can be reduced in comparison with those of conventional techniques. Therefore, damage of relevant position adjustment section 4150 due to an excessive increase of stress applied to wing 4144 when the lower jaw oral appliance is displaced in the right-left direction, in relevant position adjustment section 4150, which is a point of load of the second-class lever, can be suppressed.

On the other hand, according to the present embodiment, an amount of forward movement of lower jaw oral appliance 4120 can be adjusted by adjustment of the positions of moving portions 4131 of stoppers 4130 by stopper adjustment sections 4132 in addition to adjustment of the positions of positioning members 4140 by position adjustment sections 4150. Therefore, the amount of forward movement of lower jaw oral appliance 4120 can be increased in comparison with conventional oral appliances in which an amount of forward movement of a lower jaw oral appliance is adjusted by position adjustment sections alone.

Also, according to the present embodiment, since each stopper 4130 includes no flange portion that protrudes downward from upper jaw oral appliance 4110, wings 4144 and stoppers 4130 are less likely to abut against each other upon movement in the mouth opening direction of lower jaw oral appliance 4120. Therefore, oral appliance 4100 of the present embodiment more facilitates movement in the mouth opening direction of lower jaw oral appliance 4120.

Also, according to the present embodiment, since each stopper 4130 includes no flange portion that protrudes downward from upper jaw oral appliance 4110, lower jaw oral appliance 4120 and stopper 4130 do not abut against each other even when lower jaw oral appliance 4120 is displaced in the right-left direction. Therefore, oral appliance 4100 of the present embodiment enables suppression of damage of stopper adjustment sections 4132 due to displacement in the right-left direction of lower jaw oral appliance.

### [Embodiment 9]

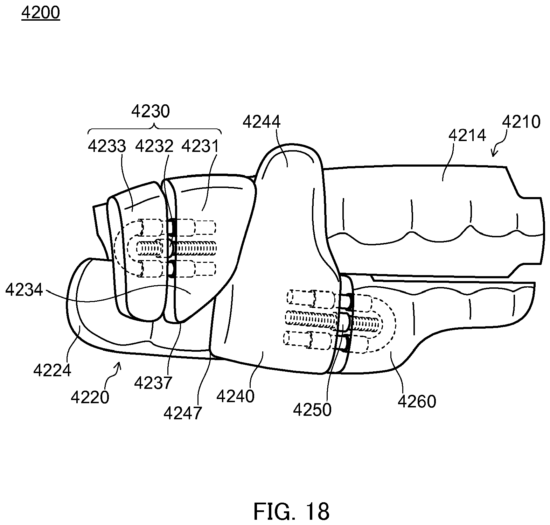

Oral appliance 4200 according to Embodiment 9 of the present invention includes a pair of upper and lower jaw oral appliances facing each other as illustrated in FIG. 18, which is a schematic side view thereof, and upper jaw oral appliance 4210 can be fitted to the upper jaw of a user and lower jaw oral appliance 4220 can be fitted to the lower jaw of the user. Also, a pair of right and left positioning members 4240 which are adjustable in position in a front-rear direction is provided on an outer side surface of lower jaw oral appliance body portion 4224 and a pair of right and left stoppers 4230 which are adjustable in position in the front-rear direction, is provided on an outer side surface of upper jaw oral appliance body portion 4214. In the below, description of components that are in common with Embodiment 8 may be omitted.

In the present embodiment, also, the positions of positioning members 4240 can be adjusted in the front-rear direction by respective position adjustment sections 4250 provided on the outer side surface of lower jaw oral appliance body portion 4224. In the present embodiment, also, each stopper 4230 includes moving portion 4231 that abuts against relevant positioning member 4240, stopper adjustment section 4232, and stopper fixing portion 4233. Respective surfaces of moving portion 4231 and stopper fixing portion 4233, the surfaces facing each other, are substantially parallel to each other, and a position of moving portion 4231 is adjusted forward by stopper adjustment section 4232 and moving portion 4231 thereby abuts against relevant positioning member 4240. The positions of positioning members 4240 are adjusted rearward by position adjustment sections 4250 or the positions of moving portions 4231 of stoppers 4230 are adjusted forward by stopper adjustment sections 4232, enabling, upon biting, limiting rearward displacement of lower jaw oral appliance 4220 while moving lower jaw oral appliance 4220 forward.

In the present embodiment, also, position adjustment sections 4250 and stopper adjustment sections 4232 are configured so that a movable width of positioning members 4240 is smaller than a movable width of moving portions 4231 of stoppers 4230.

In the present embodiment, each stopper 4230 includes extension portion 4234 that extends downward from upper jaw oral appliance body portion 4214. When lower jaw oral appliance 4220 is displaced in a right-left direction upon biting, each extension portion 4234 abuts against lower jaw oral appliance 4220 and thereby restricts the displacement in the right-left direction.

With the above configuration, when lower jaw oral appliance 4220 is displaced in the right-left direction upon biting, wings 4244 of positioning members 4240 abut against upper jaw oral appliance 4210 and extension portions 4234 of stoppers 4230 abut against lower jaw oral appliance 4220. Therefore, stress caused by the displacement in the right-left direction is distributed to positioning members 4240 and stoppers 4230, enabling more effective suppression of damage of position adjustment sections 4250 by the stress. In addition, as a result of the distribution of the stress, damage of stopper adjustment sections 4232 is less likely to occur.

Stoppers 4230 and wings 4244 have respective shapes that when lower jaw oral appliance 4220 is moved in a mouth opening direction during wearing, prevent stoppers 4230 from limiting movement of wings 4244. More specifically, each stopper 4230 may include inclined portion 4236 in moving portion 4231, inclined portion 4236 facilitating movement in the mouth opening direction of lower jaw oral appliance 4220. Also, each wing 4244 may include inclined portion 4246 that facilitates movement in the mouth opening direction of lower jaw oral appliance 4220. In other words, each stopper 4230 may include a rearward inclined surface in a front end portion thereof, the inclined surface including an upper end positioned further on the front side and a lower end positioned further on the rear side, and each wing 4244 may include a rearward inclined surface in a rear end portion thereof, the rearward inclined surface including an upper end positioned further on the front side and a lower end positioned further on the rear side. Inclined portions 4246 of wings 4244 abut against respective stoppers 4230 upon biting.

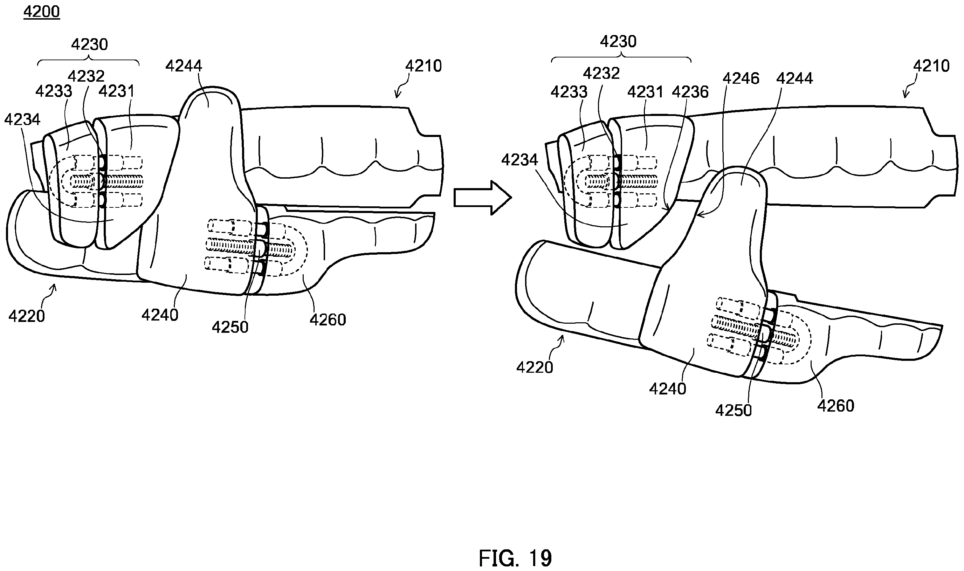

As illustrated in FIG. 19, when lower jaw oral appliance 4220 is moved in the mouth opening direction during wearing, lower jaw oral appliance 4220 is moved on a circular arc-like path. In such movement, movement of wings 4244 is not limited by stoppers 4230 as a result of the front end portions of stoppers 4230 and the rear end portions of wings 4244 including the respective inclined surfaces described above, making it easy for the user wearing oral appliance 4200 to open the mouth. The effect of making opening of the mouth easy as a result of the stoppers 4230 and wings 4244 including the respective inclined surfaces are more significant in the present embodiment in which extension portions 4234 can be disposed on a path of wings 4244 upon opening of the mouth, than that of Embodiment 8.

In each stopper 4230 including the inclined surface in the front end portion, the upper end of the front end portion thereof (front end portion of moving portion 4231) may only be positioned further on the front side and the rear end of the front end portion thereof may only be positioned further on the rear side. In this case, the front end portion of each moving portion 4231 can have a shape of, for example, a cutout portion, which is illustrated in FIG. 20A, a flat inclined portion, which is illustrated in FIG. 20B, or a curved portion, which is illustrated in FIG. 20C.

Likewise, in each wing 4244 including the inclined surface, the upper end of the rear end portion thereof may only be positioned further on the front side and the rear end of the rear end portion thereof may only be positioned on further on the rear side. In this case, as with the front end portion of each stopper 4230, the rear end portion of each wing 4244 can have a shape of, for example, a cutout portion, a flat inclined portion, or a curved portion.

Here, the front end portion of each stopper 4230 (moving portion 4231) is inclined rearward in extension portion 4234. Also, extension portions 4234 have a height that is equal to or less than half a height of stoppers 4230, and upon biting, a lower end of each extension portion 4234 is positioned above a lower end surface of lower jaw oral appliance body portion 4224. Also, upon biting, lower end 4237 of the front end portion of each stopper 4230 and lower end 4247 of the rear end portion of relevant positioning member 4240 are preferably spaced from each other. Also, upon biting, a front surface of each stopper 4230 (front surface of each extension portion 4234) and a rear surface of relevant positioning member 4240 are preferably spaced from each other below an occlusal surface, and also more preferably consistently spaced from each other when the lower jaw oral appliance and/or the upper jaw oral appliance are moved in the mouth opening direction. Consequently, extension portions 4234 are less likely to limit movement in the mouth opening direction of lower jaw oral appliance 4220 during wearing, more facilitating movement in the mouth opening direction of lower jaw oral appliance 4220. Here, the height of each extension portion 4234 means a height of a part of a rear end portion of relevant moving portion 4231, the part extending from a lower end of upper jaw oral appliance body portion 4214, and the height of each stopper 4230 means a height of the rear end portion of moving portion 4231.

In this case, from the perspective of suppression of damage of stoppers 4230 due to displacement in the right-left direction of the lower jaw oral appliance, where the front end portion of each stopper 4230 includes a cutout portion such as illustrated in FIG. 20A, lower end 4237 of the cutout portion is preferably obtuse. Also, likewise, from the perspective of suppression of damage of stoppers 4230, the projection area of projection of each stopper 4230 to lower jaw oral appliance body portion 4224 from a lateral side direction upon biting is preferably 3 mm² or more. An upper limit of the projection area is not specifically provided. The projection area is, for example, preferably 3 mm² to 1000 mm², more preferably 3 mm² to 500 mm², further preferably 3 mm² to 450 mm², particularly preferably 50 mm² to 450 mm².

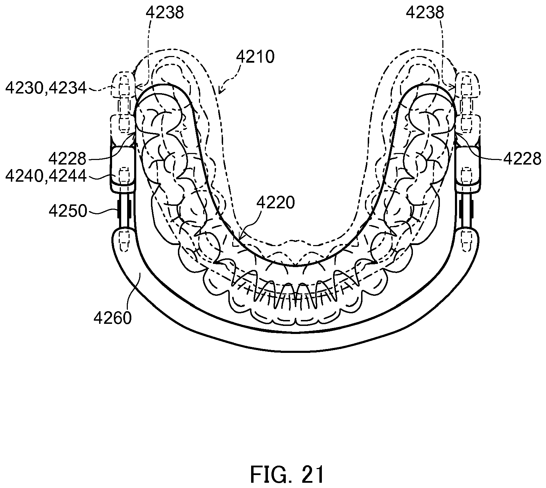

In this case, also, a shape and disposition of each of portions of stoppers 4230, the portions abutting against lower jaw oral appliance body portion 4224, and portions of lower jaw oral appliance 4220, the portions abutting against respective stoppers 4230, are those that do not limit forward movement of lower jaw oral appliance 4220. More specifically, as illustrated in FIG. 21, which is a schematic plan view of oral appliance 4200 (the upper jaw oral appliance and the stoppers are each indicated by a dashed line and the lower jaw oral appliance, the positioning members, and a fixing portion are each indicated by a solid line), each of abutment portions 4228 of lower jaw oral appliance body portion 4224, abutment portions 4228 abutting against respective stoppers 4230, have a substantially flat shape. Also, a pair of abutment surfaces 4238 of extension portions 4234 of the pair of stoppers 4230, abutment surfaces 4238 abutting against lower jaw oral appliance 4220, are substantially parallel to each other, and abutment portions 4228 positioned in right and left parts of the outer side surface of lower jaw oral appliance body portion 4224, abutment portions 4228 abutting against respective extension portions 4234 of the pair of stoppers 4230, are substantially parallel to each other.

Consequently, lower jaw oral appliance 4220 can be moved forward without movement of lower jaw oral appliance 4220 being restricted by extension portions 4234. In this case, for example, a thickness in the right-left direction of each part, in the vicinity of the back teeth, of lower jaw oral appliance body portion 4224 is larger on the front side (for example, a part, in the vicinity of the first molar tooth (tooth number: 6), of the outer side surface) and is smaller on the rear side (for example, a part, in the vicinity of the second molar tooth (tooth number: 7) or the third molar tooth (tooth number: 8), of the outer side surface).

In the present embodiment, also, fixing portion 4260 may be detachably attachable to lower jaw oral appliance body portion 4224 but may be bonded to lower jaw oral appliance body portion 4224.

In the present embodiment, also, stopper fixing portions 4233 may be detachably attachable to upper jaw oral appliance body portion 4214 but may be bonded to upper jaw oral appliance body portion 4214.

According to the present embodiment, stress that is conventionally applied only to wings 4244 of positioning members 4240 upon displacement in the right-left direction is applied also to extension portions 4234 of stoppers 4230 and thus distributed to both of wings 4244 and extension portions 4234. Therefore, oral appliance 4200 according to the present embodiment enables suppression of damage of position adjustment sections 4250 by stress caused when wings 4244 of positioning members 4240 abut against upper jaw oral appliance body portion 4214 as a result of displacement in the right-left direction.

Furthermore, according to the present embodiment, each stopper 4230 includes extension portion 4234 that extends downward and each positioning member 4240 includes wing 4244 that protrudes upward, and thus, for example, even when the user opens the mouth during wearing, the front surface of stopper 4230 and the rear surface of wing 4244 can abut against each other. Therefore, oral appliance 4200 according to the present embodiment is less likely to cause a misalignment between stoppers 4230 and positioning members 4240 due to, for example, opening of the mouth during wearing and enables more effective suppression of rearward displacement of the lower jaw of a user wearing oral appliance 4200.

### [Embodiment 10]

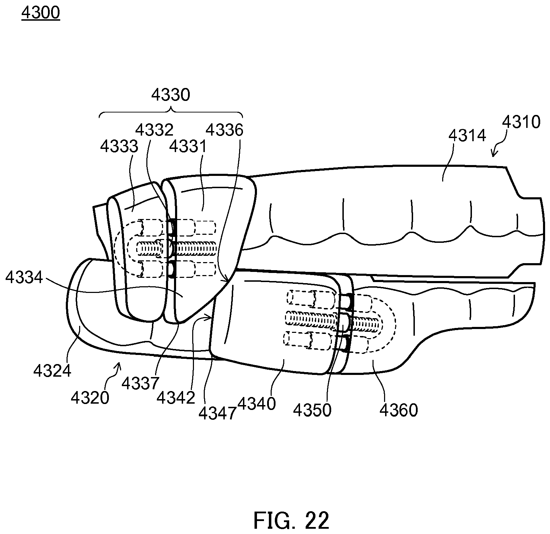

Oral appliance 4300 according to Embodiment 10 of the present invention includes a pair of upper and lower jaw oral appliances as illustrated in FIG. 22, which is a schematic side view thereof, and upper jaw oral appliance 4310 can be fitted to the upper jaw of the user and lower jaw oral appliance 4320 can be fitted to the lower jaw of the user. Also, a pair of right and left positioning members 4340 which are adjustable in position in a front-rear direction is provided on an outer side surface of lower jaw oral appliance body portion 4324 and a pair of right and left stoppers 4330 which are adjustable in position in the front-rear direction is provided on an outer side surface of upper jaw oral appliance body portion 4314. In the below, description of components that are in common with Embodiment 8 or Embodiment 9 may be omitted.

In the present embodiment, also, the positions of positioning members 4340 can be adjusted in the front-rear direction by respective position adjustment sections 4350 provided on the outer side surface of lower jaw oral appliance body portion 4324. In the present embodiment, also, each stopper 4330 includes moving portion 4331 that abuts against relevant positioning member 4340, stopper adjustment section 4332, and stopper fixing portion 4333, respective surfaces of moving portion 4331 and stopper fixing portion 4333, the surfaces facing each other, are substantially parallel to each other, and a position of moving portion 4331 is adjusted forward by stopper adjustment section 4332 and moving portion 4331 thereby abuts against positioning member 4340. The positions of positioning members 4340 are adjusted rearward by position adjustment sections 4350 or the positions of moving portions 4331 of stoppers 4330 are adjusted forward by stopper adjustment sections 4332, enabling, upon biting, limiting rearward displacement of lower jaw oral appliance 4320 while moving lower jaw oral appliance 4320 forward.

In the present embodiment, also, position adjustment sections 4350 and stopper adjustment sections 4332 are configured so that a movable width of positioning members 4340 is smaller than a movable width of moving portions 4331 of stoppers 4330.

In the present embodiment, also, each stopper 4330 includes extension portion 4334 that extends downward from upper jaw oral appliance body portion 4314.

In the present embodiment, positioning members 4340 have a height that is equal to or lower than a height of lower jaw oral appliance body portion 4324.

Note that the height of lower jaw oral appliance may vary depending on the position. In this case, in the present embodiment, the height of each positioning member 4340 may only be equal to or lower than a height of a part, in which positioning member 4340 is provided, of lower jaw oral appliance body portion 4324.

With the above configuration, when lower jaw oral appliance 4320 is displaced in the right-left direction upon biting, positioning members 4340 are less likely to abut against upper jaw oral appliance 4310 and stress is less likely to be applied to positioning members 4340. Therefore, oral appliance 4300 according to the present embodiment enables suppression of damage of position adjustment sections 4350 due to displacement in the right-left direction.

In this case, also, stoppers 4330 each have a shape that when lower jaw oral appliance 4320 is moved in an mouth opening direction during wearing, prevents stopper 4330 from limiting movement of relevant positioning member 4340. More specifically, each stopper 4330 may include inclined portion 4336 that facilitates movement in the mouth opening direction of lower jaw oral appliance 4320. In other words, a front end portion of each stopper 4330 may include a rearward inclined surface including an upper end positioned further on the front side and a lower end positioned further on the rear side.

In each stopper 4330 including the inclined surface in the front end portion, the upper end of the front end portion thereof may only be positioned further on the front side and the rear end of the front end portion thereof may only be positioned further on the rear side. In this case, as in Embodiment 9, the front end portion of each stopper 4330 can have a shape of, for example, a cutout portion, a flat inclined portion, or a curved portion.

In this case, also, the front end portion of each stopper 4330 is inclined further rearward in extension portion 4334. Also, extension portions 4334 have a height that is equal to or less than half a height of stoppers 4330, and upon biting, a lower end of each extension portion 4334 is positioned above a lower end surface of lower jaw oral appliance body portion 4324. Also, upon biting, lower end 4337 of the front end portion of each stopper 4330 and lower end 4347 of the rear end portion of relevant positioning member 4340 are preferably spaced from each other. Also, upon biting, a front surface of each stopper 4330 (front surface of each extension portion 4334) and a rear surface of relevant positioning member 4340 are preferably spaced from each other below an occlusal surface, and also more preferably consistently spaced from each other when lower jaw oral appliance and/or the upper jaw oral appliance are moved in the mouth opening direction.

In this case, from the perspective of suppression of damage of stoppers 4330 due to displacement in the right-left direction of lower jaw oral appliance, where the front end portion of each stopper 4330 includes a cutout portion such as illustrated in FIG. 20A, lower end 4337 of the cutout portion is preferably obtuse. Also, likewise, from the perspective of suppression of damage of stoppers 4330, the projection area of projection of each stopper 4330 to lower jaw oral appliance body portion 4324 from a lateral side direction upon biting is preferably 3 mm² or more. An upper limit of the projection area is not specifically provided. The projection area is, for example, preferably 3 mm² to 1000 mm², more preferably 3 mm² to 500 mm², further preferably 3 mm² to 450 mm², particularly preferably 50 mm² to 450 mm².

In this case, also, a shape and disposition of each of portions of stoppers 4330, the portions abutting against lower jaw oral appliance body portion 4324, and portions of lower jaw oral appliance 4320, the portions abutting against respective stoppers 4330, are those that do not limit forward movement of lower jaw oral appliance 4320. More specifically, portions of lower jaw oral appliance body portion 4324, the portions abutting against respective stoppers 4330, have a substantially flat shape. Also, a pair of abutment surfaces of extension portions 4334 of the pair of stoppers 4330, abutment surfaces abutting against lower jaw oral appliance 4320, are substantially parallel to each other, and abutment portions positioned in right and left parts of the outer side surface of lower jaw oral appliance body portion 4324, the abutment portions abutting against respective extension portions 4334 of the pair of stoppers 4330, are substantially parallel to each other.

In the present embodiment, also, fixing portion 4360 may be detachably attachable to lower jaw oral appliance body portion 4324 but may be bonded to lower jaw oral appliance body portion 4324.

In the present embodiment, also, stopper fixing portions 4333 may be detachably attachable to upper jaw oral appliance body portion 4314 but may be bonded to upper jaw oral appliance body portion 4314.

The height of each positioning member 4340 may partially larger than that of lower jaw oral appliance body portion 4324, but is preferably consistently equal to or lower than the height of lower jaw oral appliance body portion 4324.

Also, an inclined surface may be provided in the rear surface of each positioning member 4340 to make respective surfaces of positioning member 4340 and relevant extension portion 4334, the surfaces abutting against each other, substantially parallel to each other.

According to the present embodiment, when lower jaw oral appliance 4320 is displaced in the right-left direction upon biting, stress is less likely to be applied to positioning members 4340. Therefore, oral appliance 4300 according to the present embodiment enables suppression of damage of position adjustment sections 4350 due to displacement in the right-left direction.

Also, according to the present embodiment, as a result of the positions of positioning members 4340 being adjusted rearward, stoppers 4330 and positioning members 4340 can be made to abut against each other with moving portions 4331 of stoppers 4330 positioned further on the rear side. Consequently, a distance between each extension portion 4334, which is a point of effort of a second-class lever, and relevant stopper fixing portion 4433, which is a fulcrum of the second-class lever, is decreased, and thus, an excessive increase of stress applied to each extension portion 4334 in relevant stopper adjustment section 4332, which is a point of load of the second-class lever, is suppressed. Therefore, positioning members 4340 enable suppression of damage of stopper adjustment sections 4332 due to displacement in the right-left direction of the lower jaw oral appliance.

Also, according to the present embodiment, since each positioning member 4340 includes no wing that protrudes upward from lower jaw oral appliance 4320, the user is less likely to have a feeling of discomfort due to the wings abutting against the inner cheeks during wearing.

Also, according to the present embodiment, each positioning member 4340 includes no wing that protrudes upward from lower jaw oral appliance 4320, and thus, upon movement in the mouth opening direction of lower jaw oral appliance 4320, positioning members 4340 and stoppers 4330 are less likely to abut against each other. Therefore, oral appliance 4300 according to the present embodiment further facilitates movement in the mouth opening direction of lower jaw oral appliance 4320.

### [Embodiment 11]

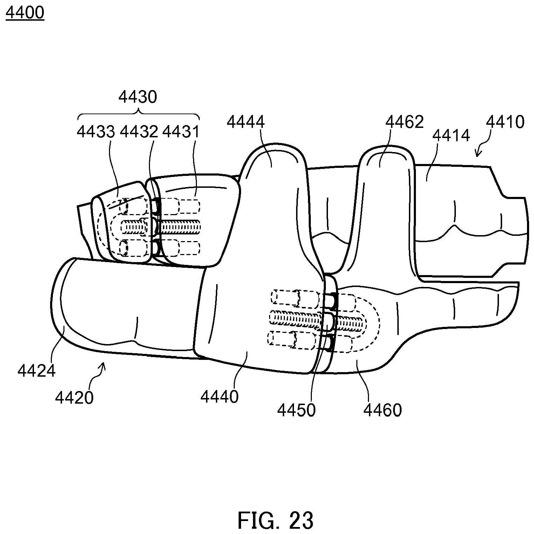

Oral appliance 4400 according to Embodiment 11 of the present invention includes a pair of upper and lower jaw oral appliances facing each other as illustrated in FIG. 23, which is a schematic side view thereof, and upper jaw oral appliance 4410 can be fitted to the upper jaw of a user and lower jaw oral appliance 4420 can be fitted to the lower jaw of the user. Also, a pair of right and left positioning members 4440 which are adjustable in position in a front-rear direction is provided on an outer side surface of lower jaw oral appliance body portion 4424 and a pair of right and left stoppers 4430 which are adjustable in position in the front-rear direction is provided on an outer side surface of upper jaw oral appliance body portion 4414. In the below, description, components that are each in common with any of Embodiments 8 to 10 may be omitted.

In the present embodiment, also, the positions of positioning members 4440 can be adjusted in the front-rear direction by respective position adjustment sections 4450 provided on the outer side surface of lower jaw oral appliance body portion 4424. In the present embodiment, also, each stopper 4430 includes moving portion 4431 that abuts against relevant positioning member 4440, stopper adjustment section 4432, and stopper fixing portion 4433, and respective surfaces of moving portion 4431 and stopper fixing portion 4433, the surfaces facing each other, are substantially parallel to each other, and a position of moving portion 4431 is adjusted forward by stopper adjustment section 4432 and moving portion 4431 thereby abuts against positioning member 4440. The positions of positioning member 4440 are adjusted rearward by position adjustment sections 4450 or the positions of moving portions 4431 of stoppers 4430 are adjusted forward by stopper adjustment sections 4432, enabling, upon biting, limiting rearward displacement of lower jaw oral appliance 4420 while moving lower jaw oral appliance 4420 forward.

In the present embodiment, also, position adjustment sections 4450 and stopper adjustment sections 4432 are configured so that a movable width of positioning members 4440 is smaller than a movable width of moving portions 4431 of stoppers 4430.

In the present embodiment, also, each positioning member 4440 includes wing 4444 that protrudes upward from lower jaw oral appliance body portion 4424. A rear surface of wing 4444, the rear surface abutting against stopper 4430, is substantially parallel to a front surface of stopper 4430, and upon biting, abuts against the front surface of stopper 4430 on the outer side of upper jaw oral appliance body portion 4414 and wing 4444 thereby limits rearward displacement of the lower jaw of the user.

In the present embodiment, fixing portion 4460 includes protrusion portions 4462 that extend upward from lower jaw oral appliance 4420. When lower jaw oral appliance 4420 is displaced in a right-left direction upon biting, each protrusion portion 4462 abuts against upper jaw oral appliance 4410 and thereby restricts the displacement in the right-left direction.

In this case, each wing 4444 is disposed at a position at which when lower jaw oral appliance is displaced in the right-left direction upon biting and protrusion portions 4462 thereby abut against upper jaw oral appliance 4410, wing 4444 is less likely to abut against upper jaw oral appliance 4410. Consequently, a majority of stress caused by the displacement in the right-left direction is applied to protrusion portions 4462 of fixing portion 4460 and the stress is less likely to be applied to positioning members 4440. Therefore, damage of position adjustment sections 4450 due to the displacement in the right-left direction of lower jaw oral appliance can be suppressed.

For example, a thickness in an inside-outside direction of wings 4444 is smaller than that of protrusion portions 4462 of fixing portion 4460 and an outer side surface of each wing 4444 is provided at a position at which the outer side surface is substantially in plane with outer side surfaces of fixing portion 4460 and relevant protrusion portion 4462. Consequently, a space between each wing 4444 and upper jaw oral appliance body portion 4414 upon biting is larger than a space between each protrusion portion 4462 and upper jaw oral appliance body portion 4414 upon biting.

Protrusion portions 4462 are preferably provided further on the rear side of the outer side surface of fixing portion 4460 and are preferably provided at respective rear ends of the outer side surface (for example, the outer side surface of the first premolar teeth (tooth number: 4)). The skin of the inner cheeks of a human more easily stretches and shrinks further on the rear side. Therefore, as protrusion portions 4462 are provided further on the rear side, which means that protrusion portions 4462 are provided at respective positions corresponding to the parts, in which the skin more easily stretches and shrinks, of the inner cheeks of the user wearing oral appliance 4400, a feeling of fitting the user wearing oral appliance 4400 has is improved.

Also, a thickness in the inside-outside direction of protrusion portions 4462 is preferably smaller than that of fixing portion 4460 and an outer side surface of each protrusion portions 4462 is preferably provided at a position at which the outer side surface is on the inner side relative to an outer side surface of fixing portion 4460. Consequently, upon opening of the mouth during wearing, the user is less likely to have a feeling of discomfort due to protrusion portions 4462 abutting against the inner cheeks.

In the present embodiment, also, fixing portion 4460 may be detachably attachable to lower jaw oral appliance body portion 4424 but may be bonded to lower jaw oral appliance body portion 4424.

In the present embodiment, also, stopper fixing portions 4433 may be detachably attachable to upper jaw oral appliance body portion 4414 but may be bonded to upper jaw oral appliance body portion 4414.

According to the present embodiment, a majority of stress that is conventionally applied to wings 4444 of positioning members 4440 upon displacement in the right-left direction is applied to fixing portion 4460. Therefore, oral appliance 4400 according to the present embodiment enables suppression of damage of position adjustment sections 4450 by stress caused when wings 4444 of positioning members 4440 abut against upper jaw oral appliance body portion 4414 as a result of displacement in the right-left direction.

### [Variation of Embodiment 11]

In Embodiment 11, as in Embodiments 9 and 10, each stopper 4430 may include an extension portion (not illustrated in FIG. 23). Consequently, even when a user opens the mouth during wearing, a front surface of each stopper 4430 and a rear surface of relevant wing 4444 can abut against each other. Therefore, oral appliance 4400 according to the present embodiment is less likely to cause misalignment between each stopper 4430 and relevant positioning member 4440 and thus enables more effective suppression of rearward displacement of the lower jaw of the user wearing oral appliance 4400.

In this case, the extension portion of each stopper 4430 is disposed at a position at which when a lower jaw oral appliance is displaced in a right-left direction upon biting and relevant protrusion portion 4462 abuts upper jaw oral appliance 4410, stopper 4430 does not abut against lower jaw oral appliance 4420. Consequently, stress caused by displacement in the right-left direction is applied to protrusion portions 4462 of fixing portion 4460 but is less likely to be applied to stoppers 4430. Therefore, damage of stoppers 4430 due to displacement in the right-left direction of the lower jaw oral appliance can be suppressed.

For example, a thickness in an inside-outside direction of the extension portion of each stopper 4430 is smaller than that of protrusion portions 4462 of fixing portion 4460 and the extension portion of each stopper 4430 is provided at a position at which the outer side surface of the extension portion is substantially in plane with outer side surfaces of fixing portion 4460 and relevant protrusion portion 4462. Consequently, a space between the extension portion of each stopper 4430 and lower jaw oral appliance body portion 4424 upon biting is larger than a space between each protrusion portion 4462 and upper jaw oral appliance body portion 4414 upon biting.

Here, as in Embodiments 9 and 10, each stopper 4430 has a shape that when lower jaw oral appliance 4420 moves in a mouth opening direction during wearing, prevents stopper 4430 from limiting movement of relevant positioning member 4440. More specifically, each stopper 4430 may include an inclined portion that facilitates movement in the mouth opening direction of lower jaw oral appliance 4420. In other words, a front end portion of each stopper 4430 may include a rearward inclined surface including an upper end positioned further on the front side and a lower end positioned further on the rear side. The inclined portion of each stopper 4430 abuts against wing 4444 of relevant positioning member 4440 upon biting.

In each stopper 4430 including the inclined surface in the front end portion, the upper end of the front end portion thereof may only be positioned further on the front side and the rear end of the front end portion thereof may only be positioned further on the rear side. In this case, as in Embodiment 9, the front end portion of each stopper 4430 can have a shape of, for example, a cutout portion, a flat inclined portion, or a curved portion.

In this case, also, the front end portion of each stopper 4430 may be inclined rearward in the extension portion. Also, the extension portions may have a height that is equal to or less than half a height of stoppers 4430, and upon biting, a lower end of each extension portion may be positioned above a lower end surface of lower jaw oral appliance body portion 4424. In this case, also, the lower end of the front end portion of each stopper 4430 and a lower end of a rear end portion of relevant positioning member 4440 are preferably spaced from each other upon biting. Also, upon biting, the front surface of each stopper 4430 (front surface of each extension portion) and a rear surface of relevant positioning member 4440 are preferably spaced from each other below an occlusal surface, and also more preferably consistently spaced from each other when the lower jaw oral appliance and/or the upper jaw oral appliance are moved in the mouth opening direction.

In this case, also, from the perspective of suppression of damage of stoppers 4430 due to displacement in the right-left direction of the lower jaw oral appliance, where the front end portion of each stopper 4430 includes a cutout portion such as illustrated in FIG. 20A, the lower end of the cutout portion is preferably obtuse. Also, likewise, from the perspective of suppression of damage of stoppers 4430, the projection area of projection of each stopper 4430 to lower jaw oral appliance body portion 4424 from a lateral side direction upon biting is preferably 3 mm² or more. An upper limit of the projection area is not specifically provided. The projection area is, for example, preferably 3 mm² to 1000 mm², more preferably 3 mm² to 500 mm², further preferably 3 mm² to 450 mm², particularly preferably 50 mm² to 450 mm².

In this case, also, a shape and disposition of each of portions of stoppers 4430, the portions abutting against lower jaw oral appliance body portion 4424, and portions of lower jaw oral appliance 4420, the portions abutting against respective stoppers 4430, are those that do not limit forward movement of lower jaw oral appliance 4420. More specifically, each of abutment portions of lower jaw oral appliance body portion 4424, the abutment portions abutting against respective stoppers 4430, have a substantially flat shape. Also, a pair of abutment surfaces of the extension portions of the pair of stoppers 4430, the abutment surfaces abutting against lower jaw oral appliance 4420, are substantially parallel to each other, and abutment portions positioned in right and left parts of the outer side surface of lower jaw oral appliance body portion 4424, the abutment portions abutting against the respective extension portions of the pair of stoppers 4430, may be substantially parallel to each other.

In this case, also, as in Embodiment 10, positioning members 4440 may have a height that is equal to or lower than a height of lower jaw oral appliance body portion 4424. Furthermore, each positioning member 4440 may include an inclined surface in the rear surface thereof to make the respective surfaces of positioning member 4440 and relevant extension portion 434, the surfaces abutting against each other, substantially parallel to each other.

### [Variations of Embodiments 8 to 11]

Each of Embodiments 8 to 11 indicates an example of the fourth aspect of the present invention, and it is a matter of course that: the fourth aspect of the present invention is not limited to the above embodiments; and various other embodiments are possible within the scope of the idea of the fourth aspect of the present invention.

For example, in each of Embodiments 8 to 11, the positioning members and the stoppers are provided on the outer side surface of the lower or upper jaw oral appliance, but positioning members and stoppers may be provided on an inner side surface of a lower or upper jaw oral appliance. In this case, as a matter of course, a fixing portion and position adjustment sections are also provided in the inner side surface of the lower or upper jaw oral appliance.

Also, in each of Embodiments 8 to 11, each of the upper jaw oral appliance body portion and lower jaw oral appliance body portion includes a teeth row shape portion that can be fitted to a row of teeth. However, the upper jaw oral appliance body portion and the lower jaw oral appliance body portion may have a shape that is not a teeth row shape as long as the shape is a shape that receives a row of teeth, and each of the upper jaw oral appliance body portion and the lower jaw oral appliance body portion may only partly include a teeth row shape portion.

Also, the upper and lower jaw oral appliances may be each formed of a plurality of materials and may be formed by a combination of a part formed of an organic substance and a part formed of an inorganic substance.

Also, in each of Embodiments 8 to 11, each of the position adjustment sections adjusts a position of the relevant positioning member by means of adjustment of amounts of thread connection between the turnbuckle portion and the two screw rods using the turnbuckle mechanism, but for each of position adjustment sections, a jackscrew mechanism that can adjust a position of a positioning member by adjusting an amount of thread connection between a male screw and a female screw may be used. Also, each position adjustment section may move the relevant positioning member via a gear using a rack jack mechanism that can adjust a position of the positioning member fixed to a rack by adjusting an amount of rotation of the rack, and fix the positioning member via a screw. Each position adjustment section may adjust a position of a positioning member using another mechanism as long as such other mechanism can move the position of the positioning member and fix the positioning member at the adjusted position.

Also, in each of Embodiments 8 to 11, each of the position adjustment sections and the stopper adjustment sections uses the turnbuckle mechanism, but the position adjustment sections and the stopper adjustment sections may adjust positions of the positioning members and the moving portions using different mechanisms, respectively.

Also, in each of Embodiments 8 to 11, the position adjustment sections and the stopper adjustment sections are configured so that a movable width of the positioning members is smaller than a movable width of the moving portions of the stoppers. However, as long as no significant damage of the position adjustment sections and the stopper adjustment sections can occur, the respective movable widths of the positioning members and the moving portions of the stoppers may be the same or the moveable width of the positioning members may be larger than the movable width of the moving portions of the stoppers.

Also, in each of the variations of Embodiments 9, 10, and 11, the front end portion of each stopper is inclined further rearward in the extension portion. However, each stopper may start being inclined further rearward above an extension portion or may be inclined further rearward from a certain point partway of the extension portion.

Also, in each of Embodiments 8 to 11, a gap is provided between each wing and the upper jaw oral appliance, a gap is provided between each extension portion and the lower jaw oral appliance, and a gap is provided between each protrusion portion and the upper jaw oral appliance to allow movement in the right-left direction of the upper jaw and the lower jaw of a user wearing the oral appliance. However, a configuration in which no gaps are provided between the aforementioned members and no movement in the right-left direction of the upper jaw and the lower jaw of the user wearing the oral appliance is allowed may be employed.

Also, each of Embodiments 9 and 10, when the lower jaw oral appliance is displaced in the right-left direction upon biting and the wings thereby abut against the upper jaw oral appliance, the extension portions also abut against the lower jaw oral appliance. However, only each of members that are not prone to breaking from among the wings (positioning members) and the extension portions (stoppers) may be disposed at a position at which the member abuts against the upper or lower jaw oral appliance.

Also, in Embodiment 11, each of the wings is disposed at a position at which when the lower jaw oral appliance is displaced in the right-left direction upon biting and the protrusion portions of the fixing portion thereby abut against the upper jaw oral appliance, the wing does not abut against the upper jaw oral appliance. Also, in the variation of Embodiment 11, each of the wings and the extension portions is disposed at a position at which when the lower jaw oral appliance is displaced in the right-left direction upon biting and the protrusion portions thereby abut against the upper jaw oral appliance, the wing or the extension portion does not abut against the upper jaw oral appliance. However, the wings or the extension portions may abut against the upper jaw oral appliance simultaneously with the protrusion portions abutting against the upper jaw oral appliance.

In this case, also, stress caused by displacement in the right-left direction is distributed also to the protrusion portions of the fixing portion. Therefore, only small stress is applied to the wings or the extension portions, and damage of the position adjustment sections or the stopper adjustment sections due to an increase of the stress applied to the wings or the extension portions, each of which is a point of effort of a second-class lever, in the position adjustment sections or the stopper adjustment sections, each of which is a point of load of the second-class lever, are suppressed.

Consequently, displacement in the right-left direction of the lower jaw oral appliance is restricted by the protrusion portions and the wings or the extension portions simultaneously. Therefore, misalignment between the upper jaw oral appliance and the lower jaw oral appliance due to, for example, teeth grinding of a user wearing the oral appliance is effectively suppressed and the feeling of fitting is further improved.

Also, in Embodiment 11, where stress caused by displacement in the right-left direction is applied only to the protrusion portions of the fixing portion, the thickness in the inside-outside direction of the wings of the positioning members is made to be smaller than that of the protrusion portions of the fixing portion. Also, in the variation of Embodiment 11, where stress caused by displacement in the right-left direction is applied only to the protrusion portions of the fixing portion, the thicknesses in the inside-outside direction of the wings of the positioning members and the extension portions of the stoppers are made to be smaller than that of the protrusion portions of the fixing portion. However, positioning members or stoppers may be disposed on the outer side relative to the fixing portion to prevent relevant wings or relevant extension portions from abutting against an upper jaw oral appliance when a lower jaw oral appliance is displaced in a right-left direction. In this case, the outer side surfaces of the positioning members and the wings thereof or the outer side surfaces of the stoppers and the extension portions thereof may fail to be substantially in plane with respective outer side surfaces of the fixing portion and the protrusion portions.

Also, in the variations of Embodiments 9, 10, and 11, the extension portion of each stopper extends from each of the relevant stopper fixing portion and the relevant moving portion. However, an extension portion of each stopper may extend from only either a stopper fixing portion or a moving portion.

### [Example Configurations of Oral Appliance Relating to Fourth Aspect]

The fourth aspect of the present invention provides
an oral appliance including:
a pair of upper and lower jaw oral appliances facing each other;
a pair of positioning members each being adjustable in position in a front-rear direction, the pair of positioning members being provided in one oral appliance of the upper and lower jaw oral appliances; and
a pair of stoppers each being adjustable in position in the front-rear direction, the pair of stoppers being provided in another oral appliance of the upper and lower jaw oral appliances, the pair of stoppers abutting against the respective positioning members and thereby restricting rearward displacement of the lower jaw oral appliance.

In the oral appliance, a distance between each positioning member and a fixing portion that fixes the positioning member is decreased and thus, an excessive increase of stress applied to the positioning member in a relevant position adjustment section is suppressed. Therefore, the stoppers enable suppression of damage of the position adjustment sections due to displacement in the right-left direction of the lower jaw oral appliance.

The fourth aspect of the present invention provides
the oral appliance in which each of the positioning members includes a wing that abuts against the relevant stopper on an outer side of the other oral appliance.

In the oral appliance, the stoppers and the wings abut against each other on an outer side of an upper jaw oral appliance body portion, enabling limiting rearward displacement of the lower jaw of a user.

The fourth aspect of the present invention provides
the oral appliance in which a width of position adjustment of the positioning members is smaller than a width of position adjustment of the stoppers.

In the oral appliance, the position of each positioning member is less likely to be adjusted rearward, enabling suppression of damage of the relevant position adjustment section due to an excessive increase of stress applied to the positioning member, which is a point of load, in the relevant position adjustment section.

The fourth aspect of the present invention provides
the oral appliance in which each of the stoppers includes a fixing portion fixed to a side surface of the other oral appliance and a moving portion that is movable in the front-rear direction, and respective surfaces of the fixing portion and the moving portion, the surface facing each other, are substantially parallel to each other.

The oral appliance enables stress caused by abutment between the fixing portion and the moving portion to be distributed to a wider range, and thus, the fixing portion and the moving portion are less likely to be damaged.

The fourth aspect of the present invention provides
the oral appliance in which the stoppers have a height that is equal to or lower than a height of the other oral appliance.

In the oral appliance, when the lower jaw oral appliance is displaced in the right-left direction, the stoppers do not substantially abut against the lower jaw oral appliance, and thus, damage of stopper adjustment sections due to the displacement in the right-left direction of the lower jaw oral appliance is suppressed.

The fourth aspect of the present invention provides
the oral appliance in which each of the stoppers includes an extension portion that extends toward the one oral appliance.

In the oral appliance, stress caused by displacement in the right-left direction is distributed to the positioning members and the stoppers, enabling more effective suppression of damage of the position adjustment sections by the stress. In addition, in the oral appliance, damage of the stopper adjustment sections is less likely to occur because of the distribution of the stress.

The fourth aspect of the present invention provides
the oral appliance in which the extension portions have a height that is equal to or less than half the height of stoppers.

In the oral appliance, the extension portions do not limit movement in a mouth opening direction of the lower jaw oral appliance during wearing, facilitating movement in the mouth opening direction of the lower jaw oral appliance.

The fourth aspect of the present invention provides the oral appliance in which the stoppers and the positioning members have respective shapes that upon biting, allow respective end portions on the one oral appliance side of the stoppers and the positioning members to be spaced from each other.

The oral appliance facilitates movement in the mouth opening direction of the lower jaw oral appliance.

Also, the fourth aspect of the present invention provides the oral appliance in which the stoppers and the positioning members have respective shapes that upon biting or upon movement in the mouth opening direction of the lower jaw oral appliance, allows respective surfaces of the stoppers and the positioning members, the surfaces facing each other, to be spaced from each other on the one oral appliance side relative to an occlusal surface between the upper and lower jaw oral appliances.

The oral appliance facilitates movement in the mouth opening direction of the lower jaw oral appliance.

The fourth aspect of the present invention provides the oral appliance in which each of the stoppers has a shape in which a lower end of an end portion on the one oral appliance side of a front end portion of the stopper is obtuse.

The oral appliance suppresses damage of the stoppers due to displacement in the right-left direction of the lower jaw oral appliance.

The fourth aspect of the present invention provides the oral appliance in which each of the stoppers has a shape that allows a projection area of projection of the stopper to the one oral appliance from a lateral side direction upon biting is 3 mm² or more.

The oral appliance suppresses damage of the stoppers due to displacement in the right-left direction of the lower jaw oral appliance.

The fourth aspect of the present invention provides
the oral appliance in which the positioning members have a height that is equal to or lower than a height of the one oral appliance.

In the oral appliance, each of the stoppers is less likely to limit position adjustment of the relevant positioning member.

The fourth aspect of the present invention provides the oral appliance in which each of the stoppers includes an inclined portion that facilitates movement in the mouth opening direction of the lower jaw oral appliance.

The oral appliance makes it easy for the user wearing the oral appliance to open the mouth.

The fourth aspect of the present invention provides
the oral appliance in which abutment portions of the one oral appliance, each of the abutment portions abutting against the respective stoppers has a substantially flat shape.

In the oral appliance, the lower jaw oral appliance can be moved forward without movement of the lower jaw oral appliance being restricted by the extension portions.

The fourth aspect of the present invention provides
the oral appliance in which respective abutment surfaces of the pair of stoppers, the abutment surfaces abutting against the one oral appliance, are substantially parallel to each other and
abutment surfaces of the one oral appliance, the abutment surfaces abutting against the pair of stoppers, are parallel to each other.

In the oral appliance, the lower jaw oral appliance can be moved forward without movement of the lower jaw oral appliance being restricted by the extension portions.

The fourth aspect of the present invention provides
the oral appliance in which the positioning members are provided in the lower jaw oral appliance.

In the oral appliance, protrusion portions are provided at respective positions corresponding to the rear parts, in which the skin more easily stretches and shrinks, of the inner cheeks of the user wearing the oral appliance, and thus, a feeling of fitting the user wearing the oral appliance has is improved.

### 5. Oral Appliance Relating to Fifth Aspect

In an oral appliance relating to the fifth aspect of the present invention, when a lower jaw oral appliance is moved forward, an amount of movement of each position adjustment section is further decreased to decrease stress applied to the position adjustment section due to the principle of second-class lever and thereby suppress damage of the position adjustment section due to displacement in the right-left direction.

### [Embodiment 12]

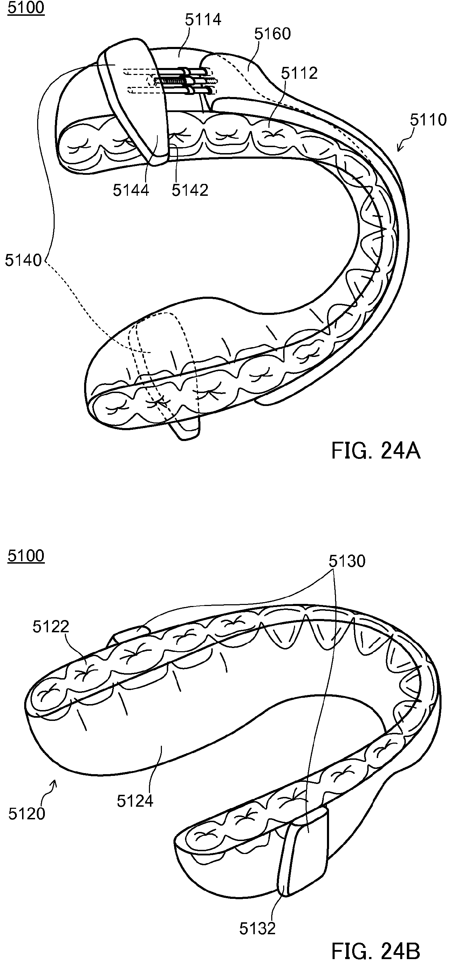

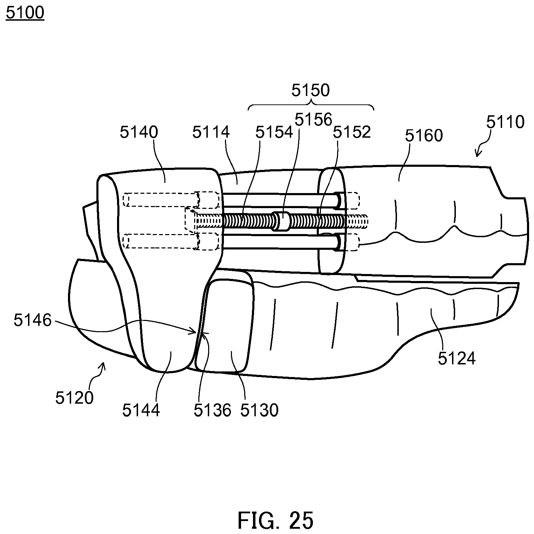

Oral appliance 5100 according to Embodiment 12 of the present invention includes a pair of upper and lower jaw oral appliances facing each other as illustrated in FIGS. 24A and 24B, which are schematic perspective views thereof, and FIG. 25, which is a side view thereof, and upper jaw oral appliance 5110 can be fitted to the upper jaw of a user and lower jaw oral appliance 5120 can be fitted to the lower jaw of the user.

As illustrated in FIGS. 24A, upper jaw oral appliance 5110 includes upper jaw oral appliance body portion 5114 with teeth row shape portion 5112 formed therein, teeth row shape portion 5112 being able to be fitted to a row of teeth of the upper jaw of the user, and on an outer side surface thereof, a pair of right and left positioning members 5140, a position of each of the pair of right and left positioning members 5140 being adjustable in a front-rear direction, is provided.

As illustrated in FIG. 24B, lower jaw oral appliance 5120 includes lower jaw oral appliance body portion 5124 with teeth row shape portion 5122 formed therein, teeth row shape portion 5122 being able to be fitted to a row of teeth of the lower jaw of the user, and on an outer side surface thereof, a pair of right and left stoppers 5130 is provided.

As illustrated in FIG. 25, the position of each positioning member 5140 can be adjusted to a position at which front surface 5142 of positioning member 5140 and rear surface 5132 of relevant stopper 5130 abut against each other when the user wearing upper jaw oral appliance 5110 and lower jaw oral appliance 5120 bites. The abutment between positioning members 5140 and stoppers 5130 limits rearward displacement of lower jaw oral appliance 5120 upon biting and thus limits rearward displacement of the lower jaw of the user wearing oral appliance 5100. Consequently, oral appliance 5100 can prevent narrowing of the airway of the user wearing oral appliance 5100 due to rearward displacement of the lower jaw of the user and thus suppress pauses in breathing or infrequent breathing during sleep.

Also, the positions of positioning members 5140 can be adjusted in the front-rear direction by respective position adjustment sections 5150 provided on the outer side surface of upper jaw oral appliance body portion 5114. The positions of positioning members 5140 are adjusted forward by position adjustment sections 5150, enabling positioning members 5140 to, upon biting, limit rearward displacement of lower jaw oral appliance 5120 while moving lower jaw oral appliance 5120 forward. Consequently, oral appliance 5100 allows the lower jaw of the user wearing oral appliance 5100 to be moved forward and thus allows the airway of the user to open more largely, enabling more effective suppression of pauses in breathing or infrequent breathing during sleep.

Each position adjustment section 5150 is provided on the front side relative to relevant positioning member 5140 and adjusts the position in the front-rear direction of positioning member 5140. More specifically, each position adjustment section 5150 is supported by fixing portion 5160 provided on the front side relative to relevant positioning member 5140 in upper jaw oral appliance 5110 while supporting positioning member 5140, and adjusts a space between fixing portion 5160 and positioning member 5140 to adjust the position of positioning member 5140.

More specifically, each position adjustment section 5150 includes male-threaded screw rod 5152 including an end fixed to fixing portion 5160 provided on the outer side surface of upper jaw oral appliance body portion 5114, male-threaded screw rod 5154 including an end fixed to positioning member 5140, and turnbuckle portion 5156 including a screw hole to which respective other end portions of screw rods 5152 and 5154 are threadably connected. Position adjustment section 5150 can adjust the position of positioning member 5140 fixed to and thereby supported by screw rod 5154, by inserting a rotation pin into a pin hole provided in turnbuckle portion 5156 and rotating turnbuckle portion 5156 to adjust amounts of the thread connection between turnbuckle portion 5156 and screw rods 5152 and 5154.

A front end of each position adjustment section 5150 (rear end of fixing portion 5160) may only be disposed at a position at which sufficient forward movement of the lower jaw of the user is enabled while damage of position adjustment section 5150 is suppressed. If the front end of each position adjustment section 5150 (rear end of fixing portion 5160) is provided at a position further on the front side (for example, a position corresponding to the relevant first premolar tooth (tooth number: 4)), a range of forward movement of the lower jaw of the user can further be widened, and if the front end of each position adjustment section 5150 (rear end of fixing portion 5160) is provided at a position further on the rear side (for example, a position corresponding to the relevant second premolar tooth (tooth number: 5) or the relevant first molar tooth (tooth number: 6)), damage of position adjustment section 5150 can more effectively be suppressed.

Each positioning member 5140 includes wing 5144 that protrudes downward from upper jaw oral appliance body portion 5114. A front surface of wing 5144 (which is also front surface 5142 of positioning member 5140, front surface 5142 abutting against relevant stopper 5130) is substantially parallel to rear surface 5132 of stopper 5130, and upon biting, abuts against rear surface 5132 of stopper 5130 on the outer side of lower jaw oral appliance body portion 5124 and wing 5144 thereby limits rearward displacement of the lower jaw of the user.

Upon biting, each stopper 5130 abuts against relevant positioning member 5140 from the side that is the same as position adjustment section 5150 and thereby limits rearward displacement of lower jaw oral appliance 5120.

In other words, each stopper 5130 is provided at a position on the outer side surface of lower jaw oral appliance 5120, at which upon biting, stopper 5130 is located on the front side relative to relevant positioning member 5140, and upon biting, rear surface 5132 of each stopper 5130 abuts against front surface 5142 of relevant positioning member 5140. In still other words, each stopper 5130 is disposed at a position at which a position in the front-rear direction of stopper 5130 is located between fixing portion 5160 and relevant positioning member 5140 upon biting. Consequently, a range of position adjustment in the front-rear direction of each positioning member 5140 can be widened, enabling an increase in the amount of forward movement of the lower jaw.

Stoppers 5130 have a height that is equal to or lower than a height of lower jaw oral appliance 5120. Note that the height of lower jaw oral appliance 5120 may vary depending on the position. In this case, the height of each stopper 5130 is preferably equal to or lower than a height of a part, in which stopper 5130 is provided, of lower jaw oral appliance body portion 5124. Consequently, each stopper 5130 does not limit adjustment of the position of relevant positioning member 5140 by relevant position adjustment section 5150, facilitating adjustment of the position of positioning member 5140.

Stoppers 5130 and wings 5144 have respective shapes that when lower jaw oral appliance 5120 is moved in a mouth opening direction during wearing, prevent wings 5144 from limiting movement of stoppers 5130. More specifically, each wing 5144 may include inclined portion 5146 that facilitates movement in the mouth opening direction of lower jaw oral appliance 5120. Also, each stopper 5130 may include inclined portion 5136 that facilitates movement in the mouth opening direction of lower jaw oral appliance 5120. In other words, each wing 5144 may include a rearward inclined surface in a front end portion thereof, the inclined surface including an upper end positioned further on the front side and a lower end positioned further on the rear side, and each stopper 5130 may include a rearward inclined surface in a rear end portion thereof, the inclined surface including an upper end positioned further on the front side and a lower end positioned further on the rear side. Inclined portions 5146 of wings 5144 and inclined portions 5136 of stoppers 5130 abut against each other upon biting.

When lower jaw oral appliance 5120 is moved in the mouth opening direction during wearing, lower jaw oral appliance 5120 is moved on a circular arc-like path. In such movement, movement of stoppers 5130 is not limited by wings 5144 as a result of the front end portions of wings 5144 and the rear end portions of stoppers 5130 including the respective inclined surfaces described above, making it easy for the user wearing oral appliance 5100 to open the mouth.

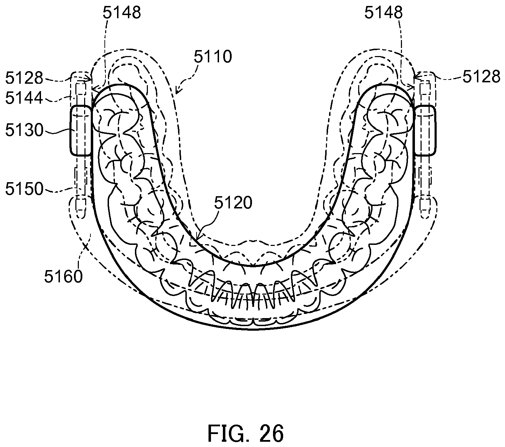

Also, a shape and disposition of each of portions of positioning members 5140, the portions abutting against lower jaw oral appliance body portion 5124, that is, portions of wings 5144, the portions abutting against lower jaw oral appliance 5120, are those that do not limit forward movement of lower jaw oral appliance 5120. More specifically, as illustrated in FIG. 26, which is a schematic plan view of oral appliance 5100 (the upper jaw oral appliance, the fixing portion, the position adjustment sections, and the positioning members are each indicated by a dashed line and the lower jaw oral appliance and the stoppers are each indicated by a solid line), each of abutment portions 5128 of lower jaw oral appliance body portion 5124, abutment portions 5128 abutting against respective wings 5144, has a substantially flat shape. Also, a pair of abutment surfaces 5148 of the pair of wings 5144, abutment surfaces 5148 abutting against lower jaw oral appliance 5120, are substantially parallel to each other and abutment portions 5128 positioned in right and left parts of the outer side surface of lower jaw oral appliance body portion 5124, abutment portions 5128 abutting against the pair of wings 5144, are substantially parallel to each other.

Consequently, lower jaw oral appliance 5120 can be moved forward without movement of lower jaw oral appliance 5120 being restricted by wings 5144. In this case, for example, a thickness in the right-left direction of each part, in the vicinity of the back teeth, of lower jaw oral appliance body portion 5124, is larger on the front side (for example, a part, in the vicinity of the first molar tooth (tooth number: 6), of the outer side surface) and is smaller on the rear side (for example, a part, in the vicinity of the second molar tooth (tooth number: 7) or the third molar tooth (tooth number: 8), of the outer side surface).

Stoppers 5130 may be detachably attachable to lower jaw oral appliance body portion 5124 but may be bonded to lower jaw oral appliance body portion 5124. Alternatively, stoppers 5130 may be integrated with the outer side surface of lower jaw oral appliance body portion 5124.

Fixing portion 5160 may be detachably attachable to upper jaw oral appliance body portion 5114 but may be bonded to upper jaw oral appliance body portion 5114. If fixing portion 5160 is bonded to upper jaw oral appliance body portion 5114, misalignment of fixing portion 5160 and positioning members 5140 is less likely to occur during wearing.

According to the present embodiment, an amount of extension of each position adjustment section 5150 when the lower jaw is advanced can be decreased. Therefore, in oral appliance 5100 according to the present embodiment, a distance between fixing portion 5160, which is a fulcrum of a second-class lever, and each wing 5144, which is a point of effort of the second-class lever, is decreased to suppress damage of position adjustment sections 5150 due to an excessive increase of stress applied to each wing 5144 when the lower jaw oral appliance is displaced in the right-left direction, in relevant position adjustment section 5150, which is a point of load of the second-class lever.

In particular, the effect of suppression of damage of position adjustment sections 5150 is significant when the position of the front end of each position adjustment section 5150 is located further on the rear side (for example, a position corresponding to the first premolar tooth (tooth number: 5)).

Also, according to the present embodiment, wings 5144 are provided at respective positions corresponding to the rear parts, in which the skin more easily stretches and shrinks, of the inner cheeks of the user wearing oral appliance 5100, and thus, a feeling of fitting the user wearing oral appliance 5100 is improved.

### [Embodiment 13]

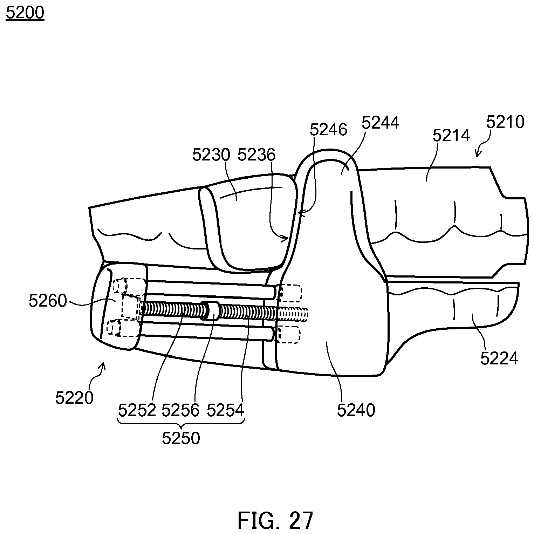

Oral appliance 5200 according to Embodiment 13 of the present invention includes a pair of upper and lower jaw oral appliances facing each other as illustrated in FIG. 27, which is a schematic side view thereof, and upper jaw oral appliance 5210 can be fitted to the upper jaw of a user and lower jaw oral appliance 5220 can be fitted to the lower jaw of the user. Also, a pair of right and left positioning members 5240 which are adjustable in position in a front-rear direction is provided on an outer side surface of lower jaw oral appliance body portion 5224 and a pair of right and left stoppers 5230 is provided on an outer side surface of upper jaw oral appliance body portion 5214. In the below, description of components that are in common with Embodiment 12 may be omitted.

In the present embodiment, as illustrated in FIG. 27, the position of each positioning member 5240 can be adjusted to a position at which a rear surface of positioning member 5240 and a front surface of relevant stopper 5230 abut against each other upon biting. The abutment between positioning members 5240 and stoppers 5230 limits rearward displacement of lower jaw oral appliance 5220 upon biting and thereby limits rearward displacement of the lower jaw of the user wearing oral appliance 5200.

Also, the position of each positioning member 5240 can be adjusted in the front-rear direction by relevant position adjustment section 5250 provided on the outer side surface of lower jaw oral appliance body portion 5224. The position of each positioning member 5240 is adjusted rearward by relevant position adjustment section 5250, enabling, upon biting, limiting rearward displacement of lower jaw oral appliance 5220 while moving lower jaw oral appliance 5220 forward.

Each position adjustment section 5250 is provided on the rear side relative to relevant positioning member 5240 and adjusts the position in the front-rear direction of positioning member 5240. More specifically, each position adjustment section 5250 is supported by fixing portion 5260 provided on the rear side relative to relevant positioning member 5240 in lower jaw oral appliance 5220 while supporting positioning member 5240, and adjusts a space between fixing portion 5260 and positioning member 5240 to adjust the position of positioning member 5240.

More specifically, each position adjustment section 5250 includes male-threaded screw rod 5252 including an end fixed to fixing portion 5260 provided on the outer side surface of lower jaw oral appliance body portion 5224, male-threaded screw rod 5254 including an end fixed to positioning member 5240, and turnbuckle portion 5256 including a screw hole to which respective other end portions of screw rods 5252 and 5254 are threadably connected. Position adjustment section 5250 can adjust the position of positioning member 5240 fixed to and thereby supported by screw rod 5254, by inserting a rotation pin into a pin hole provided in turnbuckle portion 5256 and rotating turnbuckle portion 5256 to adjust amounts of the thread connection between turnbuckle portion 5256 and screw rods 5252 and 5254.

Each positioning member 5240 includes wing 5244 that protrudes upward from lower jaw oral appliance body portion 5224. A rear surface of wing 5244 (which is also a rear surface of positioning member 5240, the rear surface abutting against relevant stopper 5230) is substantially parallel to a front surface of stopper 5230, and upon biting, abuts against the front surface of stopper 5230 on the outer side of upper jaw oral appliance body portion 5214 and wing 5244 thereby limits rearward displacement of the lower jaw of the user.

Upon biting, each stopper 5230 abuts against relevant positioning member 5240 from the side that is the same as position adjustment section 5250 and thereby limits rearward displacement of lower jaw oral appliance 5220.

In other words, each stopper 5230 is provided at a position on the outer side surface of lower jaw oral appliance 5220, at which upon biting, stopper 5230 is located on the rear side relative to relevant positioning member 5240, and upon biting, the front surface of stopper 5230 abuts against the rear surface of relevant positioning member 5240. In still other words, each stopper 5230 is disposed at a position at which a position in the front-rear direction of stopper 5230 is located between fixing portion 5260 and relevant positioning member 5240 upon biting. Consequently, a range of position adjustment in the front-rear direction of each positioning member 5240 can be widened, enabling an increase in the amount of forward movement of the lower jaw.

Also, each stopper 5230 may only be disposed at a position at which sufficient forward movement of the lower jaw of the user is enabled while damage of position adjustment section 5250 is suppressed. If each stopper 5230 is provided at a position further on the front side (for example, a position corresponding to the relevant first molar tooth (tooth number: 6)), a range of forward movement of the lower jaw of the user can further be widened, and if each stopper 5230 is provided at a position further on the rear side (for example, a position corresponding to the relevant second molar tooth (tooth number: 7)), damage of position adjustment section 5150 can more effectively be suppressed.

Stoppers 5230 have a height that is equal to or lower than a height of upper jaw oral appliance 5210. Note that the height of upper jaw oral appliance 5210 may vary depending on the position. In this case, the height of each stopper 5230 is preferably equal to or lower than a height of a part, in which stopper 5230 is provided, of upper jaw oral appliance body portion 5214. Consequently, each stopper 5230 does not limit adjustment of the position of relevant positioning member 5240 by relevant position adjustment section 5250, facilitating adjustment of the position of positioning member 5240.

Stoppers 5230 and wings 5244 have respective shapes that when lower jaw oral appliance 5220 is moved in a mouth opening direction during wearing, prevents stoppers 5230 from limiting movement of wings 5244. More specifically, each stopper 5230 may include inclined portion 5236 that facilitates movement in the mouth opening direction of lower jaw oral appliance 5220. Also, each wing 5244 may include inclined portion 5246 that facilitates movement in the mouth opening direction of lower jaw oral appliance 5220. In other words, each stopper 5230 may include a rearward inclined surface in a front end portion thereof, the inclined surface including an upper end positioned further on the front side and a lower end positioned further on the rear side, and each wing 5244 may include a rearward inclined surface in a rear end portion thereof, the inclined surface including an upper end positioned further on the front side and a lower end positioned further on the rear side. Inclined portions 5236 of stoppers 5230 and inclined portions 5246 of wings 5244 abut against each other upon biting.

In Embodiment 13, also, stoppers 5230 may be detachably attachable to upper jaw oral appliance body portion 5214 but may be bonded to upper jaw oral appliance body portion 5214.

In Embodiment 13, also, fixing portion 5260 may be detachably attachable to lower jaw oral appliance body portion 5224 but may be bonded to lower jaw oral appliance body portion 5224.

According to the present embodiment, there is no need to adjust a thickness in the right-left direction of lower jaw oral appliance 5220 to facilitate forward movement of lower jaw oral appliance 5220, a feeling of fitting the user wearing oral appliance 5200 has is improved.

In other words, one of oral appliance 5100 according to Embodiment 12 and oral appliance 5200 according to Embodiment 13, one conforming to the shapes of the rows of teeth of a user and thus providing an improved feeling of fitting, can arbitrarily be selected.

### [Embodiment 14]

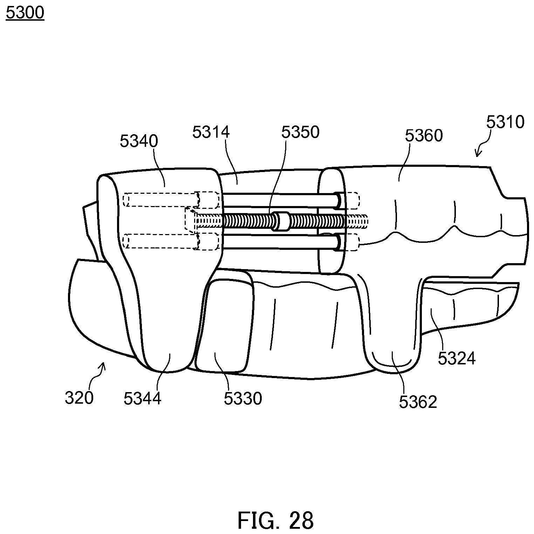

Oral appliance 5300 according to Embodiment 14 of the present invention includes a pair of upper and lower jaw oral appliances facing each other as illustrated in FIG. 28, which is a schematic side view thereof, and upper jaw oral appliance 5310 can be fitted to the upper jaw of a user and lower jaw oral appliance 5320 can be fitted to the lower jaw of the user. Also, as in Embodiment 12, a pair of right and left positioning members 5340 which are adjustable in position in a front-rear direction is provided on an outer side surface of upper jaw oral appliance body portion 5314 and a pair of right and left stoppers 5330 is provided on an outer side surface of lower jaw oral appliance body portion 5324. In the below, description of components that are in common with Embodiment 12 may be omitted.

In the present embodiment, also, the positions of positioning members 5340 can be adjusted in the front-rear direction by respective position adjustment sections 5350 provided on the outer side surface of upper jaw oral appliance body portion 5314. The positions of positioning members 5340 are adjusted forward by position adjustment sections 5350, enabling, upon biting, limiting rearward displacement of lower jaw oral appliance 5320 while moving lower jaw oral appliance 5320 forward.

In the present embodiment, also, each positioning member 5340 includes wing 5344 that protrudes downward from upper jaw oral appliance body portion 5314. A front surface of wing 5344, the front surface abutting against relevant stopper 5330, is substantially parallel to a rear surface of stopper 5330, and upon biting, abuts against the rear surface of stopper 5330 on the outer side of lower jaw oral appliance body portion 5324 and wing 5344 thereby limits rearward displacement of the lower jaw of the user.

In the present embodiment, also, upon biting, each stopper 5330 abuts against relevant positioning member 5340 from the side that is the same as position adjustment section 5350 and thereby limits rearward displacement of lower jaw oral appliance 5320. In other words, each stopper 5330 is provided at a position on the outer side surface of lower jaw oral appliance 5320, at which upon biting, stopper 5330 is located on the front side relative to relevant positioning member 5340, and upon biting, rear surface 332 of stopper 5330 abuts against front surface 342 of positioning member 5340. In still other words, each stopper 5330 is disposed at a position at which a position in the front-rear direction of stopper 5330 is located between fixing portion 5360 and positioning member 5340 upon biting.

In the present embodiment, also, stoppers 5330 preferably have a height that is equal to or lower than a height of lower jaw oral appliance 5320.

In the present embodiment, fixing portion 5360 includes protrusion portions 5362 that protrude downward from upper jaw oral appliance 5310. When lower jaw oral appliance 5320 is displaced in the right-left direction upon biting, protrusion portions 5362 abut against lower jaw oral appliance 5320 and thereby restrict the displacement in the right-left direction of lower jaw oral appliance 5320.

In this case, each positioning member 5340 is disposed at a position at which when the lower jaw oral appliance is displaced in the right-left direction upon biting and protrusion portions 5362 abut against lower jaw oral appliance 5320, the positioning member 5340 is less likely to abut against lower jaw oral appliance 5320. Consequently, a majority of stress caused by the displacement in the right-left direction is applied to protrusion portions 5362 of fixing portion 5360 and is less likely to be applied to positioning members 5340. Therefore, damage of position adjustment sections 5350 due to displacement in the right-left direction of lower jaw oral appliance 5320 can be suppressed.

For example, a thickness in an inside-outside direction of wings 5344 is smaller than that of protrusion portions 5362 of fixing portion 5360 and an outer side surface of each wing 5344 is provided at a position at which the outer side surface is substantially in plane with outer side surfaces of fixing portion 5360 and relevant protrusion portion 5362. Consequently, a space between each wing 5344 and lower jaw oral appliance body portion 5324 upon biting is larger than a space between each protrusion portion 5362 and lower jaw oral appliance body portion 5324 upon biting.

Protrusion portions 5362 are preferably provided further on the rear side of the outer side surface of fixing portion 5360 and are preferably provided at respective rear ends of the outer side surface. Consequently, as protrusion portions 5362 are provided at respective positions corresponding to the rear parts, in which the skin more easily stretches and shrinks, of the inner cheeks of the user wearing oral appliance 5300, a feeling of fitting the user wearing oral appliance 5300 has is improved.

Also, a thickness in the inside-outside direction of protrusion portions 5362 is preferably smaller than that of fixing portion 5360 and an outer side surface of each protrusion portion 5362 is preferably provided at a position at which the outer side surface is located on the inner side relative to the outer side surface of fixing portion 5360. Consequently, upon opening of the mouth during wearing, the user is less likely to have a feeling of discomfort due to protrusion portions 5362 abutting against the inner cheeks.

In Embodiment 14, also, each stopper 5330 may be detachably attachable to lower jaw oral appliance body portion 5324 but may be bonded to lower jaw oral appliance body portion 5324.

In Embodiment 14, also, fixing portion 5360 may be detachably attachable to upper jaw oral appliance body portion 5314 but may be bonded to upper jaw oral appliance body portion 5314.

According to the present embodiment, a majority of stress that is conventionally applied only to wings 5344 of positioning members 5340 upon displacement in the right-left direction is applied mainly to protrusion portions 5362 of fixing portion 5360. Therefore, oral appliance 5300 according to the present embodiment enables more effective suppression of damage of position adjustment sections 5350 by stress caused when wings 5344 of positioning members 5340 abut against upper jaw oral appliance body portion 5314 upon displacement in the right-left direction.

In the present embodiment, as in Embodiment 13, the positioning members may be provided in the lower jaw oral appliance and the stoppers may be provided in the upper jaw oral appliance. In this case, as a matter of course, the fixing portion and the position adjustment sections are also provided in the lower jaw oral appliance. Also, in this case, the fixing portion and the position adjustment sections are disposed on the rear side relative to the positioning members and the stoppers are also disposed on the rear side relative to the positioning members. Also, in this case, the front surface and the front end portion of each positioning member are deemed to be replaced with a rear surface and a rear end portion of each positioning member, and the rear surface and the rear end portion of each stopper are deemed to be replaced with a front surface and a front end portion of each stopper.

### [Embodiment 15]

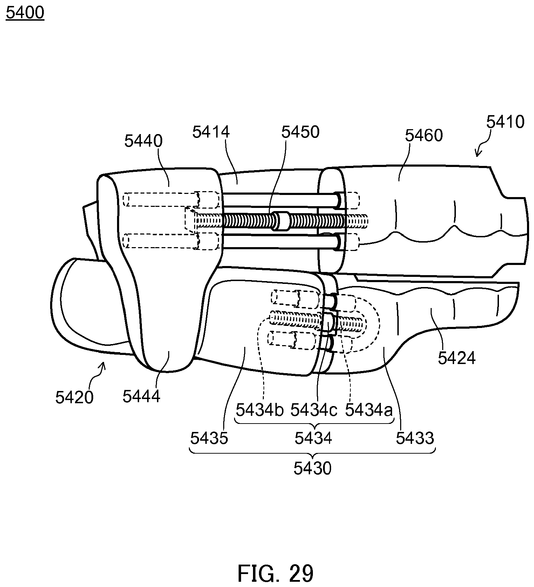

Oral appliance 5400 according to Embodiment 15 of the present invention includes a pair of upper and lower jaw oral appliances facing each other as illustrated in FIG. 29, which is a schematic side view thereof, and upper jaw oral appliance 5410 can be fitted to the upper jaw of a user and lower jaw oral appliance 5420 can be fitted to the lower jaw of the user. Also, as in Embodiment 12, a pair of right and left positioning members 5440 which are adjustable in position in a front-rear direction is provided on an outer side surface of upper jaw oral appliance body portion 5414 and a pair of right and left stoppers 5430 is provided on an outer side surface of lower jaw oral appliance body portion 5424. In the below, description of components that are in common with Embodiment 12 may be omitted.

In the present embodiment, also, the positions of positioning members 5440 can be adjusted in the front-rear direction by respective position adjustment sections 5450 provided on the outer side surface of upper jaw oral appliance body portion 5414. The positions of positioning members 5440 are adjusted forward by position adjustment sections 5450 to, upon biting, limit rearward displacement of lower jaw oral appliance 5420 while moving lower jaw oral appliance 5420 forward.

In the present embodiment, also, each positioning member 5440 includes wing 5444 that protrudes downward from upper jaw oral appliance body portion 5414. A front surface of wing 5444, the front surface abutting against relevant stopper 5430, is substantially parallel to a rear surface of stopper 5430, and upon biting, abuts against the rear surface of stopper 5430 on the outer side of lower jaw oral appliance body portion 5424 and wing thereby limits rearward displacement of the lower jaw of the user.

In the present embodiment, each stopper 5430 includes moving portion 5433 that abuts against positioning member 5440, stopper adjustment section 5434 that adjusts a position of moving portion 5433 in the front-rear direction, and stopper fixing portion 5435 fixed to the outer side surface of lower jaw oral appliance body portion 5424. Respective surfaces of moving portion 5433 and stopper fixing portion 5435, the surfaces facing each other, are substantially parallel to each other.

The position of each moving portion 5433 is adjusted rearward by relevant stopper adjustment section 5434 and moving portion 5433 thereby abuts against relevant positioning member 5440, enabling, upon biting, limiting rearward displacement of lower jaw oral appliance 5420 while making lower jaw oral appliance 5420 protrude forward.

Each stopper adjustment section 5434 includes male-threaded screw rod 5434a including an end fixed to relevant stopper fixing portion 5435, male-threaded screw rod 5434b including an end fixed to moving portion 5433, and turnbuckle portion 5434c including a screw hole to which respective other ends of the screw rods 5434a and 5434b are threadably connected. Stopper adjustment section 5434 can adjust the position of moving portion 5433 fixed to screw rod 5434b, by inserting a rotation pin into a pin hole provided in turnbuckle portion 5434c and rotating turnbuckle portion 5434c to adjust amounts of the thread connection between turnbuckle portion 5434c and screw rods 5434a and 5434b.

According to the present embodiment, when lower jaw oral appliance 5420 is moved forward by adjusting the position of each moving portion 5433 rearward, an amount of movement of relevant position adjustment section 5450 can be decreased. Therefore, a distance between each wing 5444, which is a point of effort of a second-class lever, and fixing portion 5460, which is a fulcrum of the second-class lever, can be decreased by decreasing a length of the relevant position adjustment section 5450. Therefore, damage of relevant position adjustment section 5450 due to an excessive increase of stress applied to wing 5444 when the lower jaw oral appliance is displaced in the right-left direction, in relevant position adjustment section 5450, which is a point of load of the second-class lever, can be suppressed.

On the other hand, according to the present embodiment, an amount of forward movement of lower jaw oral appliance 5420 can be adjusted by adjustment of the positions of moving portions 5433 of stoppers 5430 by stopper adjustment sections 5434 in addition to adjustment of the positions of positioning members 5440 by position adjustment sections 5450. Therefore, the amount of forward movement of lower jaw oral appliance 5420 can be increased in comparison with conventional oral appliances in which an amount of forward movement of a lower jaw oral appliance is adjusted by position adjustment sections alone.

In the present embodiment, also, as in Embodiment 13, the positioning members may be provided in the lower jaw oral appliance and the stoppers may be provided in the upper jaw oral appliance. In this case, as a matter of course, the fixing portion and the position adjustment sections are also provided in the lower jaw oral appliance. Also, in this case, the fixing portion and the position adjustment sections are disposed on the rear side relative to the positioning members and the stoppers are also disposed on the rear side relative to the positioning members. Also, in this case, the front surface and the front end portion of each positioning member are deemed to be replaced with a rear surface and a rear end portion of each positioning member and the rear surface and the rear end portion of each stopper are deemed to be replaced with a front surface and a front end portion of each stopper.

### [Variations of Embodiments 12 to 15]

Each of Embodiments 12 to 15 indicates an example of the fifth aspect of the present invention, and it is a matter of course that: the fifth aspect of the present invention is not limited to the above embodiments; and various other embodiments are possible within the scope of the idea of the fifth aspect of the present invention.

For example, in each of Embodiments 12 to 15, the positioning members and the stoppers are provided on the outer side surface of the lower or upper jaw oral appliance, but the positioning members and the stoppers may be provided on an inner side surface of the lower or upper jaw oral appliance. In this case, as a matter of course, the fixing portion and the position adjustment sections are also provided on the inner side surface of the lower or upper jaw oral appliance.

Also, in each of Embodiments 12 to 15, the upper jaw oral appliance body portion and the lower jaw oral appliance body portion each include a teeth row shape portion that can be fitted to a row of teeth. However, the upper jaw oral appliance body portion and the lower jaw oral appliance body portion may have a shape that is not a teeth row shape as long as the shape is a shape that receives a row of teeth and each of the upper jaw oral appliance body portion and the lower jaw oral appliance body portion may only partly include a teeth row shape portion.

Also, the upper and lower jaw oral appliances may be each formed of a plurality of materials and may be formed by a combination of a part formed of an organic substance and a part formed of an inorganic substance.

Also, in each of Embodiments 12 to 15, each of the position adjustment sections (and the stopper adjustment sections in Embodiment 14) adjusts the position of the relevant positioning member by means of adjustment of amounts of thread connection between the turnbuckle portion and the two screw rods using the turnbuckle mechanism, but for each of position adjustment sections (and stopper adjustment sections), a jackscrew mechanism that can adjust a position of a positioning member by adjusting an amount of thread connection between a male screw and a female screw may be used. Also, each position adjustment section (and each stopper adjustment section) may move the relevant positioning member via a gear using a rack jack mechanism that can adjust a position of the positioning member fixed to a rack by adjusting an amount of rotation of the rack, and fix the positioning member via a screw. Each position adjustment section (and each stopper adjustment section) may adjust a position of a positioning member using another mechanism as long as such other mechanism can move the position of the positioning member and fix the positioning member at the adjusted position.

Also, in each of Embodiments 12 to 15, a gap is provided between each wing and the upper or lower jaw oral appliance and a gap is provided between each protrusion portion and the upper or lower jaw oral appliance to allow movement in the right-left direction of the upper jaw and the lower jaw of a user wearing the oral appliance. However, a configuration in which no gaps are provided between the aforementioned members and no movement in a right-left direction of the upper jaw and the lower jaw of a user wearing the oral appliance is allowed may be employed.

Also, in Embodiment 14, each of the wings is disposed at a position at which when the lower jaw oral appliance is displaced in the right-left direction upon biting and the protrusion portions abut against the lower jaw oral appliance, the wing does not abut against the lower jaw oral appliance. However, the wings may abut against the lower jaw oral appliance simultaneously with the protrusion portions abutting against the lower jaw oral appliance.

Consequently, stress caused by displacement in the right-left direction is applied not only to the wings of the positioning members but also to the protrusion portions of the fixing portion and is thus distributed to both the wings and the protrusion portions. Therefore, only small stress is applied to the wings and damage of the position adjustment sections due to an increase of the stress applied to the wings, each of which is a point of effort of a second-class lever, in the position adjustment sections, each of which is a point of load of the second-class lever, is suppressed.

Consequently, displacement in the right-left direction of the lower jaw oral appliance is restricted by the wings and the protrusion portions simultaneously. Therefore, misalignment between the upper jaw oral appliance and the lower jaw oral appliance due to, for example, teeth grinding of a user wearing the oral appliance is more effectively suppressed and the feeling of fitting is further improved.

Also, in Embodiment 14, where stress caused by displacement in the right-left direction is applied only to the protrusion portions of the fixing portion, the thickness in the inside-outside direction of the wings of the positioning members is made to be smaller than that of the protrusion portions of the fixing portion. However, the positioning members may be disposed on the outer side relative to the respective stoppers or the fixing portion to prevent the respective wings from abutting against the lower jaw oral appliance when the lower jaw oral appliance is displaced in a right-left direction. In this case, outer side surfaces of the positioning members and the wings thereof may fail to be substantially in plane with respective outer side surfaces of the fixing portion and the protrusion portions thereof.

### [Example Configurations of Oral Appliance According to Fifth Aspect]

The fifth aspect of the present invention provides
an oral appliance including:
a pair of upper and lower jaw oral appliances facing each other;
a pair of positioning members each being adjustable in position in a front-rear direction, the pair of positioning members being provided in one oral appliance of the upper and lower jaw oral appliances;
position adjustment sections provided on a front or rear side relative to the respective positioning members of the one oral appliance, the position adjustment sections adjusting the respective positions in the front-rear direction of the positioning members; and
a pair of stoppers provided in another oral appliance of the upper and lower jaw oral appliances, each of the pair of stoppers abutting against the relevant positioning member from a side that is the same as the relevant position adjustment section and thereby restricts rearward displacement of the lower jaw oral appliance.

In the oral appliance, position adjustment sections for adjusting respective positions of the lower jaw oral appliance and the upper jaw oral appliance upon biting are less likely to be broken even by displacement in a right-left direction upon biting.

The fifth aspect of the present invention further provides
the oral appliance in which the stoppers have a height that is equal to or lower than a height of the other oral appliance.

In the oral appliance, the stoppers are less likely to limit adjustment of the positions of the respective positioning members.

The fifth aspect of the present invention further provides
the oral appliance in which:
each position adjustment section is supported by a fixing portion provided in the one oral appliance while supporting the relevant positioning member; and
each stopper is disposed between the fixing portion and the relevant positioning member.

In the oral appliance, the position adjustment sections for adjusting the positions of the lower jaw oral appliance and the upper jaw oral appliance upon biting are less likely to be broken even by displacement in the right-left direction upon biting.

The fifth aspect of the present invention further provides
the oral appliance in which the fixing portion includes protrusion portions that abut against the other oral appliance and thereby restrict displacement in the right-left direction of the upper jaw oral appliance and the lower jaw oral appliance.

In the oral appliance, stress due to displacement in the right-left direction is applied also to the protrusion portions of the fixing portion and is less likely to be applied to the positioning members. Therefore, the oral appliance enables suppression of damage of the position adjustment sections due to displacement in the right-left direction of the lower jaw oral appliance.

The fifth aspect of the present invention further provides
the oral appliance in which each positioning member is disposed at a position at which when the relevant protrusion portion abuts against the other oral appliance, the positioning member does not abut against the other oral appliance.

In the oral appliance, a majority of stress caused by displacement in the right-left direction is applied to the protrusion portions of the fixing portion and thus is less likely to be applied to the positioning members. Therefore, the oral appliance enables suppression of damage of the position adjustment sections due to displacement in the right-left direction of the lower jaw oral appliance.

The fifth aspect of the present invention further provides
the oral appliance in which respective abutment surfaces of each positioning member and the relevant stopper, the abutment surfaces abutting against each other, are substantially parallel to each other.

In the oral appliance, stress caused by abutment between the positioning members and the stoppers can more broadly be distributed and the positioning members and the stoppers are further less likely to be damaged.

The fifth aspect of the present invention further provides
the oral appliance in which the pair of positioning members include a pair of wings that abut against the respective stoppers on the outer side of the other oral appliance.

In the oral appliance, the stoppers and the wings abut against each other on the outer side of a lower jaw oral appliance body portion, enabling limiting rearward displacement of the lower jaw of a user.

The fifth aspect of the present invention further provides
the oral appliance in which each of the wings includes an inclined portion that abuts against the relevant stopper upon biting and facilitates movement in a mouth opening direction of the lower jaw oral appliance.

The oral appliance makes it easy for a user wearing the oral appliance to open the mouth and further reduces a feeling of discomfort the user has.

The fifth aspect of the present invention further provides
the oral appliance in which each of the wings includes an inclined portion that abuts against the relevant stopper upon biting and facilitates movement in the mouth opening direction of the lower jaw oral appliance.

The oral appliance makes it easy for a user wearing the oral appliance to open the mouth and further reduces a feeling of discomfort the user has.

The fifth aspect of the present invention further provides
the oral appliance in which each of abutment portions of the other oral appliance, the abutment portions abutting the respective wings, has a substantially flat shape.

In the oral appliance, the lower jaw oral appliance can be moved forward without movement of the lower jaw oral appliance being restricted by the wings.

The fifth aspect of the present invention further provides
the oral appliance in which:
respective abutment surfaces of the pair of wings, the abutment surfaces abutting against the other oral appliance, are substantially parallel to each other; and
abutment surfaces of the other oral appliance, the abutment surfaces abutting the pair of wings, respectively, are substantially parallel to each other.

In the oral appliance, the lower jaw oral appliance can be moved forward without movement of the lower jaw oral appliance being restricted by extension portions.

The fifth aspect of the present invention further provides
the oral appliance in which each of the stoppers includes a fixing portion fixed to a side surface of the other oral appliance and a moving portion that is movable in the front-rear direction and respective surfaces of the fixing portion and the moving portion, the surfaces facing each other, are substantially parallel to each other.

In the oral appliance, a distance between each positioning member and the fixing portion that fixes the positioning member is decreased, suppressing an excessive increase of stress applied to the positioning members, in the position adjustment sections. Therefore, the stoppers enable suppression of damage of the position adjustment sections due to displacement in the right-left direction of the lower jaw oral appliance.

The fifth aspect of the present invention further provides
the oral appliance in which the stoppers are provided in the lower jaw oral appliance.

In the oral appliance, the protrusion portions are provided at respective positions corresponding to the rear parts, in which the skin more easily stretches and shrinks, of the inner cheeks of a user wearing the oral appliance, and thus, a feeling of fitting the user wearing the oral appliance has is improved.

### 6. Oral Appliance Relating to Sixth Aspect

In the sixth aspect of the present invention, either of upper and lower jaw oral appliances is fitted to only a part of a row of teeth to reduce a feeling of discomfort a user has because of wearing the oral appliance and thereby improve a feeling of fitting the user has.

### [Embodiment 16]

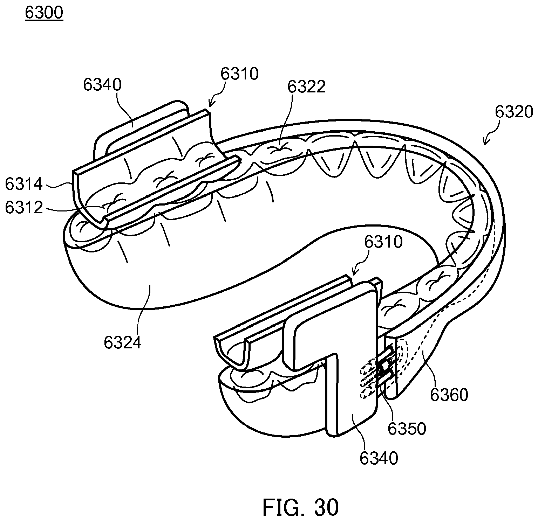

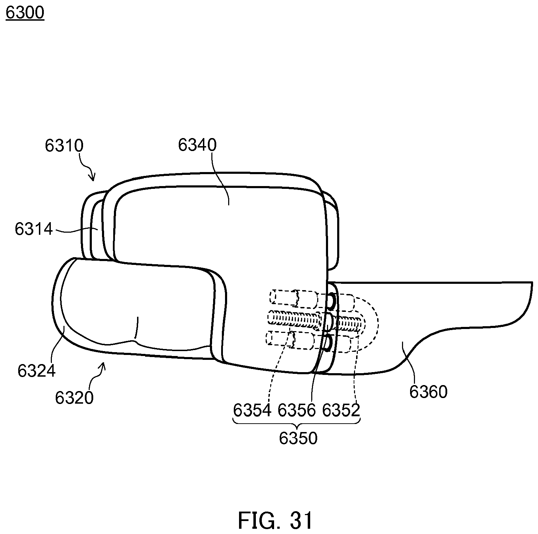

Oral appliance 6300 according to Embodiment 16 of the present invention includes a pair of upper jaw oral appliances separated into the right and the left and a lower jaw oral appliance as illustrated in FIG. 30, which is a schematic perspective view thereof, and FIG. 31, which is a side view thereof, and upper jaw oral appliances 6310 can be fitted to the upper jaw of a user and lower jaw oral appliance 6320 can be fitted to the lower jaw of the user.

As illustrated in FIG. 30, upper jaw oral appliances 6310 include a pair of right and left upper jaw oral appliance body portions 6314 that each can be fitted to a part of the row of teeth of the upper jaw of the user, each of upper jaw oral appliance body portions 6314 including teeth row shape portion 6312 formed therein, teeth row shape portion 6312 being able to be fitted to the relevant part of the row of teeth of the upper jaw of the user.

As illustrated in FIG. 30, lower jaw oral appliance 6320 includes lower jaw oral appliance body portion 6324 that can be fitted to the entire row of teeth of the lower jaw of the user, lower jaw oral appliance 6320 including teeth row shape portion 6322 formed therein, teeth row shape portion 6322 being able to be fitted to the row of teeth of the lower jaw of the user.

Lower jaw oral appliance body portion 6324 is joined to the respective upper jaw oral appliance body portions 6314 via a pair of right and left joining portions 6340 provided on a side surface thereof.

Each of joining portions 6340, which is fixed to a side surface of a relevant one of upper jaw oral appliance body portions 6314, is positioned on the side surface of lower jaw oral appliance body portion 6324 and thereby fixes respective positions of relevant one upper jaw oral appliance 6310 and lower jaw oral appliance body portion 6324 relative to each other. Consequently, respective positions in a mouth opening direction, respective positions in a front-rear direction, and respective positions in a right-left direction of lower jaw oral appliance 6320 and each upper jaw oral appliance 6310 relative to each other are fixed.

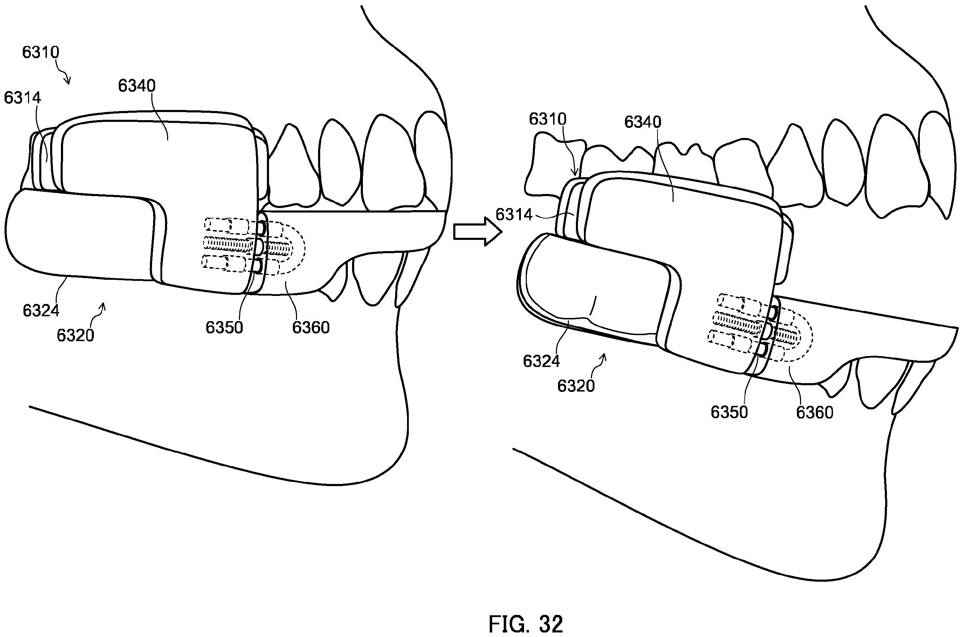

Each joining portion 6340 fixes the respective positions in the mouth opening direction of lower jaw oral appliance 6320 and relevant upper jaw oral appliance 6310 relative to each other. Therefore, when the user wearing oral appliance 6300 opens the mouth, lower jaw oral appliance 6320 is moved in the mouth opening direction along with movement of the lower jaw of the user and upper jaw oral appliances 6310 fixed to lower jaw oral appliance 6320 are moved in the mouth opening direction at the same time. In this case, as illustrated in FIG. 32, which is a side view illustrating a manner in which the user wearing oral appliance 6300 opens the mouth, if the user moves the lower jaw in the mouth opening direction, upper jaw oral appliances 6310 are removed from the row of teeth of the upper jaw, and if the user moves the lower jaw in a biting direction, upper jaw oral appliances 6310 are fitted to the row of teeth of the upper jaw. Consequently, limitation of opening of the mouth by flange portions of upper and lower jaw oral appliances of oral appliances described in, for example, PTL 1 and PTL 2 is less likely to occur, making it easy for the user to open the mouth.

In this case, since upper jaw oral appliances 6310 are separated into the right and the left, an area of contact between each upper jaw oral appliance 6310 and the row of teeth of the upper jaw of the user can further be decreased. Consequently, each upper jaw oral appliance 6310 can easily be removed from the row of teeth, making it easier for the user to open the mouth.

Also, each joining portion 6340 fixes the respective positions in the front-rear direction of lower jaw oral appliance 6320 and relevant upper jaw oral appliance 6310 relative to each other. Consequently, joining portions 6340 can prevent narrowing of the airway of the user wearing oral appliance 6300 due to rearward displacement of the lower jaw of the user and thus suppress pauses in breathing or infrequent breathing during sleep.

Also, each joining portion 6340 fixes the respective positions in the right-left direction of lower jaw oral appliance 6320 and relevant upper jaw oral appliance 6310 relative to each other. Consequently, joining portions 6340 can limit displacement in the right-left direction of lower jaw oral appliance 6320 due to, for example, teeth grinding of the user wearing oral appliance 6300 and thus suppress damage of guide sections due to displacement in the right-left direction.

A position of each joining portion 6340 is adjustable in the front-rear direction along relevant guide section 6350 provided on an outer side surface of lower jaw oral appliance body portion 6324. The position of joining portion 6340 is adjusted rearward on the side surface of lower jaw oral appliance 6320 along guide section 6350. Consequently, each upper jaw oral appliance 6310 fixed to relevant joining portion 6340 is adjusted rearward relative to lower jaw oral appliance 6320 along relevant guide section 6350. In other words, a position of lower jaw oral appliance 6320 is adjusted forward relative to respective upper jaw oral appliances 6310 along respective guide sections 6350.

Consequently, upon biting, each joining portion 6340 fixes the respective positions in the aforementioned directions of relevant upper jaw oral appliance 6310 and lower jaw oral appliance body portion 6324 relative to each other while moving the position in the front-rear direction of lower jaw oral appliance 6320 relative to upper jaw oral appliances 6310 forward. Therefore, oral appliance 6300 allows the lower jaw of the user wearing oral appliance 6300 to be moved forward and thus allows the airway of the user to open more largely, enabling more effective suppression of pauses in breathing or infrequent breathing during sleep.

Each guide section 6350 includes male-threaded screw rod 6352 including an end fixed to fixing portion 6360 provided on a side surface of lower jaw oral appliance body portion 6324, male-threaded screw rod 6354 including an end fixed to relevant joining portion 6340, and turnbuckle portion 6356 including a screw hole to which respective other end portions of screw rods 6352 and 3654 are threadably connected. Guide section 6350 can adjust the position of joining portion 6340 fixed to and supported by screw rod 6354, by inserting a rotation pin into a pin hole provided in turnbuckle portion 6356 and rotating turnbuckle portion 6356 to adjust amounts of the thread connection between turnbuckle portion 6356 and screw rods 6352 and 6354.

Each joining portion 6340 may be detachably attachable to relevant upper jaw oral appliance body portion 6314 and lower jaw oral appliance body portion 6324 but may be bonded to relevant upper jaw oral appliance body portion 6314 and lower jaw oral appliance body portion 6324. If each joining portion 6340 is bonded to relevant upper jaw oral appliance body portion 6314 and lower jaw oral appliance body portion 6324, misalignment of upper jaw oral appliance 6310 and lower jaw oral appliance 6320 is less likely to occur during wearing.

In FIGS. 30 and 31, each upper jaw oral appliance 6310 can be fitted to a row of teeth on the rear side from relevant joining portion 6340, in the row of teeth of the upper jaw of the user, but may be fitted to a row of teeth, on the front side from relevant joining portion 6340. However, the skin of the inner cheeks of a human being more easily stretches and shrinks further on the rear side. Therefore, each upper jaw oral appliance 6310 preferably can be fitted to a row of teeth, on the rear side from relevant joining portion 6340, in the row of teeth and preferably can be fitted to a row of teeth including molar teeth (specifically, relevant first molar tooth (tooth number: 6) and the relevant second molar tooth (tooth number: 7)) of the user.

According to the present embodiment, when the user wearing oral appliance 6300 opens the mouth, upper jaw oral appliances 6310 are removed from the row of teeth of the upper jaw of the user and are moved together with lower jaw oral appliance 6320. Also, upper jaw oral appliances 6310 are separated into the right and the left, enabling a decrease of the area of contact between each upper jaw oral appliance 6310 and the row of teeth of the upper jaw of the user. Consequently, the user wearing oral appliance 6300 can more easily open the mouth.

Also, according to the present embodiment, each upper jaw oral appliance 6310 can be fitted to a part of the row of teeth of the upper jaw. Therefore, when the user wearing oral appliance 6300 bites after opening the mouth, each upper jaw oral appliance 6310 is fitted to predetermined teeth in the row of teeth of the upper jaw, making it easy for the user to bite with the lower jaw moved forward.

Also, according to the present embodiment, since an increase in thickness in the right-left direction of the oral appliance can be suppressed, a feeling of fitting the user has is further improved.

### [Embodiment 17]

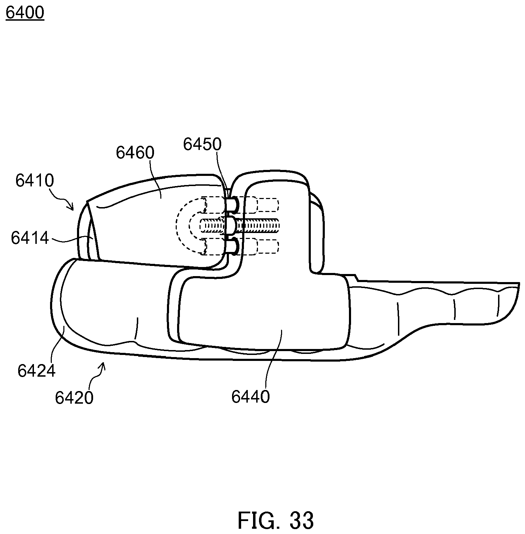

Oral appliance 6400 according to Embodiment 17 of the present invention includes a pair of upper jaw oral appliances separated into the right and the left and a lower jaw oral appliance as illustrated in FIG. 33, which is a schematic side view thereof, and upper jaw oral appliances 6410 can be fitted to the upper jaw of a user and lower jaw oral appliance 6420 can be fitted to the lower jaw of the user. As in Embodiment 16, upper jaw oral appliances 6410 and lower jaw oral appliance 6420 are joined via a pair of right and left joining portions 6440. In the below, description of components that are in common with Embodiment 16 may be omitted.

In the present embodiment, each of joining portions 6440 is positioned on a side surface of a relevant one of upper jaw oral appliance body portion 6414 and fixed to a side surface of lower jaw oral appliance body portion 6424 and thereby fixes respective positions of relevant one upper jaw oral appliance 6410 and lower jaw oral appliance body portion 6424 relative to each other. Consequently, respective positions in a mouth opening direction, respective positions in a front-rear direction, and respective positions in a right-left direction of lower jaw oral appliance 6420 and each upper jaw oral appliance 6410 relative to each other are fixed.

Respective positions of joining portions 6440 are adjustable in the front-rear direction along a pair of guide sections 6450 provided on an outer side surface of upper jaw oral appliance body portion 6414 relative to fixing portion 6460 fixing guide sections 6450. A position of each joining portion 6440 is adjusted forward on the side surface of upper jaw oral appliance 6410 along relevant guide section 6450. In other words, a position of each upper jaw oral appliance 6410 is adjusted rearward along relevant guide section 6450 relative to lower jaw oral appliance 6420. Also, a position of lower jaw oral appliance 6420 is adjusted forward relative to each upper jaw oral appliance 6410 along relevant guide section 6450.

Consequently, upon biting, each joining portion 6440 fixes the respective positions in the aforementioned directions of relevant upper jaw oral appliance 6410 and lower jaw oral appliance body portion 6424 relative to each other while moving the position in the front-rear direction of lower jaw oral appliance 6420 relative to upper jaw oral appliance 6410 forward. Therefore, oral appliance 6400 allows the lower jaw of the user wearing oral appliance 6400 to be moved forward and thus allows the airway of the user to open more largely, enabling more effective suppression of pauses in breathing or infrequent breathing during sleep.

According to the present embodiment, in position adjustment by guide sections 6450, it is not necessary to move joining portions 6440 relative to lower jaw oral appliance 6420 and it is only necessary to move upper jaw oral appliances 6410, which are smaller members, which makes the position adjustment easier.

Also, according to the present embodiment, since an increase in thickness in the right-left direction of the oral appliance can be suppressed, a feeling of fitting the user has is further improved.

### [Embodiment 18]

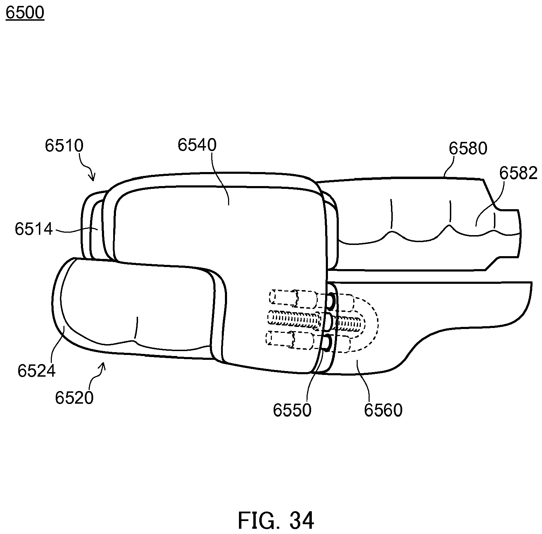

Oral appliance 6500 according to Embodiment 18 of the present invention includes a pair of upper jaw oral appliances separated into the right and the left and a lower jaw oral appliance as illustrated in FIG. 34, which is a schematic side view thereof, and upper jaw oral appliances 6510 can be fitted to the upper jaw of a user and lower jaw oral appliance 6520 can be fitted to the lower jaw of the user. As in Embodiment 16, upper jaw oral appliances 6510 and lower jaw oral appliance 6520 are joined via joining portions 6540. In the below, description of components that are in common with Embodiment 16 may be omitted.

In the present embodiment, oral appliance 6500 includes spacer 6580 that can be fitted to a row of teeth of the upper jaw of the user.

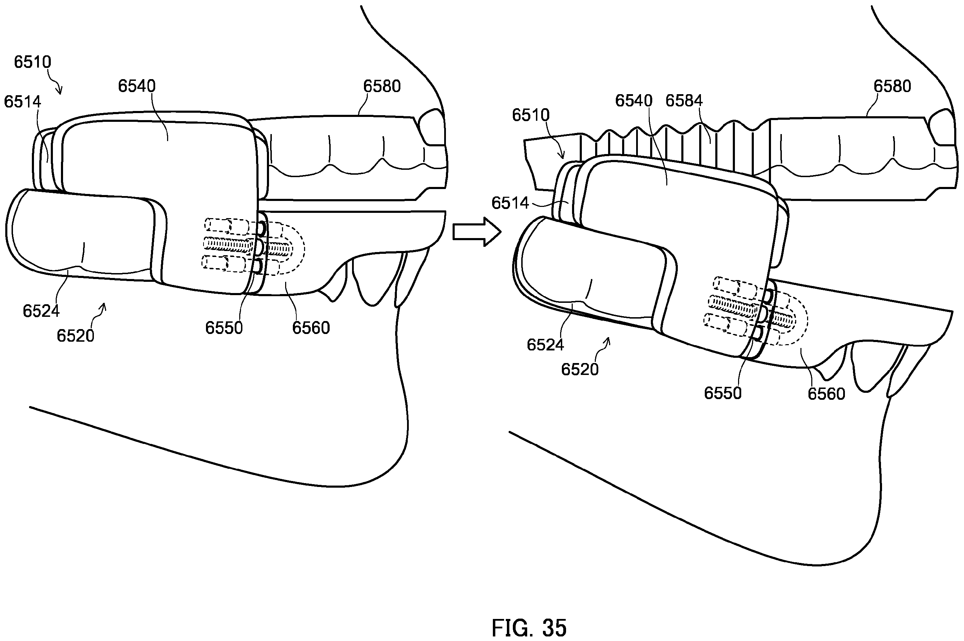

As illustrated in FIG. 35, which is a side view illustrating a manner in which the user wearing oral appliance 6500 opens the mouth, if the user moves the lower jaw in the mouth opening direction, upper jaw oral appliances 6510 are removed from the row of teeth of the upper jaw and if the user moves the lower jaw in a biting direction, upper jaw oral appliances 6510 are fitted to the row of teeth of the upper jaw. Here, spacer 6580 is fitted to the row of teeth of the upper jaw of the user throughout the opening of the mouth and the biting, upper jaw oral appliances 6510 are removed from spacer 6580 upon opening of the mouth and are fitted onto spacer 6580 upon biting.

Spacer 6580 includes teeth row shape portion 6582 formed in an inner side surface thereof, teeth row shape portion 6582 being able to be fitted to a row of teeth of the upper jaw of the user, and has a shape that can be fitted to the entire row of teeth of the upper jaw. Also, spacer 6580 includes fitting portions 6584 that can be fitted in inner side surfaces of respective upper jaw oral appliances 6510, fitting portions 6584 being formed in right and left rear regions of an outer side surface thereof, and has a shape that enables right and left upper jaw oral appliances 6510 to be fitted onto spacer 6580 upon biting during wearing oral appliance 6500.

Each fitting portion 6584 has a shape that when relevant upper jaw oral appliance 6510 is fitted into spacer 6580 upon biting, limits displacement in the front-rear direction of upper jaw oral appliance 6510 but does not limit movement in the mouth opening direction of the fitted upper jaw oral appliance 6510. For example, each fitting portion 6584 has a shape, for example, that is substantially the same as the outer side surface of a relevant row of teeth of the upper jaw or a pleated shape in which a plurality of ridge portions and groove portions are formed in the mouth opening direction.

In this case, the inner side surface of each upper jaw oral appliance 6510 in which fitting portion 6584 is fitted has a shape that is a counterpart of fitting portion 6584. The shape of each fitting portion 6584 and the shape of the inner side surface of relevant upper jaw oral appliance 6510 are preferably shapes that enable upper jaw oral appliance 6510 to be fitted into only particular positions of spacer 6580. For example, each fitting portion 6584 may have a shape that is substantially the same as the outer side surface of the relevant row of teeth of the upper jaw and the inner side surface of relevant upper jaw oral appliance 6510 may have a shape that is an inversion of the shape of fitting portion 6584. Alternatively, each fitting portion 6584 and the inner side surface of relevant upper jaw oral appliance 6510 may only have respective pleated shapes that are inversions of each other and respective widths in the front-rear direction of ridge portions and groove portions included in each pleated shape may be different between a front part and a rear part of the pleated shape.

According to the present embodiment, a feeling of discomfort a user has because of wearing an oral appliance can be reduced by forming spacer 6580 that abuts against the row of teeth of the upper jaw of the user from a material that gives an improved feeling of fitting. Also, according to the present embodiment, spacer 6580 limits movement in the front-rear direction of upper jaw oral appliances 6510, enabling enhancement of a feeling of close contact of upper jaw oral appliances 6510.

Also, according to the present embodiment, spacer 6580 limits misalignment in the front-rear direction of upper jaw oral appliances and thus suppresses positional deviation of the upper jaw oral appliances from the row of teeth of the upper jaw, enabling improvement of a feeling of fitting the user has.

In addition, according to the present embodiment, spacer 6580 enables displacement in the mouth opening direction of upper jaw oral appliances 6510 during wearing, making it easy for the user wearing oral appliance 6500 to open the mouth.

Furthermore, according to the present embodiment, when the user wearing oral appliance 6500 bites after opening the mouth, respective upper jaw oral appliances 6510 are fitted to only the particular positions in respective fitting portions 6584 of spacer 6580, making it easy for the user to bite with the lower jaw moved forward.

In this case, no shapes that are similar to fitting portions 6584 are provided in parts of an outer surface of spacer 6580, the parts not abutting against respective upper jaw oral appliances 6510, making it easier for the user to recognize the position of the lower jaw upon biting.

### [Alternations of Embodiments 16 to 18]

Each of Embodiments 16 to 18 described above indicates an example of the sixth aspect of the present invention, and it is a matter of course that: the sixth aspect of the present invention is not limited to the above embodiments; and various other embodiments are possible within the scope of the idea of the sixth aspect of the present invention.

For example, in each of Embodiments 16 to 18, the upper jaw oral appliances are separated into the right and the left and are fitted to respective parts of the row of teeth of the upper jaw and the lower jaw oral appliance is fitted to the entire row of teeth of the lower jaw. However, lower jaw oral appliances may be separated into the right and the left and be fitted to respective parts of the row of teeth of the lower jaw and an upper jaw oral appliance may be fitted to the entire row of teeth of the upper jaw. Also, upper jaw oral appliances may be separated into the right and the left and be fitted to respective parts of the row of teeth of the upper jaw and lower jaw oral appliances may also be separated into the right and the left and be fitted to respective parts of the row of teeth of the lower jaw.

However, where upper jaw oral appliances are separated into the right and the left and fitted to respective parts of the row of teeth of the upper jaw, upper jaw oral appliances that are removed from the row of teeth upon opening of the mouth are fitted to only the respective parts of the row of teeth, making it easy to remove the upper jaw oral appliances upon opening of the mouth and thus making it easy for the user to open the mouth.

Also, in each of Embodiments 16 to 18, the joining portions, the guide sections, and the fixing portion are provided on the outer side surface of the lower jaw oral appliance or the upper jaw oral appliances, but the joining portions, the guide sections, and the fixing portion may be provided on an inner side surface of a lower jaw oral appliance or upper jaw oral appliances.

Although the joining portions, the guide sections, and the fixing portion may be either the outer side surface or the inner side surface of the lower jaw oral appliance or the upper jaw oral appliances, the joining portions, the guide sections, and the fixing portion are preferably all provided on a same side surface that is the outer side surface or the inner side surface of the lower jaw oral appliance or the upper jaw oral appliances.

Also, in each of Embodiments 16 to 18, the upper jaw oral appliance body portions and the lower jaw oral appliance body portion each include the teeth row shape portion that can be fitted to the row of teeth. However, the upper jaw oral appliance body portions and the lower jaw oral appliance body portion may only have a shape that receives the row of teeth and thus may be a shape that covers the row of teeth and each of the upper jaw oral appliance body portions and the lower jaw oral appliance body portion may only partly include the teeth row shape portion.

Also, the upper and lower jaw oral appliances may be each formed of a plurality of materials or may be formed by a combination of a part formed of an organic substance and a part formed of an inorganic substance.

Also, in each of Embodiments 16 to 18, each of the guide sections adjusts a position of the relevant positioning member by means of adjustment of amounts of thread connection between a turnbuckle portion and two screw rods using a turnbuckle mechanism, but for each of the guide sections, a jackscrew mechanism that can adjust a position of a positioning member by adjusting an amount of thread connection between a male screw and a female screw may be used. Also, each guide section may move the relevant positioning member via a gear using a rack jack mechanism that can adjust a position of the positioning member fixed to a rack by adjusting an amount of rotation of the rack, and fix the positioning member via a screw. Each guide section may adjust a position of a positioning member using another mechanism as long as such other mechanism can move the position of the positioning member and fix the positioning member at the adjusted position.

Also, in each of Embodiments 16 to 18, guide sections are provided in either of the upper and lower jaw oral appliances. However, guide sections may be provided in each of the upper and lower jaw oral appliances to enable respective positions of joining portions to be adjusted at two sites.

Here, where the guide sections are only provided in the lower jaw oral appliance, durability of the oral appliance tends to be higher.

### [Example Configurations Relating to Sixth Aspect]

The sixth aspect of the present invention provides
an oral appliance including:
one oral appliance that can be fitted to a row of teeth of one of an upper jaw and a lower jaw;
a pair of other oral appliances that can be fitted to respective parts of a row of teeth of another one of the upper jaw and the lower jaw, the pair of other oral appliances being separated into a right and a left;
joining portions that each join the one oral appliance and the relevant other oral appliance to each other and fixes respective positions in a mouth opening direction of the one oral appliance and the relevant other oral appliance relative to each other; and
guide sections that each adjust a relative position in a front-rear direction of the relevant other oral appliance relative to the one oral appliance.

In the oral appliance, the other oral appliances are fitted to only the respective parts of the row of teeth and thus a feeling of discomfort due to wearing the oral appliance is reduced, providing improvement in feeling of fitting the user has.

The sixth aspect of the present invention further provides
the oral appliance including a spacer that can be fitted to the row of teeth of the other one of the upper jaw and the lower jaw,
wherein the spacer has a shape that upon biting, allows the spacer to abut against the other oral appliances and thereby limit displacement in a front-rear direction of the other oral appliances and, upon opening of the mouth, enables displacement of a position of the spacer relative to the other oral appliances.

A feeling of discomfort a user has because of wearing the oral appliance can be reduced by forming the spacer that abuts against the row of teeth of the upper jaw of the user from a material that gives an improved feeling of fitting. Also, in the oral appliance, the spacer limits movement in the front-rear direction of the other oral appliances, enabling enhancement of a feeling of close contact of the other oral appliances. Also, in the oral appliance, the spacer limits misalignment in the front-rear direction of the other oral appliances and thus suppresses positional deviation of the other oral appliances during wearing, enabling improvement of a feeling of fitting the user has.

The sixth aspect of the present invention further provides
the oral appliance in which each of the guide sections is provided at a position at which the guide section adjusts a position in the front-rear direction of the relevant joining portion to adjust the relative position in the front-rear direction of the relevant other oral appliance fixed to the relevant joining portion.

The oral appliance allows the lower jaw of the user wearing the oral appliance to be moved forward and thus allows the airway of the user to open largely, enabling more effective suppression of pauses in breathing or infrequent breathing during sleep.

The sixth aspect of the present invention further provides
the oral appliance in which the other oral appliances each have a shape that enables the other oral appliance to be fitted to a molar tooth of the row of teeth of the other jaw.

In the oral appliance, the other oral appliances are disposed at respective positions corresponding to the rear parts, in which the skin easily stretches and shrinks, of the inner cheeks of the user wearing the oral appliance, providing improvement of a feeling of fitting the user wearing oral appliance has.

The sixth aspect of the present invention further provides
the oral appliance in which the guide sections are provided on respective side surfaces of the other oral appliances.

The oral appliance allows durability of the oral appliance to tend to be higher.

The sixth aspect of the present invention further provides
the oral appliance in which the one oral appliance has a shape that enables the one oral appliance to be fitted to the row of teeth of the lower jaw.

In the oral appliance, the upper and lower jaw oral appliances are moved in the mouth opening direction together upon opening of the mouth and the upper jaw oral appliances removed from the row of teeth at that time are fitted to only the respective parts of the row of teeth, making it easy to remove the upper jaw oral appliances and thus making it easy for the user to open the mouth.

### 7. Materials

Each of the upper jaw oral appliance body portion(s) and the lower jaw oral appliance body portion described in each of the above embodiments is formed of a material that is less likely to be damaged by a human being's normal biting force.

For example, each of the upper jaw oral appliance body portion(s) and the lower jaw oral appliance body portion may be formed of a single hard material having a flexural modulus of 2000 MPa to 3000 MPa (for example, an acrylic resin), the flexural modulus being measured according to JIS T 6501, or a material that is a combination of a soft material of 10 MPa to 300 MPa and a hard material of 1000 MPa to 3000 MPa.

Also, for enhancement of capability of following the teeth of a user, each of the upper jaw oral appliance body portion(s) and the lower jaw oral appliance body portion may be formed of a relatively soft material having a tensile strength of 150 N to less than 2000 N, preferably 150 N to 500 N.

Here, the tensile strength means a strength with which when an oral appliance (thickness: 3 mm) fabricated using a Nissin standard study model, the oral appliance including a hole of ϕ1.5 mm created in a tooth of tooth number 6 in an upper jaw oral appliance thereof, was subjected to a tensile test in a direction toward the molar teeth (rear direction), the upper jaw oral appliance was ruptured.

Examples of the material having a tensile strength of 150 N to less than 2000 N include an olefin-based resin, a polyester-based resin, an urethane-based resin, a polyamide-based resin, and an acrylic-based rubber resin. From among these resins, an olefin-based resin is preferable.

An olefin-based resin is a polymer formed by homopolymerization of an olefin or a copolymer formed by polymerization of an olefin and another monomer. For the olefin, olefins with 2 to 6 carbons, including ethylene, propylene, butene, methylpentene, and hexene, are preferable. Examples of the other monomer include vinyl acetate.

For the olefin-based resin, for example, polyethylene (PE), a polyethylene-based resin, polypropylene (PP), a polypropylene-based resin, and an ethylene-vinyl acetate copolymer (EVA) are preferable, and, for example, polyethylene (PE), a polyethylene-based resin, polypropylene (PP), and a polypropylene-based resin are more preferable.

A polyester-based resin is a polycondensate of a polycarboxylic acid (for example, a dicarboxylic acid) and a polyalcohol (for example, diol). Examples of the polyester-based resin include polyethylene terephthalate (PET).

An urethane-based resin is a polycondensate of a compound including an isocyanate group and a compound including a hydroxyl group. Examples of the urethane-based resin include thermoplastic polyurethane (TPU).

A polyamide-based resin is a (co-)polymer formed by amide binding of many monomers. Examples of the polyamide-based resin include, nylon, para-amide, and meta-amide.

An acrylic-based rubber resin is a (co-)polymer containing acrylic-based rubber as a main component. Examples of the acrylic-based resin include a block copolymer of methyl methacrylate and butyl acrylate.

For a material having a tensile strength of 150 N to less than 2000 N, a commercially available one, for example, F327 (polypropylene-based resin) manufactured by Prime Polymer, Co., Ltd., may be used.

Each of the positioning members, the fixing portion, the front fixing portion, the rear fixing portions, the protrusion portions, the stoppers, the stopper fixing portions, the moving portions, and the joining portions is formed of, for example, a hard material having a flexural modulus of 2000 MPa to 3000 MPa (for example, an acrylic resin), the flexural modulus being measured according to JIS T 6501. Consequently, damage of these members by stress caused when the positioning members, the fixing portion, and the stoppers abut against the upper jaw oral appliance body portion or the lower jaw oral appliance body portion upon displacement in the right-left direction can be suppressed.

Spacer 6580 of a sixth oral appliance is formed of a material that is less likely to be damaged by a human being's normal biting force. Spacer 6580 may be formed of a resin material, for example, a polyester resin or an acrylic resin. Also, from the perspective of enhancement of capability of following the shape of the row of teeth, a thickness in an inside-outside direction of spacer 6580 is preferably 0.5 mm to 5.0 mm.

Also, from the perspective of facilitating fitting and removal of spacer 6580 of the sixth oral appliance, upper jaw oral appliances 6510 are preferably formed of a material having a Shore A hardness of no less than 90, the Shore A hardness being measured according to JIS K 7215.

The present application is filed claiming the priority from Japanese Patent Application No. 2016-253255 filed on December 27, 2016, Japanese Patent Application No. 2016-253263 filed on December 27, 2016, Japanese Patent Application No. 2016-253265 filed on December 27, 2016, Japanese Patent Application No. 2017-021254 filed on February 8, 2017, Japanese Patent Application No. 2017-037311 filed on February 28, 2017, and Japanese Patent Application No. 2017-108996 filed on June 1, 2017.

### Industrial Applicability

Each of the oral appliances relating to the first to fifth aspects of the present invention enables limiting rearward displacement of the lower jaw of a user wearing the oral appliance while adjusting a position of the lower jaw of the user. Also, each of the oral appliances relating to the first to fifth aspects of the present invention the oral appliances are highly durable in comparison with conventional oral appliances. Furthermore, an oral appliance relating to the sixth aspect of the present invention can reduce a user's feeling of discomfort. The oral appliances according to the present invention are usable for, for example, prevention and treatment of temporomandibular joint disorder and suppression of teeth grinding in addition to prevention and treatment of sleep apnea syndrome.

### Reference Signs List

1100, 1200, 1300 oral appliance
1110, 1210, 1310 upper jaw oral appliance
1120, 1220, 1320 lower jaw oral appliance
1112 teeth row shape portion
1114, 1214, 1314 upper jaw oral appliance body portion
1122 teeth row shape portion
1124, 1224, 1324 lower jaw oral appliance body portion
1128 abutment portion abutting against stopper
1130, 1230, 1330 stopper
1132 front surface of stopper
1134, 1234 extension portion
1136, 1236 inclined portion
1137 lower end of front end portion
1138 abutment surface abutting against lower jaw oral appliance
1140, 1240, 1340 positioning member
1142, 1242 rear surface of positioning member
1144, 1344 wing
1146 inclined portion
1147 lower end of rear end portion
1150, 1250, 1350 position adjustment section
1152, 1154 screw rod
1156 turnbuckle portion
1160, 1260, 1360 fixing portion
1362 protrusion portion
2100, 2200 oral appliance
2110, 2210 upper jaw oral appliance
2120, 2220 lower jaw oral appliance
2112 teeth row shape portion
2114, 2214 upper jaw oral appliance body portion
2122 teeth row shape portion
2124, 2224 lower jaw oral appliance body portion
2130, 2230 stopper
2130a front surface of stopper
2140, 2240 positioning member
2140a rear surface of positioning member
2144, 2244 wing
2150 position adjustment section
2152, 2154 screw rod
2156 turnbuckle portion
2160, 2260 guide section
2162, 2262 front fixing portion
2164, 2264 rear fixing portion
2166, 2168, 2266, 2268 support bar
2228 abutment portion abutting against stopper
2237 lower end of rear end portion
2247 lower end of front end portion
2248 abutment surface abutting against lower jaw oral appliance
3100, 3200 oral appliance
3110, 3210 upper jaw oral appliance
3120, 3220 lower jaw oral appliance
3112 teeth row shape portion
3114, 3214 upper jaw oral appliance body portion
3122 teeth row shape portion
3124, 3224 lower jaw oral appliance body portion
3130, 3230 stopper
3132 front surface of stopper
3136, 3236 inclined portion
3140, 3240 positioning member
3142 rear surface of positioning member
3146, 3246 inclined portion
3150, 3250 guide section
3152, 3154 screw rod
3156 turnbuckle portion
3160, 3260 fixing portion
3170, 3270 protrusion portion
3228 abutment portion abutting against protrusion portion
3229 abutment portion abutting against positioning member
3248 abutment surface abutting against lower jaw oral appliance
3278 abutment surface abutting against lower jaw oral appliance
4100, 4200, 4300, 4400 oral appliance
4110, 4210, 4310, 4410 upper jaw oral appliance
4120, 4220, 4320, 4420 lower jaw oral appliance
4112 teeth row shape portion
4114, 4214, 4314, 4414 upper jaw oral appliance body portion
4122 teeth row shape portion
4124, 4224, 4324, 4424 lower jaw oral appliance body portion
4130, 4230, 4330, 4430 stopper
4130a front surface of stopper
4131, 4231, 4331, 4431 moving portion
4132, 4232, 4332, 4432 stopper adjustment section
4132a, 4132b screw rod
4132c turnbuckle portion
4133, 4233, 4333, 4433 stopper fixing portion
4140, 4240, 4340, 4440 positioning member
4140a rear surface of positioning member
4144, 4244, 4444 wing
4150, 4250, 4350, 4450 position adjustment section
4152, 4154 screw rod
4156 turnbuckle portion
4160, 4260, 4360, 4460 fixing portion
4228 abutment portion abutting against stopper
4234, 4334 extension portion
4236, 4336 inclined portion
4238 abutment surface abutting against lower jaw oral appliance
4246 inclined portion
4462 protrusion portion
5100, 5200, 5300, 5400 oral appliance
5110, 5210, 5310, 5410 upper jaw oral appliance
5120, 5220, 5320, 5420 lower jaw oral appliance
5112 teeth row shape portion
5114, 5214, 5314, 5414 upper jaw oral appliance body portion
5122 teeth row shape portion
5124, 5224, 5324, 5424 lower jaw oral appliance body portion
5128 abutment portion abutting against stopper
5130, 5230, 5330, 5430 stopper
5132 rear surface of stopper
5136, 5236 inclined portion
5140, 5240, 5340, 5440 positioning member
5142 front surface of positioning member
5144, 5244, 5344, 5444 wing
5146, 5246 inclined portion
5148 abutment surface abutting against lower jaw oral appliance
5150, 5250, 5350, 5450 position adjustment section
5152, 5154, 5252, 5254 screw rod
5156, 5256 turnbuckle portion
5160, 5260, 5360, 5460 fixing portion
5362 protrusion portion
5433 moving portion
5434 stopper adjustment section
5434a, 5434b screw rod
5434c turnbuckle portion
5435 stopper fixing portion
6300, 6400, 6500 oral appliance
6310, 6410, 6510 upper jaw oral appliance
6320, 6420, 6520 lower jaw oral appliance
6312 teeth row shape portion
6314, 6414 upper jaw oral appliance body portion
6322 teeth row shape portion
6324, 6424 lower jaw oral appliance body portion
6350, 6450 guide section
6352, 6354 screw rod
6356 turnbuckle portion
6360, 6460 fixing portion
6340, 6440, 6540 joining portion
6580 spacer
6582 teeth row shape portion
6584 fitting portion

## Claims

1. An oral appliance (1000, 4000), comprising:
a pair of upper and lower jaw oral appliances (1110, 1120, 1210, 1220, 4210, 4220, 4310, 4320) facing each other;
a pair of positioning members (1140, 1240, 4240, 4340) each being adjustable in position in a front-rear direction, the pair of positioning members (1140, 1240, 4240, 4340) being provided in one oral appliance (1120, 1220, 4220, 4320) of the upper and lower jaw oral appliances (1110, 1120, 1210, 1220, 4210, 4220, 4310, 4320); and
a pair of stoppers (1130, 1230, 4230, 4330) provided in another oral appliance (1110, 1210, 4210, 4310) of the upper and lower jaw oral appliances (1110, 1120, 1210, 1220, 4210, 4220, 4310, 4320), the pair of stoppers (1130, 1230, 4230, 4330) abutting against the respective positioning members (1140, 1240, 4240, 4340) and thereby restricting rearward displacement of the lower jaw oral appliance (1120, 1220, 4220, 4320), and each of the stoppers (1130, 1230, 4230, 4330) including an extension portion (1134, 1234, 4234, 4334) that abuts against the oral appliance (1120, 1220, 4220, 4320) thereby restricting displacement in a right-left direction of the upper jaw oral appliance (1110, 1210, 4210, 4310) and lower jaw oral appliance (1120, 1220, 4220, 4320), the extension portion (1134, 1234, 4234, 4334) having a height that is equal to or less than half a height of the stoppers (1130, 1230, 4230, 4330), and
**characterized in that** each of the stoppers (1130, 1230, 4230, 4330) include an inclined surface (1136, 1236, 4236, 4336) which abuts against relevant positioning members (1140, 1240, 4240, 4340), and the inclined surface (1136, 1236, 4236, 4336) is further inclined in the extension portion (1134, 1234, 4234, 4334).

2. The oral appliance (1000, 4000) according to claim 1, wherein each of the positioning members (1140, 4240) includes a wing (1144, 4244) that abuts against the corresponding stopper (1130, 4230) on an outer side of the other oral appliance (1110, 4210).

3. The oral appliance (1000, 4000) according to claim 2, wherein each of the wings (1144, 4244) is disposed at a position at which when the corresponding stopper abuts against the one oral appliance as a result of movement in the right-left direction, the wing (1144, 4244) does not abut against the other oral appliance (1110, 4210).

4. The oral appliance (1000, 4000) according to any one of claims 1 to 3, wherein each of the stoppers (1130, 1230, 4230, 4330) has a shape that is obtuse in a lower end of an end portion on the one oral appliance (1120, 1220, 4220, 4320) side of the front end portion of the stopper (1130, 1230, 4230, 4330).

5. The oral appliance (1000, 4000) according to any one of claims 1 to 4, wherein each of the stoppers (1130, 1230, 4230, 4330) has a shape that makes a projection area of projection of the stopper (1130, 1230, 4230, 4330) to the one oral appliance (1120, 1220, 4220, 4320) from a lateral side direction upon biting be 3 mm² or more.

6. The oral appliance (1000, 4000) according to any one of claims 1 to 5, wherein:
abutment surfaces of the pair of stoppers (1130, 1230, 4230, 4330) against the one oral appliance (1120, 1220, 4220, 4320) are substantially parallel to each other; and
abutment surfaces of the one oral appliance (1120, 1220, 4220, 4320) against the pair of stoppers (1130, 1230, 4230, 4330) are substantially parallel to each other.

7. The oral appliance (1000, 4000) according to any one of claims 1 to 6, further comprising guide sections (2160, 2260, 3150, 3250, 6350, 6450) each including opposite ends supported by a pair of fixing portions (2162, 2262, 2164, 2264, 3160, 3260, 6360, 6460) provided in one oral appliance (1120, 1220, 4220, 4320) of the upper and lower jaw oral appliances (1110, 1120, 1210, 1220, 4210, 4220, 4310, 4320);
wherein the pair of positioning members are adjustable in position in a front-rear direction along respective guide sections (2160, 2260, 3150, 3250, 6350, 6450).

8. The oral appliance (1000, 4000) according to claim 7, wherein the stoppers (1130, 1230, 4230, 4330) have a height that is equal to or lower than a height of the other oral appliance (1110, 4210).

9. The oral appliance according to any one of claims 1 to 8, further comprising protrusion portions (3170, 3270) fixed to another oral appliance (3110, 3210) of the upper and lower jaw oral appliances (3110, 3120, 3210, 3220), the protrusion portions (3170, 3270) protruding from the other oral appliance (3110, 3210) toward the one oral appliance (3120, 3220);
wherein the pair of positioning members (3140, 3240) are adjusted in position in a front-rear direction along a guide section (3150, 3250) including an end supported by the corresponding protrusion portion (3170, 3270), at a position at which the positioning member (3140, 3240) is located lateral to the one oral appliance (3120, 3220) upon biting, and each of the positioning members(3140, 3240) abuts against the corresponding stopper (3130, 3230) and thereby restricting rearward displacement of the lower jaw oral appliance (3120, 3220).

## Patentansprüche

1. Mundgerät (1000, 4000), umfassend:
ein Paar von Ober- und Unterkiefer-Mundgeräten (1110, 1120, 1210, 1220, 4210, 4220, 4310, 4320), die einander gegenüberliegen;
ein Paar von Positionierungselementen (1140, 1240, 4240, 4340), die jeweils in einer Vorwärts-Rückwärts-Richtung positionsverstellbar sind, wobei das Paar von Positionierungselementen (1140, 1240, 4240, 4340) in einem Mundgerät (1120, 1220, 4220, 4320) der Ober- und Unterkiefer-Mundgeräte (1110, 1120, 1210, 1220, 4210, 4220, 4310, 4320) vorgesehen ist; und
ein Paar von Stoppern (1130, 1230, 4230, 4330), die in einem anderen Mundgerät (1110, 1210, 4210, 4310) der Ober- und Unterkiefer-Mundgeräte (1110, 1120, 1210, 1220, 4210, 4220, 4310, 4320) vorgesehen sind, wobei das Paar von Stoppern (1130, 1230, 4230, 4330) an den entsprechenden Positionierungselementen (1140, 1240, 4240, 4340) anliegt und dadurch die Rückwärtsverschiebung des Unterkiefer-Mundgeräts (1120, 1220, 4220, 4320) begrenzt, und wobei jeder der Stopper (1130, 1230, 4230, 4330) einen Verlängerungsabschnitt (1134, 1234, 4234, 4334) umfasst, der an dem Mundgerät (1120, 1220, 4220, 4320) anliegt und dadurch die Verschiebung in einer Rechts-Links-Richtung des Oberkiefer-Mundgeräts (1110, 1210, 4210, 4310) und des Unterkiefer-Mundgeräts (1120, 1220, 4220, 4320) begrenzt,
wobei der Verlängerungsabschnitt (1134, 1234, 4234, 4334) eine Höhe hat, die gleich oder kleiner als die Hälfte einer Höhe der Stopper (1130, 1230, 4230, 4330) ist, und
**dadurch gekennzeichnet, dass** jeder Stopper (1130, 1230, 4230, 4330) eine geneigte Oberfläche (1136, 1236, 4236, 4336) umfasst, die an den entsprechenden Positionierungselementen (1140, 1240, 4240, 4340) anliegt, und die geneigte Oberfläche (1136, 1236, 4236, 4336) ferner im Verlängerungsabschnitt (1134, 1234, 4234, 4334) geneigt ist.

2. Mundgerät (1000, 4000) nach Anspruch 1, wobei jedes der Positionierungselemente (1140, 4240) einen Flügel (1144, 4244) umfasst, der an dem entsprechenden Stopper (1130, 4230) auf einer Außenseite des anderen Mundgeräts (1110, 4210) anliegt.

3. Mundgerät (1000, 4000) nach Anspruch 2, wobei jeder der Flügel (1144, 4244) in einer Position angeordnet ist, in der, wenn der entsprechende Stopper infolge einer Bewegung in der Rechts-Links-Richtung an dem einen Mundgerät anliegt, der Flügel (1144, 4244) nicht an dem anderen Mundgerät (1110, 4210) anliegt.

4. Mundgerät (1000, 4000) nach einem der Ansprüche 1 bis 3, wobei jeder der Stopper (1130, 1230, 4230, 4330) eine Form hat, die in einem unteren Ende eines Endabschnitts auf der Seite des einen Mundgeräts (1120, 1220, 4220, 4320) des vorderen Endabschnitts des Stoppers (1130, 1230, 4230, 4330) stumpf ist.

5. Mundgerät (1000, 4000) nach einem der Ansprüche 1 bis 4, wobei jeder der Stopper (1130, 1230, 4230, 4330) eine Form hat, die eine Projektionsfläche einer Projektion des Stoppers (1130, 1230, 4230, 4330) auf das eine Mundgerät (1120, 1220, 4220, 4320) von einer lateralen Seitenrichtung beim Beißen 3 mm² oder mehr sein lässt.

6. Mundgerät (1000, 4000) nach einem der Ansprüche 1 bis 5, wobei:
Anlageflächen des Paars von Stoppern (1130, 1230, 4230, 4330) gegen das eine Mundgerät (1120, 1220, 4220, 4320) im Wesentlichen parallel zueinander sind; und
Anlageflächen des einen Mundgeräts (1120, 1220, 4220, 4320) gegen das Paar von Stoppern (1130, 1230, 4230, 4330) im Wesentlichen parallel zueinander sind.

7. Mundgerät (1000, 4000) nach einem der Ansprüche 1 bis 6, ferner umfassend Führungsabschnitte (2160, 2260, 3150, 3250, 6350, 6450), die jeweils entgegengesetzte Enden umfassen, die von einem Paar von Befestigungsabschnitten (2162, 2262, 2164, 2264, 3160, 3260, 6360, 6460) getragen werden, die in einem Mundgerät (1120, 1220, 4220, 4320) der Ober- und Unterkiefer-Mundgeräte (1110, 1120, 1210, 1220, 4210, 4220, 4310, 4320) vorgesehen sind;
wobei das Paar von Positionierungselementen in einer Vorwärts-Rückwärts-Richtung entlang entsprechender Führungsabschnitte (2160, 2260, 3150, 3250, 6350, 6450) positionsverstellbar ist.

8. Mundgerät (1000, 4000) nach Anspruch 7, wobei die Stopper (1130, 1230, 4230, 4330) eine Höhe haben, die gleich oder geringer als eine Höhe des anderen Mundgeräts (1110, 4210) ist.

9. Mundgerät nach einem der Ansprüche 1 bis 8, ferner umfassend Vorsprungsabschnitte (3170, 3270), die an einem anderen Mundgerät (3110, 3210) der Ober- und Unterkiefer-Mundgeräte (3110, 3120, 3210, 3220) befestigt sind, wobei die Vorsprungsabschnitte (3170, 3270) von dem anderen Mundgerät (3110, 3210) in Richtung des einen Mundgeräts (3120, 3220) vorstehen;
wobei das Paar von Positionierungselementen (3140, 3240) in einer Vorwärts-Rückwärts-Richtung entlang eines Führungsabschnitts (3150, 3250), der ein Ende umfasst, das von dem entsprechenden Vorsprungsabschnitt (3170, 3270) getragen wird, in eine Position verstellt ist, in der sich das Positionierungselement (3140, 3240) beim Beißen seitlich von dem einen Mundgerät (3120, 3220) befindet und jedes der Positionierungselemente (3140, 3240) an dem entsprechenden Stopper anliegt (3130, 3230) und dadurch eine Rückwärtsverschiebung des Unterkiefer-Mundgeräts (3120, 3220) begrenzt.

## Revendications

1. Appareil buccal (1000, 4000), comprenant :
une paire d'appareils buccaux de mâchoire supérieure et inférieure (1110, 1120, 1210, 1220, 4210, 4220, 4310, 4320) se faisant face ;
une paire d'éléments de positionnement (1140, 1240, 4240, 4340), chacun étant réglable en position dans un sens avant-arrière, la paire d'éléments de positionnement (1140, 1240, 4240, 4340) étant disposée dans un appareil buccal (1120, 1220, 4220, 4320) des appareils buccaux de mâchoire supérieure et inférieure (1110, 1120, 1210, 1220, 4210, 4220, 4310, 4320) ; et
une paire de butées (1130, 1230, 4230, 4330) disposées dans un autre appareil buccal (1110, 1210, 4210, 4310) des appareils buccaux de mâchoire supérieure et inférieure (1110, 1120, 1210, 1220, 4210, 4220, 4310, 4320), la paire de butées (1130, 1230, 4230, 4330) venant en butée contre les éléments de positionnement respectifs (1140, 1240, 4240, 4340) et limitant ainsi le déplacement vers l'arrière de l'appareil buccal de mâchoire inférieure (1120, 1220, 4220, 4320) et chacune des butées (1130, 1230, 4230, 4330) comprenant une partie d'extension (1134, 1234, 4234, 4334) qui vient en butée contre l'appareil buccal (1120, 1220, 4220, 4320), limitant ainsi le déplacement dans un sens droite-gauche de l'appareil buccal de mâchoire supérieure (1110, 1210, 4210, 4310) et de l'appareil buccal de mâchoire inférieure (1120, 1220, 4220, 4320),
la partie d'extension (1134, 1234, 4234, 4334) présentant une hauteur qui est inférieure ou égale à la moitié de la hauteur des butées (1130, 1230, 4230, 4330) et
**caractérisé en ce que** chacune des butées (1130, 1230, 4230, 4330) comprend une surface inclinée (1136, 1236, 4236, 4336) qui vient en butée contre des éléments de positionnement correspondants (1140, 1240, 4240, 4340) et la surface inclinée (1136, 1236, 4236, 4336) est en outre inclinée dans la partie d'extension (1134, 1234, 4234, 4334).

2. Appareil buccal (1000, 4000) selon la revendication 1, chacun des éléments de positionnement (1140, 4240) comprenant une aile (1144, 4244) qui vient en butée contre la butée correspondante (1130, 4230) sur un côté externe de l'autre appareil buccal (1110, 4210).

3. Appareil buccal (1000, 4000) selon la revendication 2, chacune des ailes (1144, 4244) étant disposée en une position dans laquelle, lorsque la butée correspondante vient en butée contre ledit un appareil buccal suite au mouvement dans le sens droite-gauche, l'aile (1144, 4244) ne vient pas en butée contre l'autre appareil buccal (1110, 4210).

4. Appareil buccal (1000, 4000) selon l'une quelconque des revendications 1 à 3, chacune des butées (1130, 1230, 4230, 4330) présentant une forme qui est obtuse dans une extrémité inférieure d'une partie d'extrémité sur le côté dudit un appareil buccal (1120, 1220, 4220, 4320) de la partie d'extrémité avant de la butée (1130, 1230, 4230, 4330).

5. Appareil buccal (1000, 4000) selon l'une quelconque des revendications 1 à 4, chacune des butées (1130, 1230, 4230, 4330) présentant une forme qui entraîne une aire de projection de la projection de la butée (1130, 1230, 4230, 4330) sur ledit un appareil buccal (1120, 1220, 4220, 4320) à partir d'une direction latérale lors de la morsure de 3 mm² ou plus.

6. Appareil buccal (1000, 4000) selon l'une quelconque des revendications 1 à 5 :
des surfaces de butée de la paire de butées (1130, 1230, 4230, 4330) contre ledit un appareil buccal (1120, 1220, 4220, 4320) étant sensiblement parallèles l'une à l'autre ; et
des surfaces de butée dudit un appareil buccal (1120, 1220, 4220, 4320) contre la paire de butées (1130, 1230, 4230, 4330) étant sensiblement parallèles l'une à l'autre.

7. Appareil buccal (1000, 4000) selon l'une quelconque des revendications 1 à 6, comprenant en outre des sections de guidage (2160, 2260, 3150, 3250, 6350, 6450) comprenant chacune des extrémités opposées supportées par une paire de parties de fixation (2162, 2262, 2164, 2264, 3160, 3260, 6360, 6460) disposées dans un appareil buccal (1120, 1220, 4220, 4320) des appareils buccaux de mâchoire supérieure et inférieure (1110, 1120, 1210, 1220, 4210, 4220, 4310, 4320) ; la paire d'éléments de positionnement étant réglable en position dans un sens avant-arrière le long de sections de guidage respectives (2160, 2260, 3150, 3250, 6350, 6450).

8. Appareil buccal (1000, 4000) selon la revendication 7, les butées (1130, 1230, 4230, 4330) présentant une hauteur qui est égale ou inférieure à une hauteur de l'autre appareil buccal (1110, 4210).

9. Appareil buccal selon l'une quelconque des revendications 1 à 8, comprenant en outre
des parties en saillie (3170, 3270) fixées à un autre appareil buccal (3110, 3210) des appareils buccaux de mâchoire supérieure et inférieure (3110, 3120, 3210, 3220), les parties en saillie (3170, 3270) faisant saillie à partir de l'autre appareil buccal (3110, 3210) vers ledit un appareil buccal (3120, 3220) ;
la paire d'éléments de positionnement (3140, 3240) étant ajustée en position dans un sens avant-arrière le long d'une section de guidage (3150, 3250) comprenant une extrémité supportée par la partie en saillie correspondante (3170, 3270), en une position dans laquelle l'élément de positionnement (3140, 3240) est situé latéralement par rapport audit un appareil buccal (3120, 3220) lors de la morsure et chacun des éléments de positionnement (3140, 3240) venant en butée contre la butée correspondante (3130, 3230) et limitant ainsi le déplacement vers l'arrière de l'appareil buccal de mâchoire inférieure (3120, 3220).
